# EUROPEAN PATENT APPLICATION

(11) **EP 4 361 174 A1**
(43) Date of publication of application: **01.05.2024**
(21) Application number: 22827700.0
(22) Date of filing: 24.06.2022
(51) Int. Cl.: C07K 14/62, A61K 38/28, A61P 3/10

(54) **ACYLATED INSULIN-CONTAINING PHARMACEUTICAL COMPOSITION**

(30) Priority: 25.06.2021 CN 202110709826
(71) Applicant: GAN & LEE PHARMACEUTICALS CO., LTD., Beijing 101109 (CN)
(72) Inventor: GAN, Zhongru, Beijing 101109 (CN); CHEN, Wei, Beijing 101109 (CN); ZHANG, Yining, Beijing 101109 (CN); BI, Juanjuan, Beijing 101109 (CN); ZHANG, Man, Beijing 101109 (CN); XUE, Fangkai, Beijing 101109 (CN); WANG, Zaiyu, Beijing 101109 (CN); NIU, Jianghong, Beijing 101109 (CN)
(74) Representative: Kraus & Lederer PartGmbB
(86) International application number: PCT/CN2022/101204
(87) International publication number: WO 2022/268208

(57) **Abstract**

The present invention relates to an acylated insulin formulation, a pharmaceutical composition of acylated insulin and long-acting GLP-1 compound, and the pharmaceutical use of the formulation and pharmaceutical composition. The formulation and pharmaceutical composition have significantly increased efficacy, longer duration of action, longer in vivo half-life, good bioavailability, and more satisfactory physical and chemical stability compared to the insulin degludec formulation and composition comprising insulin degludec and liraglutide.

## Description

### Cross References to Related Applications

This application claims the priority to Chinese invention patent application No. CN202110709826.5, filed on June 25, 2021, which is incorporated herein by reference in its entirety.

### TECHNICAL FIELD

The present invention relates to the field of pharmaceutical compositions for the treatment of medical conditions associated with diabetes, specifically pharmaceutical compositions of acylated insulin, pharmaceutical compositions of acylated insulin and long-acting GLP-1 compounds, and medical use of the pharmaceutical compositions.

### BACKGROUND

Insulin, which is necessary in maintaining normal metabolic regulation, can be used for the treatment of diabetes and the diseases associated with or resulting therefrom. However, natural insulins such as human insulin have a short duration of action, requiring frequent injections and causing many injection-related discomforts for patients.

Currently marketed Insulin drugs include: Insulin Lispro, Insulin Aspart, Insulin Aspart 30, Insulin Detemir, Insulin Glargine, Insulin Degludec and others. But so far, no insulin product that is injected less frequently than once a day has been approved for marketing. Therefore, people have been devoting themselves to obtaining insulin derivatives or analogues with good drug efficacy, longer action time and lower injection frequency to improve the inconvenience and discomfort caused by the higher frequency of insulin injection.

CN101573133B and WO2009/010428 disclose a preparation containing PEG (PEGylated) extended insulin, which has a longer action time than that containing unmodified insulin.

WO2013086927A1 and WO2018/024186 disclose an acylated derivative of a long-acting human insulin analogue.

Furthermore, glucagon-like peptide 1 (GLP-1) and its analogs and derivatives are another very important peptide for the treatment of diabetes and diseases associated therewith. GLP-1 is an intestinal peptide hormone, mainly secreted by L cells in the terminal ileum, colon and rectum, which can promote glucose-dependent insulin secretion, promote β-cell differentiation and proliferation, and inhibit glucagon secretion. With the rapid increase in the type 2 diabetes population worldwide, there is a greater need for more effective drugs that are easier to administer. For example, a combination preparation containing two active ingredients, insulin and GLP-1 peptide, may be a very effective therapeutic agent.

WO2009/063072 discloses a pharmaceutical composition comprising GLP-1 peptides (such as liraglutide) and basal insulin derivatives (such as insulin degludec), but the injection frequency of the composition has not yet broken through once a day.

Therefore, there is still a need for compound preparations with better physical and chemical stability, longer duration of action, and better drug efficacy.

### SUMMARY

In order to overcome or improve at least one disadvantage of the prior art, or to provide a useful substitute, the first aspect of the present invention provides a novel pharmaceutical composition comprising an acylated insulin. Compared to the insulin degludec (trade name "Tresiba") already on the market or other compositions of certain insulin derivatives, the new pharmaceutical composition containing the acylated insulin has unexpected and significant increased potency, efficacy or drug efficacy, longer duration of action, longer half-life in vivo, better bioavailability, better safety, and more satisfactory physical stability, chemical stability, and solubility.

The second aspect of the present invention also provides a novel pharmaceutical composition comprising an acylated insulin and a novel long-acting GLP-1 compound which not only do not impair each other, but also unexpectedly exhibit superior efficacy, physical stability, chemical stability, duration of action, half-life in vivo compared to single composition of the acylated insulin and the GLP-1 compound, especially the acylated insulin and GLP-1 compound in the pharmaceutical composition of the present invention have unexpected synergistic drug effects, such as synergistic blood glucose-lowering effect and Hb1Ac-lowering effect. And compared with other combination fomulations of acylated insulin and long-acting GLP-1 compounds (such as the combination fomulation of liraglutide and insulin degludec (trade name: Xultophy)), the combination fomulation of the present invention has unexpectedly better drug efficacy,, duration of action, half-life in vivo, physical stability, chemical stability, etc. The combination fomulation provided by the present invention containing the acylated insulin and GLP-1 compound is well capable of achieving long pharmacokinetics (hereinafter also referred to as the PK)characteristics, enabling subcutaneous treatment of diabetic patients twice a week, once a week, once every two weeks, or less frequently.

The pharmaceutical composition described in the first aspect of the present invention comprises: an acylated insulin; 2.3 moles of zinc ions/6 moles of acylated insulin; 45 mM-60 mM phenol; 0-10mM *m-cresol;* 10 mM-20 mM NaCl; 1.5% (weight/weight) of glycerol; and 5 mM-10 mM Na₂HPO₄, wherein, the insulin parent of the acylated insulin is A14E, B16H, B25H, desB30 human insulin (SEQ ID NO: 1 and SEQ ID NO: 2, representing A chain and B chain respectively) or A14E, B16E, B25H, desB30 human insulin (SEQ ID NO: 3 and SEQ ID NO: 4, representing chain A and chain B respectively), the acyl moiety of the acylated insulin is linked to an amino group of a lysine residue or an N-terminal amino acid residue of the insulin parent, and the acyl moiety is shown as formula (D):

W1-(W2)ₘ-(W3)ₙ- (D),

wherein,
m is 0, 1, 2, 3, 4, 5, 6, 7, 8, 9 or 10, n is 5, 6, 7, 8, 9, 10, 11, 12, 13, 14, 15, 16, 17, 18, 19 or 20;
W3 is a neutral and alkylene glycol-containing amino acid residue;
W2 is an acidic amino acid residue;
W1 is a fatty diacid comprising 20-24 carbon atoms, wherein formally a hydroxyl group has been removed from one of the carboxyl groups of the fatty diacid;
W1, W2, and W3 are linked by amide bonds; and
the order of W2 and W3 presented in formula (D) can be interchanged independently.

In one embodiment, n is 5, 6, 7, 8, 9, 11, 12, 13, 14, 15, 16, 17, 18 or 19, preferably, n is 5, 6, 7, 8, 11, 12, 13, 14, 15, 16, 17, or 18, preferably, n is 5, 6, 7, 8, 11, 12, 13, 14, 15, or 16, Preferably, n is 5, 6, 7, 8, 11, 12, 13, 14, or 15.

In one embodiment, m is an integer of 1 to 6, preferably, m is 1, 2, 3, or 4, preferably, m is 1 or 2, preferably, m is 1.

In another embodiment, W1 is a fatty diacid comprising 20-23 carbon atoms, preferably W1 is a fatty diacid comprising 20, 21, or 22 carbon atoms, wherein formally a hydroxyl group has been removed from the one of the carboxyl groups of the fatty diacid.

In one embodiment, W3 is: -HN-(CH₂)₂-O-(CH₂)₂-O-CH₂-CO-, -HN-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₃-O-(CH₂)₄-O-(CH₂)₃-NH-CO-, -HN-(CH₂)₃-O-(CH₂)₄-O-(CH₂)₃-NH-CO-CH₂-O-CH₂-CO-, -HN-(CH₂)₃-O-(CH₂)₄-O-(CH₂)₃-NH-CO-(CH₂)₂-CO-, -HN-(CH₂)₂-O-(CH₂)₂-O-CH₂-CO-CH₂-O-CH₂-CO-, -HN-(CH₂)₃-O-(CH₂)₂-O-(CH₂)₂-O-(CH₂)₃-NH-CO-(CH₂)₂-CO-, -HN-(CH₂)₃-O-(CH₂)₂-O-(CH₂)₂-O-(CH₂)₃-NH-CO-CH₂-O-CH₂-CO-, -HN-(CH₂)₂-O-(CH₂)₂-O-(CH₂)₂-NH-CO-(CH₂)₂-CO-, - HN-(CH₂)₂-O-(CH₂)₂-O-(CH₂)₂-NH-CO-CH₂-O-CH₂-CO-, -HN-(CH₂)₃-O-(CH₂)₂-O-(CH₂)₂-O-(CH₂)₃-NH-CO-CH₂-O-CH₂-CO-, -HN-(CH₂)₃-O-(CH₂)₃-O-CH₂-CO-, or -HN-(CH₂)₄-O-(CH₂)₄-O-CH₂-CO-; Preferably W3 is -HN-(CH₂)₂-O-(CH₂)₂-O-CH₂-CO-; and/or

In another embodiment, W2 is an amino acid residue selected from the group consisting of yGlu, αGlu, βAsp, αAsp, γ-D-Glu, α-D-Glu, β-D-Asp or α-D-Asp, preferably W2 is selected from yGlu or βAsp; and/or In another embodiment, W1 is HOOC-(CH₂)₁₈-CO-, HOOC-(CH₂)₁₉-CO-, HOOC-(CH₂)₂₀-CO-, HOOC-(CH₂)₂₁-CO or HOOC-(CH₂)₂₂-CO-, preferably W1 is HOOC-(CH₂)₁₈-CO-, HOOC-(CH₂)₂₀-CO- or HOOC-(CH₂)₂₂-CO-.

In one embodiment, the formula (D) is is linked to the amino group of the lysine residue or the N-terminal amino acid residue of the insulin parent through the C-terminal of W3.

In one embodiment, the acyl moiety is linked to an ε amino group of the lysine residue of the insulin parent.

In one embodiment, the acylated insulin is selected from the following insulins: A14E, B16H, B25H, B29K (N(ε)-eicosanedioyl-γGlu-5xOEG), desB30 human insulin; A14E, B16H, B25H, B29K (N(ε)-eicosanedioyl-yGlu-6xOEG), desB30 human insulin; A14E, B16H, B25H, B29K (N(ε)-docosanedioyl-yGlu-5xOEG), desB30 human insulin; A14E, B16H, B25H, B29K (N(ε)-docosanedioyl-γGlu-6xOEG), desB30 human insulin; A14E, B16H, B25H, B29K (N(ε)-eicosanedioyl-γGlu-7xOEG), desB30 human insulin; A14E, B16H, B25H, B29K (N(ε)-eicosanedioyl-γGlu-8xOEG), desB30 human insulin; A14E, B16H, B25H, B29K (N(ε)-docosanedioyl-yGlu-7xOEG), desB30 human insulin; A14E, B16H, B25H, B29K (N(ε)-docosanedioyl-γGlu-8xOEG), desB30 human insulin; A14E, B16H, B25H, B29K (N(ε)-eicosanedioyl-γGlu-9xOEG), desB30 human insulin; A14E, B16H, B25H, B29K (N(ε)-docosanedioyl-γGlu-9xOEG), desB30 human insulin; or A14E, B16H, B25H, B29K (N(ε)-docosanedioyl-yGlu-10xOEG), desB30 human insulin; A14E, B16H, B25H, B29K (N(ε)-eicosanedioyl-γGlu-12xOEG), desB30 human insulin; A14E, B16H, B25H, B29K (N(ε)-docosanedioyl-γGlu-12xOEG), desB30 human insulin; A14E, B16H, B25H, B29K (N(ε)-eicosanedioyl-γGlu-11xOEG), desB30 human insulin; A14E, B16H, B25H, B29K (N(ε)-docosanedioyl-γGlu-11xOEG), desB30 human insulin; A14E, B16H, B25H, B29K (N(ε)- heneicosanedioyl-yGlu-12xOEG), desB30 human insulin; A14E, B16H, B25H, B29K (N(ε)-tricosanedioyl-γGlu-12xOEG), A14E, B16H, B25H, B29K(N(ε)-tetracosanedioyl-γGlu-12xOEG), desB30 human insulin; desB30 human insulin; A14E, B16H, B25H, B29K (N(ε)-eicosanedioyl-γGlu-13xOEG), desB30 human insulin; A14E, B16H, B25H, B29K (N(ε)-eicosanedioyl-γGlu-14xOEG), desB30 human insulin; A14E, B16H, B25H, B29K (N(ε)-eicosanedioyl-γGlu-15xOEG), desB30 human insulin; A14E, B16H, B25H, B29K (N(ε)-docosanedioyl-γGlu-13xOEG), desB30 human insulin; A14E, B16H, B25H, B29K (N(ε)-docosanedioyl-yGlu-14xOEG), desB30 human insulin; A14E, B16H, B25H, B29K (N(ε)-eicosanedioyl-γGlu-15xOEG), desB30 human insulin; A14E, B16H, B25H, B29K (N(ε)-eicosanedioyl-γGlu-16xOEG), desB30 human insulin; A14E, B16H, B25H, B29K (N(ε)-eicosanedioyl-γGlu-17xOEG), desB30 human insulin; A14E, B16H, B25H, B29K (N(ε)-docosanedioyl-yGlu-16xOEG), desB30 human insulin; A14E , B16H, B25H, B29K (N(ε)-docosanedioyl-γGlu-17xOEG), desB30 human insulin; A14E, B16H, B25H, B29K (N(ε)-eicosanedioyl-γGlu-18xOEG ), desB30 human insulin; A14E, B16H, B25H, B29K (N(ε)-eicosanedioyl-yGlu-19xOEG), desB30 human insulin; A14E, B16H, B25H, B29K (N(ε)-docosanedioyl-yGlu-18xOEG), desB30 human insulin; A14E, B16H, B25H, B29K (N(ε)-docosanedioyl-γGlu-19xOEG), desB30 human insulin; A14E, B16H, B25H, B29K (N(ε)-eicosanedioyl-γGlu-20xOEG), desB30 human insulin; A14E, B16H, B25H, B29K (N(ε)-docosanedioyl-yGlu-20xOEG), desB30 human insulin; A14E , B16H, B25H, B29K (N(ε)-docosanedioyl-yGlu-24xOEG), desB30 human insulin; A14E, B16E, B25H, B29K (N(ε)-eicosanedioyl-γGlu-5xOEG ), desB30 human insulin; A14E, B16E, B25H, B29K (N(ε)-eicosanedioyl-γGlu-6xOEG), desB30 human insulin; A14E, B16E, B25H, B29K (N(ε)-docosanedioyl-γGlu-5xOEG), desB30 human insulin; A14E, B16E, B25H, B29K (N(ε)-docosanedioyl-yGlu-6xOEG), desB30 human insulin; A14E, B16E, B25H, B29K ( N(ε)-eicosanedioyl-γGlu-7xOEG), desB30 human insulin; A14E, B16E, B25H, B29K (N(ε)-eicosanedioyl-γGlu-8xOEG), desB30 human insulin; A14E, B16E, B25H, B29K (N(ε)-docosanedioyl-γGlu-7xOEG), desB30 human insulin; A14E, B16E, B25H, B29K (N(ε)-docosanedioyl-yGlu-8xOEG), desB30 human insulin; A14E, B16E, B25H, B29K (N(ε)-eicosanedioyl-yGlu-9xOEG), desB30 human insulin; A14E, B16E, B25H, B29K (N(ε)-docosanedioyl-γGlu-9xOEG), desB30 human insulin; or A14E, B16E, B25H, B29K (N(ε)-docosanedioyl-γGlu-10xOEG), desB30 human insulin; A14E, B16E, B25H, B29K (N(ε)-eicosanedioyl-γGlu-12xOEG), desB30 human insulin; A14E, B16E, B25H, B29K (N(ε)-docosanedioyl-yGlu-12xOEG), desB30 human insulin; A14E, B16E, B25H, B29K (N(ε)-eicosanedioyl-γGlu-11xOEG), desB30 human insulin; A14E, B16E, B25H, B29K (N(ε)- docosanedioyl-γGlu-11xOEG), desB30 human insulin; A14E, B16E, B25H, B29K (N(ε)-heneicosanedioyl-γGlu-12xOEG), desB30 human insulin; A14E, B16E, B25H, B29K (N(ε)-tricosanedioyl-yGlu-12xOEG), desB30 human insulin; A14E, B16E, B25H, B29K (N(ε)-tetracosanedioyl-γGlu-12xOEG), desB30 human insulin; A14E, B16E, B25H, B29K (N(ε)-eicosanedioyl-γGlu-13xOEG), desB30 human insulin; A14E, B16E, B25H, B29K (N(ε)-eicosanedioyl-yGlu-14xOEG), desB30 human insulin; A14E, B16E, B25H, B29K (N(ε)-eicosanedioyl-yGlu-15xOEG), desB30 human insulin; A14E, B16E, B25H, B29K (N(ε)-docosanedioyl-γGlu-13xOEG), desB30 human insulin; A14E, B16E, B25H, B29K (N(ε)-docosanedioyl-γGlu-14xOEG), desB30 human insulin; A14E, B16E, B25H, B29K (N(ε)-eicosanedioyl-yGlu-15xOEG), desB30 human insulin; A14E, B16E, B25H, B29K (N(ε)-eicosanedioyl-yGlu-16xOEG), desB30 human insulin; A14E, B16E, B25H, B29K (N(ε)-eicosanedioyl-γGlu-17xOEG), desB30 human insulin; A14E, B16E, B25H, B29K (N(ε)-docosanedioyl-γGlu-16xOEG), desB30 human insulin; A14E, B16E, B25H, B29K (N(ε)-docosanedioyl-γGlu-17xOEG), desB30 human insulin; A14E, B16E, B25H, B29K (N(ε)-eicosanedioyl-yGlu-18xOEG), desB30 human insulin; A14E, B16E, B25H, B29K (N(ε)-eicosanedioyl-γGlu-19xOEG), desB30 human insulin; A14E, B16E, B25H, B29K(N(ε)-docosanedioyl-γGlu-18xOEG), desB30 human insulin; A14E, B16E, B25H, B29K(N(ε)-docosanedioyl-γGlu-19xOEG), desB30 human insulin; A14E, B16E, B25H, B29K(N(ε)-eicosanedioyl-yGlu-20xOEG), desB30 human insulin; or A14E, B16E, B25H, B29K (N(ε)-docosanedioyl-γGlu-20xOEG), desB30 human insulin;

In one embodiment, the acylated insulin is selected from the following insulins: A14E, B16H, B25H, B29K(N(ε)-eicosanedioyl-γGlu-5xOEG), desB30 human insulin; A14E, B16H, B25H, B29K (N(ε)-docosanedioyl-yGlu-5xOEG), desB30 human insulin; A14E, B16H, B25H, B29K (N(ε)-eicosanedioyl-yGlu-6xOEG), desB30 human insulin; A14E, B16H, B25H, B29K (N(ε)-docosanedioyl-γGlu-6xOEG), desB30 human insulin; A14E, B16H, B25H, B29K (N(ε)-eicosanedioyl-γGlu-8xOEG ), desB30 human insulin; A14E, B16H, B25H, B29K (N(ε)-docosanedioyl-γGlu-8xOEG), desB30 human insulin; A14E, B16H, B25H, B29K (N(ε)-eicosanedioyl-γGlu-11xOEG), desB30 human insulin; A14E, B16H, B25H, B29K (N(ε)-eicosanedioyl-yGlu-12xOEG), desB30 human insulin; A14E, B16H, B25H, B29K(N(ε)-docosanedioyl-γGlu-11xOEG), desB30 human insulin; A14E, B16H, B25H, B29K(N(ε)-docosanedioyl-γGlu-12xOEG), desB30 human insulin; or A14E, B16H, B25H, B29K (N(ε)-docosanedioyl-γGlu-18xOEG), desB30 human insulin;

In one embodiment, the acylated insulin is selected from the following insulins: A14E, B16H, B25H, B29K(N(ε)-eicosanedioyl-γGlu-12xOEG), desB30 human insulin; or A14E, B16H, B25H, B29K (N(ε)-docosanedioyl-yGlu-12xOEG), desB30 human insulin.

In one embodiment, the content of the acylated insulin is more than about 0.3 mM, preferably about 0.3-9 mM, preferably about 0.6-8.4 mM, preferably about 0.6-7.2 mM, preferably about 0.6-6.0 mM, preferably about 0.6-4.2 mM, preferably about 0.6-3.6 mM, preferably about 0.6-3.0 mM, preferably about 0.6-2.4 mM, preferably about 0.6-2.1 mM , preferably about 0.9-1.8 mM, preferably about 0.9-1.5 mM, preferably about 1.2-1.5 mM.

In another embodiment, the content of phenol is about 45 mM, about 46 mM, about 47 mM, about 48 mM, about 49 mM, 50 mM, about 51 mM, about 52 mM, about 53 mM, about 54 mM, about 55 mM, about 56 mM, about 57 mM, about 58 mM, about 59 mM, or about 60 mM.

In another embodiment, the content of m-cresol is about 0 mM, about 1 mM, about 2 mM, about 3 mM, about 4 mM, about 5 mM, about 6 mM, about 7 mM, about 8 mM, about 9 mM, or about 10 mM.

In another embodiment, the content of NaCl is about 10 mM, about 11 mM, about 12 mM, about 13 mM, about 14 mM, about 15 mM, about 16 mM, about 17 mM, about 18 mM, about 19 mM, or about 20 mM.

In another embodiment, the pharmaceutical composition has a pH value of 6.5 to 8.5; preferably a pH value of 6.5-8.0; preferably a pH value of 7.0-7.8; preferably a pH value of 7.2-7.6; more preferably a pH value of 7.4. In one embodiment, the pharmaceutical composition described in the first aspect of the present invention comprising: about 0.9-1.5 mM acylated insulin; about 2.3 moles of zinc ions/6 moles of acylated insulin; about 45 mM phenol; about 10 mM m-cresol; about 20mM NaCl; about 15mg/ml glycerin; about 5-10 mM Na₂HPO₄; and having a pH value of about 6.5-8.0, wherein the acylated insulin is A14E, B16H, B25H, B29K (N(ε)-eicosanedioyl-yGlu-5xOEG), desB30 human insulin; A14E, B16H, B25H, B29K (N(ε)-docosanedioyl-γGlu-5xOEG), desB30 human insulin; A14E, B16H, B25H, B29K (N(ε)-eicosanedioyl-γGlu-6xOEG), desB30 human insulin; A14E, B16H, B25H, B29K (N(ε)-docosanedioyl-γGlu-6xOEG), desB30 human insulin; A14E, B16H, B25H, B29K (N(ε)-eicosanedioyl-yGlu-8xOEG), desB30 human insulin; A14E, B16H, B25H, B29K (N(ε)-docosanedioyl-yGlu-8xOEG), desB30 human insulin; A14E, B16H, B25H, B29K (N(ε)-eicosanedioyl-γGlu-11xOEG ), desB30 human insulin; A14E, B16H, B25H, B29K (N(ε)-eicosanedioyl-γGlu-12xOEG), desB30 human insulin; A14E, B16H, B25H, B29K (N(ε)-docosanedioyl-γGlu-11xOEG), desB30 human insulin; A14E, B16H, B25H, B29K (N(ε)-docosanedioyl-yGlu-12xOEG), desB30 human insulin; or A14E, B16H, B25H, B29K (N(ε)-docosanedioyl-γGlu-18xOEG), desB30 human insulin. In one embodiment, the pharmaceutical composition described in the first aspect of the present invention comprising: about 1.2mM acylated insulin; about 2.3 moles of zinc ions/6 moles of acylated insulin; about 45 mM phenol; about 10 mM m-cresol; about 20 mM NaCl; about 15 mg/ml glycerin; about 5 mM Na₂HPO₄; and having a pH value of about 7.4, wherein the acylated insulin is A14E, B16H, B25H, B29K (N(ε)-eicosanedioyl-yGlu-5xOEG), desB30 human insulin; A14E, B16H, B25H, B29K (N(ε)-docosanedioyl-γGlu-5xOEG), desB30 human insulin; A14E, B16H, B25H, B29K (N(ε)-eicosanedioyl-γGlu-6xOEG), desB30 human insulin; A14E, B16H, B25H, B29K (N(ε)-docosanedioyl-γGlu-6xOEG), desB30 human insulin; A14E, B16H, B25H, B29K (N(ε)-eicosanedioyl-yGlu-8xOEG), desB30 human insulin; A14E , B16H, B25H, B29K (N(ε)-docosanedioyl-yGlu-8xOEG), desB30 human insulin; A14E, B16H, B25H, B29K (N(ε)-eicosanedioyl-γGlu-11xOEG ), desB30 human insulin; A14E, B16H, B25H, B29K (N(ε)-eicosanedioyl-γGlu-12xOEG), desB30 human insulin; A14E, B16H, B25H, B29K (N(ε)-docosanedioyl-γGlu-11xOEG), desB30 human insulin; A14E, B16H, B25H, B29K (N(ε)-docosanedioyl-yGlu-12xOEG), desB30 human insulin; or A14E, B16H, B25H, B29K (N(ε)-docosanedioyl-γGlu-18xOEG), desB30 human insulin. A pharmaceutical composition described in the second aspect of the present invention comprises: insulinotropic GLP-1 compounds and acylated insulins, wherein, the molar ratio of the insulinotropic GLP-1 compound to the acylated insulin is at least about 1:100, preferably at least about 3:100, preferably at least about 5:100, preferably at least about 8:100, preferably at least about (3:100)-(100:100), preferably about (5:100)-(80:100), preferably about (8:100)-(50:100), preferably about (10:100)-(50:100), preferably about (13:100)-(50:100), preferably about (13:100)-(40:100), preferably about (13:100)-(35: 100), preferably about (13:100)-(27:100), preferably about (13:100)-(20:100),
the acylated insulin is the acylated insulin in the pharmaceutical composition described in the first aspect of the present invention.

In one embodiment, theinsulinotropic GLP-1 compound is *N-*ε²⁶-(17-carboxyheptadecanoylamino)-4(*S*)-carboxybutanoyl-[Arg34]GLP-1-(7-37) peptide, *N*-ε²⁶-(17-carboxyheptadecanoylamino)-4(*S*)-carboxybutanoyl-[Gly8, Arg34]GLP-1-(7-37) peptide, *N*-ε²⁶-[2-(2-[2-(2-[2-(2-[4-(17-carboxyheptadecanoylamino)-4(*S*)-carboxybutanoylamino]ethoxy)ethoxy]acetylamino)ethoxy]ethoxy)acetyl ][Aib8, Arg34]GLP-1-(7-37) peptide, and *N-*ε²⁶-[2-(2-[2-(2-[2-(2-[4-(17-carboxyheptadecanoylamino)-4(*S*)-carboxybutanoylamino] ethoxy)ethoxy]acetylamino)ethoxy]ethoxy)acetyl ][Gly8, Arg34]GLP-1-(7-37) peptide, or *N*-ε³⁰-[2-(2-[2-(2-[2-(2-[4-(17-Carboxyheptadecanylamino)-4(*S*)-carboxybutanoylamino]ethoxy)ethoxy]Acetylamino)ethoxy]ethoxy) acetyl] (Val⁸ Glu²² Lys³⁰ Arg^{26,34}-GLP-1(7-37)) peptide, or *N*-ε²³-[2-(2-[2-(2-[2-(2-[4-(17-carboxyheptadecanylamino)-4(S)-carboxybutanoylamino] ethoxy)ethoxy]acetylamino)ethoxy]ethoxy)acetyl ] (Val⁸ Glu²² Lys²³ Arg^{26,34}-GLP-1(7-37)) peptide,
In one embodiment, the insulinotropic GLP-1 compound is represented by formula (B),

[Acy-(L1)ᵣ-(L2)_{q}]-G1 (B),

wherein G1 is a GLP-1 analogue with Arg and Ala or Gly, respectively at positions corresponding to position 34 and position 8, respectively, of GLP-1(7-37) (SEQ ID NO: 5), and [Acy-(L1)ᵣ-(L2)_{q}] is a substituent linked to an ε amino group of the Lys residue at position 26 of the GLP-1 analogue, wherein
r is an integer from 1 to 10, and q is an integer of 0 or from 1 to 10;
Acy is a fatty diacid comprising 20-24 carbon atoms, wherein formally a hydroxyl group has been removed from one of the carboxyl groups of the fatty diacid;
L1 is an amino acid residue selected from the group consisting of yGlu, αGlu, βAsp, αAsp, γ-D-Glu, α-D-Glu, β-D-Asp or α-D-Asp;
L2 is a neutral and alkylene glycol-containing amino acid residue;
Acy, L1, and L2 are linked by amide bonds; and
the order of L1 and L2 presented in formula (B) can be interchanged independently.

The inventors unexpectedly found that when the molar ratio of the insulinotropic GLP-1 compound to the acylated insulin in the pharmaceutical composition of the second aspect of the present invention is about 8:100, preferably about 10: 100, preferably about 13: 100, better drug efficacy can be achieved than a single preparation containing double the content of acylated insulin, when the molar ratio of the insulinotropic GLP-1 compound and the acylated insulin in the pharmaceutical composition of the second aspect of the present invention is about 20:100, better drug efficacy can be achieved simultaneously compared to the single preparation containing double the content of acylated insulin and the single preparation containing double the content of GLP-1 compound. In one embodiment, G1 is [Gly8, Arg34]GLP-1-(7-37) peptide (SEQ ID NO:6) or [Arg34]GLP-1-(7-37) peptide (SEQ ID NO:7), preferably [Gly8, Arg34] GLP-1-(7-37) peptide.

In another embodiment, r is 1, 2, 3, 4, 5 or 6, preferably r is 1, 2, 3 or 4, preferably r is 1 or 2, preferably r is 1; and/or

In another embodiment, q is 0, 1, 2, 3, 4, 5, 6, 7 or 8, preferably, q is 0, 1, 2, 3 or 4, more preferably, q is 0, 1 or 2.

In another embodiment, Acy is a fatty diacid comprising 20-23 carbon atoms, preferably Acy is a fatty diacid comprising 20, 21, or 22 carbon atoms, wherein formally a hydroxyl group has been removed from one of the carboxyl groups of the fatty diacid.

In one embodiment, L2 is -HN-(CH₂)₂-O-(CH₂)₂-O-CH₂-CO-, -HN-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₃-O-(CH₂)₄-O-(CH₂)₃-NH-CO-, -HN-(CH₂)₃-O-(CH₂)₄-O-(CH₂)₃-NH-CO-CH₂-O-CH₂-CO-, -HN-(CH₂)₃-O-(CH₂)₄-O-(CH₂)₃-NH-CO-(CH₂)₂-CO-, -HN-(CH₂)₂-O-(CH₂)₂-O-CH₂-CO-CH₂-O-CH₂-CO-, -HN-(CH₂)₃-O-(CH₂)₂-O-(CH₂)₂-O-(CH₂)₃-NH-CO-(CH₂)₂-CO-, -HN-(CH₂)₃-O-(CH₂)₂-O-(CH₂)₂-O-(CH₂)₃-NH-CO-CH₂-O-CH₂-CO-, -HN-(CH₂)₂-O-(CH₂)₂-O-(CH₂)₂-NH-CO-(CH₂)₂-CO-, - HN-(CH₂)₂-O-(CH₂)₂-O-(CH₂)₂-NH-CO-CH₂-O-CH₂-CO-, -HN-(CH₂)₃-O-(CH₂)₂-O-(CH₂)₂-O-(CH₂)₃-NH-CO-CH₂-O-CH₂-CO-, -HN-(CH₂)₃-O-(CH₂)₃-O-CH₂-CO-, or -HN-(CH₂)₄-O-(CH₂)₄-O-CH₂-CO-; preferably L2 is -HN-(CH₂)₂-O-(CH₂)₂-O-CH₂-CO-.

In another embodiment, L1 is selected from yGlu or βAsp, preferably L1 is yGlu.

In another embodiment, Acy is HOOC-(CH₂)₁₈-CO-, HOOC-(CH₂)₁₉-CO-, HOOC-(CH₂)₂₀-CO-, HOOC-(CH₂)₂₁-CO- or HOOC-(CH₂)₂₂-CO-, preferably, Acy is HOOC-(CH₂)₁₈-CO-, HOOC-(CH₂)₂₀-CO- or HOOC-(CH₂)₂₂-CO-.

In one embodiment, in the formula (B), Acy, L1, and L2 are sequentially connected by an amide bond, and the C-terminal of L2 is connected to the ε amino group of the Lys residue at position 26 of the GLP-1 analogue. In one embodiment, the insulinotropic GLP-1 compound is selected from the following compounds:
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl] [Gly8, Arg34]GLP-1-(7-37) peptide,
*N*-ε²⁶-[2-(2-[2-(2-[2-(2-[4-(21-carboxyheneicosanoylamino)-4(*S*)-carboxybutanoylamino] ethoxy)ethoxy]acetylamino)ethoxy]ethoxy)acetyl ][Gly8, Arg34]GLP-1-(7-37) peptide,
N-ε²⁶-[2-(2-[2-(4-[21-carboxyheneicosanoylamino]-4(*S*)-carboxybutanoylamino)ethoxy]ethoxy)acetyl] [Gly8, Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl] [Gly8, Arg34]GLP-1-(7-37) peptide,
*N*-ε²⁶-(23-carboxytricosanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(21-carboxyheneicosanoylamino)-4(S)-carboxybutanoyl-[Gly8, Arg34]GLP-1-(7-37) peptide,
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl] [Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl] [Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl] [Arg34]GLP-1-(7-37) peptide,
*N*-ε²⁶-(23-carboxytricosanoylamino)-4(*S*)-carboxybutanoyl-[Arg34]GLP-1-(7-37) peptide,
*N*-ε²⁶-(19-carboxynonadecanoylamino)-4(*S*)-carboxybutanoyl-[Arg34]GLP-1-(7-37) peptide,
*N*-ε²⁶-(21-carboxyheneicosanoylamino)-4(*S*)-carboxybutanoyl-[Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl] [Gly8, Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[22-carboxydocosanoylamino]-4(S)-carboxybutanoylamino)ethoxy]ethoxy)acetyl] [Gly8, Arg34]GLP-1-(7-37) peptide,
*N-*ε²⁶-(20-carboxyeicosanoylamino)-4(*S*)-carboxybutanoyl-[Gly8, Arg34]GLP-1-(7-37) peptide,
*N*-ε²⁶-(22-carboxydocosanoylamino)-4(*S*)-carboxybutanoyl-[Gly8, Arg34]GLP-1-(7-37) peptide,
*N-*ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl] [Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl] [Arg34]GLP-1-(7-37) peptide,
*N*-ε²⁶-(20-carboxyeicosanoylamino)-4(*S*)-carboxybutanoyl-[Arg34]GLP-1-(7-37) peptide, or
*N*-ε²⁶-(22-carboxydocosanoylamino)-4(*S*)-carboxybutanoyl-[Arg34]GLP-1-(7-37) peptide.

In another embodiment, the acylated insulin is selected from the following insulins: A14E, B16H, B25H, B29K (N(ε)-eicosanedioyl-γGlu-5xOEG), desB30 human insulin; A14E, B16H, B25H, B29K (N(ε))-eicosanedioyl-γGlu-6xOEG), desB30 human insulin; A14E, B16H, B25H, B29K (N(ε)-docosanedioyl-yGlu-5xOEG), desB30 human insulin; A14E, B16H, B25H, B29K (N(ε)-docosanedioyl-γGlu-6xOEG), desB30 human insulin; A14E, B16H, B25H, B29K (N(ε)-eicosanedioyl-γGlu-7xOEG), desB30 human insulin; A14E, B16H, B25H, B29K (N(ε)-eicosanedioyl-γGlu-8xOEG), desB30 human insulin; A14E, B16H, B25H, B29K (N(ε)-docosanedioyl-yGlu-7xOEG), desB30 human insulin; A14E, B16H, B25H, B29K (N(ε)-docosanedioyl-γGlu-8xOEG), desB30 human insulin; A14E, B16H, B25H, B29K (N(ε))-eicosanedioyl-γGlu-9xOEG), desB30 human insulin; A14E, B16H, B25H, B29K (N(ε)-docosanedioyl-γGlu-9xOEG), desB30 human insulin; or A14E, B16H , B25H, B29K (N(ε)-docosanedioyl-γGlu-10xOEG), desB30 human insulin; A14E, B16H, B25H, B29K (N(ε)-eicosanedioyl-γGlu-12xOEG), desB30 human insulin; A14E, B16H, B25H, B29K(N(ε)-docosanedioyl-γGlu-12xOEG), desB30 human insulin; A14E, B16H, B25H, B29K (N(ε)-eicosanedioyl-γGlu-11xOEG), desB30 human insulin; A14E, B16H, B25H, B29K (N(ε)-docosanedioyl-γGlu-11xOEG), desB30 human insulin; A14E, B16H, B25H, B29K (N(ε)- heneicosanedioyl-yGlu-12xOEG), desB30 human insulin; A14E, B16H, B25H, B29K (N(ε)-tricosanedioyl-γGlu-12xOEG), desB30 human insulin; A14E, B16H, B25H, B29K(N(ε)-tetracosanedioylyGlu-12xOEG), desB30 human insulin; A14E, B16H, B25H, B29K (N(ε)-eicosanedioyl-yGlu-13xOEG), desB30 human insulin; A14E, B16H, B25H, B29K (N(ε)-eicosanedioyl-γGlu-14xOEG), desB30 human insulin; A14E, B16H, B25H, B29K (N(ε)-eicosanedioyl-γGlu-15xOEG), desB30 human insulin; A14E, B16H, B25H, B29K (N(ε)-docosanedioyl-γGlu-13xOEG), desB30 human insulin; A14E, B16H, B25H, B29K (N(ε)-eicosanedioyl-yGlu-14xOEG), desB30 human insulin; A14E, B16H, B25H, B29K (N(ε)-eicosanedioyl-γGlu-15xOEG), desB30 human insulin; A14E, B16H, B25H, B29K (N(ε)-eicosanedioyl-γGlu-16xOEG), desB30 human insulin; A14E, B16H, B25H, B29K (N(ε)-eicosanedioyl-γGlu-17xOEG), desB30 human insulin; A14E, B16H, B25H, B29K (N(ε)-docosanedioyl-yGlu-16xOEG), desB30 human insulin; A14E, B16H, B25H, B29K (N(ε)-docosanedioyl-γGlu-17xOEG), desB30 human insulin; A14E, B16H, B25H, B29K (N(ε)-eicosanedioyl-γGlu-18xOEG), desB30 human insulin; A14E, B16H, B25H, B29K (N(ε)-eicosanedioyl-yGlu-19xOEG), desB30 human insulin; A14E, B16H, B25H, B29K (N(ε)-docosanedioyl-yGlu-18xOEG), desB30 human insulin; A14E, B16H, B25H, B29K (N(ε)-docosanedioyl-γGlu-19xOEG), desB30 human insulin; A14E, B16H, B25H, B29K (N(ε)-eicosanedioyl-γGlu-20xOEG), desB30 Human insulin; A14E, B16H, B25H, B29K (N(ε)-docosanedioyl-yGlu-20xOEG), desB30 human insulin; A14E, B16H, B25H, B29K (N(ε)-docosanedioyl-yGlu-24xOEG), desB30 human insulin; A14E, B16E, B25H, B29K (N(ε)-eicosanedioyl-γGlu-5xOEG), desB30 human insulin; A14E, B16E, B25H, B29K (N(ε)-eicosanedioyl-γGlu-6xOEG), desB30 human insulin; A14E, B16E, B25H, B29K (N(ε)-docosanedioyl-γGlu-5xOEG), desB30 human insulin; A14E, B16E, B25H, B29K (N(ε)-docosanedioyl-yGlu-6xOEG), desB30 human insulin; A14E, B16E, B25H, B29K (N(ε)-eicosanedioyl-γGlu-7xOEG), desB30 Human insulin; A14E, B16E, B25H, B29K (N(ε)-eicosanedioyl-γGlu-8xOEG), desB30 human insulin; A14E, B16E, B25H, B29K (N(ε)-docosanedioyl-γGlu-7xOEG), desB30 human insulin; A14E, B16E, B25H, B29K (N(ε)-docosanedioyl-yGlu-8xOEG), desB30 human insulin; A14E, B16E, B25H, B29K (N(ε))-eicosanedioyl-yGlu-9xOEG), desB30 human insulin; A14E, B16E, B25H, B29K (N(ε)-docosanedioyl-γGlu-9xOEG), desB30 human insulin; or A14E, B16E, B25H, B29K (N(ε)-docosanedioyl-γGlu-10xOEG), desB30 human insulin; A14E, B16E, B25H, B29K (N(ε)-eicosanedioyl-γGlu-12xOEG), desB30 human insulin; A14E, B16E, B25H, B29K (N(ε)-docosanedioyl-yGlu-12xOEG), desB30 human insulin; A14E, B16E, B25H, B29K (N(ε)-eicosanedioyl-γGlu-11xOEG), desB30 human insulin; A14E, B16E, B25H, B29K (N(ε)-docosanedioyl-γGlu-11xOEG), desB30 human insulin; A14E, B16E, B25H, B29K (N(ε)-heneicosanedioyl-γGlu-12xOEG), desB30 human insulin; A14E, B16E, B25H, B29K (N(ε)-tricosanedioyl-yGlu-12xOEG), desB30 human insulin; A14E, B16E, B25H, B29K (N(ε)-tetracosanedioyl-γGlu-12xOEG), desB30 human insulin; A14E, B16E, B25H, B29K (N(ε)-eicosanedioyl-γGlu-13xOEG), desB30 human insulin; A14E, B16E, B25H, B29K (N(ε)-eicosanedioyl-yGlu-14xOEG), desB30 human insulin; A14E, B16E, B25H, B29K (N(ε)-eicosanedioyl-yGlu-15xOEG), desB30 human insulin; A14E, B16E, B25H, B29K (N(ε)-docosanedioyl-γGlu-13xOEG), desB30 human insulin; A14E, B16E, B25H, B29K (N(ε)-docosanedioyl-γGlu-14xOEG), desB30 human insulin; A14E, B16E, B25H, B29K (N(ε)-eicosanedioyl-yGlu-15xOEG), desB30 human insulin; A14E, B16E , B25H, B29K(N(ε)-eicosanedioyl-yGlu-16xOEG), desB30 human insulin; A14E, B16E, B25H, B29K (N(ε)-eicosanedioyl-γGlu-17xOEG), desB30 human insulin; A14E, B16E, B25H, B29K (N(ε)-docosanedioyl-γGlu-16xOEG), desB30 human insulin; A14E, B16E, B25H, B29K (N(ε)-docosanedioyl-γGlu-17xOEG), desB30 human insulin; A14E, B16E, B25H, B29K(N(ε)-eicosanedioyl-yGlu-18xOEG), desB30 human insulin; A14E, B16E, B25H, B29K (N(ε))-eicosanedioyl-γGlu-19xOEG), desB30 human insulin; A14E, B16E, B25H, B29K (N(ε)- docosanedioyl-γGlu-18xOEG), desB30 human insulin; A14E, B16E, B25H, B29K (N(ε)-docosanedioyl-yGlu-19xOEG), desB30 human insulin; A14E, B16E, B25H, B29K (N(ε)-eicosanedioyl-yGlu-20xOEG), desB30 human insulin; or A14E, B16E, B25H, B29K (N(ε)-docosanedioyl-γGlu-20xOEG), desB30 human insulin. In one embodiment, the content of the acylated insulin is more than about 0.3 mM, preferably about 0.3-9 mM, preferably about 0.6-8.4 mM, preferably about 0.6- 7.2 mM, preferably about 0.6-6.0 mM, preferably about 0.6-4.2 mM, preferably about 0.6-3.6 mM, preferably about 0.6-3.0 mM, preferably about 0.6-2.4 mM, preferably about 0.6-2.1 mM, preferably about 0.9-1.8 mM, preferably about 0.9-1.5 mM, preferably about 1.2-1.5 mM.

In one embodiment, the pharmaceutical composition described in the second aspect of the present invention further comprises: zinc ions, glycerol, phenol, m-cresol, NaCl, and/or Na₂HPO₄.

In one embodiment, the content of zinc ions is at least about 1.5 moles of zinc ions/6 moles of acylated insulin, preferably about 1.5-12 moles of zinc ions/6 moles of acylated insulin, preferably about 1.5-10 moles of zinc ions/6 moles of acylated insulin, preferably about 1.5-8 moles of zinc ions/6 moles of acylated insulin, preferably about 1.5-6 moles of zinc ions/6 moles of acylated insulin, preferably about 1.5-4.5 moles of zinc ions/6 moles of acylated insulin, preferably about 1.5-3.5 moles of zinc ions /6 moles of acylated insulin, preferably about 1.5-2.3 moles of zinc ions/6 moles of acylated insulin.

In one embodiment, the content of glycerin is no more than about 2.5% (weight/weight), preferably no more than about 2% (weight/weight), preferably about 0.3% to about 2% (weight/weight), preferably about 0.5% to about 1.8% (weight/weight), preferably about 0.7% to about 1.8% (weight/weight), preferably about 1% to about 1.8% (weight/weight), preferably about 1.5% to about 1.7% (weight/weight).

In another embodiment, the content of the phenol is about 30-70 mM, preferably about 40-65 mM, preferably about 45-60 mM; preferably about 45 mM, about 46 mM, about 47 mM, about 48 mM, about 49 mM, 50 mM, about 51 mM, about 52 mM, about 53 mM, about 54 mM, about 55 mM, about 56 mM, about 57 mM, about 58 mM, about 59 mM, about 60 mM, about 61 mM, about 62 mM, about 63 mM, about 64 mM, or about 65 mM. In another embodiment, the content of the *m-cresol* is about 0-35 mM, preferably about 0-20mM, preferably about 0-10mM, preferably about 0 mM, about 1 mM, about 2 mM, about 3 mM, about 4 mM, about 5 mM, about 6 mM, about 7 mM, about 8 mM, about 9 mM, about 10 mM, about 11 mM, about 12 mM, about 13 mM, about 14 mM, or about 15 mM.

In another embodiment, the content of the NaCl is about 0-150 mM, preferably about 5-120 mM, preferably about 10-120 mM, preferably about 10-100 mM, preferably about 10-75 mM, preferably about 10-50mM, preferably about 10-30 mM, preferably about 10-20 mM; preferably about 10 mM, about 11 mM, about 12 mM, about 13 mM, about 14 mM, about 15 mM, about 16 mM, about 17 mM, about 18 mM, about 19 mM, or about 20 mM.

In another embodiment, the content of Na₂HPO₄ is about 0-75 mM, preferably about 5-60 mM, preferably about 5-50 mM, preferably about 5-25 mM, preferably about 5-15 mM; preferably about 5-10 mM.

In another embodiment, the pharmaceutical composition described in the second aspect of the present invention has a pH value of about 6.5-8.5; preferably a pH value of about 6.8-8.2; preferably a pH value of about 7.0-8.2; preferably a pH value of 7.2-7.6; more preferably a pH value of 7.4 or 7.6.

In one embodiment, the pharmaceutical composition described in the second aspect of the present invention comprises:
about 0.9-1.5 mM acylated insulin;
insulinotropic GLP-1 compound, the molar ratio of the insulinotropic GLP-1 compound to the acylated insulin is at least about 8:100, preferably about (8:100)-(50:100), preferably about (10:100)-(50:100), preferably about (13:100)-(50:100), preferably about (13:100)-(40:100), preferably about (13:100)- (35:100), preferably about (13:100)-(27:100), preferably about (13:100)-(20:100);
about 1.5-2.3 moles of zinc ions per 6 moles of acylated insulin;
about 45mM-60 mM phenol;
about 0-10 mM m-cresol;
about 10-20 mM NaCl;
about 15-17 mg/ml glycerin;
about 5-10 mM Na₂HPO₄; and
has a pH value of about 6.5-8.0,
wherein the acylated insulin is A14E, B16H, B25H, B29K (N(ε)-eicosanedioyl-yGlu-5xOEG), desB30 human insulin; A14E, B16H, B25H, B29K (N(ε)-docosanedioyl-γGlu-5xOEG), desB30 human insulin; A14E, B16H, B25H, B29K (N(ε)-eicosanedioyl-γGlu-6xOEG), desB30 human insulin; A14E, B16H, B25H, B29K (N(ε)-docosanedioyl-γGlu-6xOEG), desB30 human insulin; A14E, B16H, B25H, B29K (N(ε)-eicosanedioyl-yGlu-8xOEG), desB30 human insulin; A14E , B16H, B25H, B29K (N(ε)-docosanedioyl-yGlu-8xOEG), desB30 human insulin; A14E, B16H, B25H, B29K (N(ε)-eicosanedioyl-γGlu-11xOEG ), desB30 human insulin; A14E, B16H, B25H, B29K (N(ε)-eicosanedioyl-γGlu-12xOEG), desB30 human insulin; A14E, B16H, B25H, B29K (N(ε)-docosanedioyl-γGlu-11xOEG), desB30 human insulin; A14E, B16H, B25H, B29K (N(ε)-docosanedioyl-yGlu-12xOEG), desB30 human insulin; or A14E, B16H, B25H, B29K (N(ε)-docosanedioyl-γGlu-18xOEG), desB30 human insulin, and
the insulinotropic GLP-1 compound is *N*-ε²⁶-[2-(2-[2-(2-[2-(2-[4-(19-carboxynonadecanoylamino)-4(*S*)
carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[19-Carboxynonadecanoylamino]-4(*S*)-carboxybutanoylamino)ethoxy]ethoxy)acetyl][Gly8, Arg34]GLP-1-(7-37) peptide,
*N*-ε²⁶-(19-Carboxynonadecanoylamino)-4(*S*)-carboxybutanoyl-[Gly8, Arg34] GLP-1-(7-37) peptide,
*N*-ε²⁶-(19-Carboxynonadecanoylamino)-4(*S*)-carboxybutanoyl-[Arg34] GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetyl)ethoxy]ethoxy)acetyl] [Gly8, Arg34] GLP-1-(7-37) peptide, or
*N*-ε²⁶-[2-(2-[2-(4-[21-carboxyheneicosanoylamino]-4(*S*)-carboxybutanoylamino)ethoxy]ethoxy)acetyl][Gly8, Arg34] GLP-1-(7-37) peptide.

In one embodiment, the pharmaceutical composition described in the second aspect of the present invention comprises:
about 1.2mM acylated insulin;
at least about 0.096mM, preferably about 0.096-0.6mM, preferably about 0.12-0.6mM, preferably about 0.16-0.6mM, preferably about 0.16-0.48mM, preferably about 0.16-0.42mM, preferably about 0.16-0.32mM, preferably about 0.16 - 0.24 mM insulinotropic GLP-1 compound;
about 1.5-2.3 moles of zinc ions per 6 moles of acylated insulin;
about 45 mM-60 mM phenol;
about 0-10 mM *m-cresol;*
about 10-20 mM NaCl;
about 15-17 mg/ml glycerin;
about 5-10 mM Na₂HPO₄; and
has a pH value of about 6.5-8.0,
wherein the acylated insulin is A14E, B16H, B25H, B29K (N(ε)-eicosanedioyl-yGlu-5xOEG), desB30 human insulin; A14E, B16H, B25H, B29K (N(ε)-docosanedioyl-γGlu-5xOEG), desB30 human insulin; A14E, B16H, B25H, B29K (N(ε)-eicosanedioyl-γGlu-6xOEG), desB30 human insulin; A14E, B16H, B25H, B29K (N(ε)-docosanedioyl-γGlu-6xOEG), desB30 human insulin; A14E, B16H, B25H, B29K (N(ε)-eicosanedioyl-yGlu-8xOEG), desB30 human insulin; A14E, B16H, B25H, B29K (N(ε)-docosanedioyl-yGlu-8xOEG), desB30 human insulin; A14E, B16H, B25H, B29K (N(ε)-eicosanedioyl-γGlu-11xOEG ), desB30 human insulin; A14E, B16H, B25H, B29K (N(ε)-eicosanedioyl-γGlu-12xOEG), desB30 human insulin; A14E, B16H, B25H, B29K (N(ε)-docosanedioyl-γGlu-11xOEG), desB30 human insulin; A14E, B16H, B25H, B29K(N(ε)-docosanedioyl-yGlu-12xOEG), desB30 human insulin; or A14E, B16H, B25H, B29K (N(ε)-docosanedioyl-γGlu-18xOEG), desB30 human insulin, and
the insulinotropic GLP-1 compound is *N-*ε²⁶ -[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl ][Gly8, Arg34] GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly8, Arg34] GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl-[Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy base] acetylamino) ethoxy] ethoxy) acetyl] [Gly8, Arg34] GLP-1-(7-37) peptide, or
*N*-ε²⁶-[2-(2-[2-(4-[21-carboxyheneicosanoylamino]-4(*S*)-carboxybutanoylamino)ethoxy]ethoxy)acetyl][Gly8, Arg34] GLP-1-(7-37) peptide.

The third aspect of the present invention provides the use of the pharmaceutical composition described in the first aspect and the second aspect of the present invention as a medicine.

The fourth aspect of the present invention provides the pharmaceutical composition described in the first aspect and the second aspect of the present invention for treating or preventing diabetes, hyperglycemia, and/or impaired glucose tolerance.

The fifth aspect of the present invention provides the use of the pharmaceutical composition described in the first aspect and the second aspect of the present invention in the preparation of a medicament for treating or preventing diabetes, hyperglycemia, and/or impaired glucose tolerance.

The sixth aspect of the present invention provides a method for treating or preventing diabetes, hyperglycemia, and/or impaired glucose tolerance, the method comprising administering an effective amount of the pharmaceutical composition described in the first aspect and the second aspect of the present invention.

### BRIEF DESCRIPTION OF THE DRAWINGS

FIG. 1a shows the hypoglycemic effect of compound 2 mono-formulation and compound 11 mono-formulation at medium dosage, pharmaceutical compositions comprising compound 2 and compound 11 at low dosage, and vehicle on db/db mice.
FIG. 1b shows, in correspondence with FIG. 1a, the △AUC of the hypoglycemic effect of the compound 2 mono-formulation and compound 11 mono-formulation at medium dosage, pharmaceutical compositions comprising compound 2 and compound 11 at low dosage and vehicle on db/db mice.
FIG. 1c shows the hypoglycemic effect of compound 2 mono-formulation, compound 11 mono-formulation and pharmaceutical compositions comprising compound 2 and compound 11 at medium dosage and vehicle on db/db mice.
FIG. 1d shows, in correspondence with FIG. 1c, the △AUC of the hypoglycemic effect of compound 2 mono-formulation, compound 11 mono-formulation and pharmaceutical compositions comprising compound 2 and compound 11 at medium dosage and the vehicle on db/db mice.
FIG. 1e shows the HbA1c-reducing effect of compound 2 mono-formulation and compound 11 mono-formulation at medium dosage, pharmaceutical compositions comprising compound 2 and compound 11 at low dosage and vehicle on db/db mice.
FIG. 1f shows the HbA1C-reducing effect of compound 2 mono-formulation, compound 11 mono-formulation and pharmaceutical compositions comprising compound 2 and compound 11 at medium dosage and vehicle on db/db mice.
FIG. 2a shows the hypoglycemic effect of compound 2 mono-formulation, compound 11 mono-formulation and the composition comprising insulin degludec and liraglutide(control compositions) at high dosage, pharmaceutical compositions comprising compound 2 and compound 11 at low dosage and vehicle on db/db mice.
Fig. 2b shows, in correspondence with Fig. 2a, the AUC of compound 2 mono-formulation, compound 11 mono-formulation and composition comprising insulin degludec and liraglutide at high dosage, pharmaceutical compositions comprising compound 2 and compound 11 at low dosage and vehicle on db/db mice.
FIG. 2c shows the HbA1C-reducing effect of compound 2 mono-formulation, compound 11 mono-formulation, composition comprising insulin degludec and liraglutide(control compositions), and pharmaceutical compositions comprising compound 2 and compound 11 at high dosage, pharmaceutical compositions comprising compound 2 and compound 11 at medium dosage, pharmaceutical compositions comprising compound 2 and compound 11 at low dosage and vehicle on db/db mice.
FIG. 2d shows the TG-reducing effect of compound 2 mono-formulation, compound 11 mono-formulation and composition comprising insulin degludec and liraglutide at high dosage, pharmaceutical compositions comprising compound 2 and compound 11 at low dosage and vehicle on db/db mice.
FIG. 3a shows the hypoglycemic effect of compound 2 mono-formulation, Control Example 1 mono-formulation, degludec formulation and the vehicle on db/db mice.

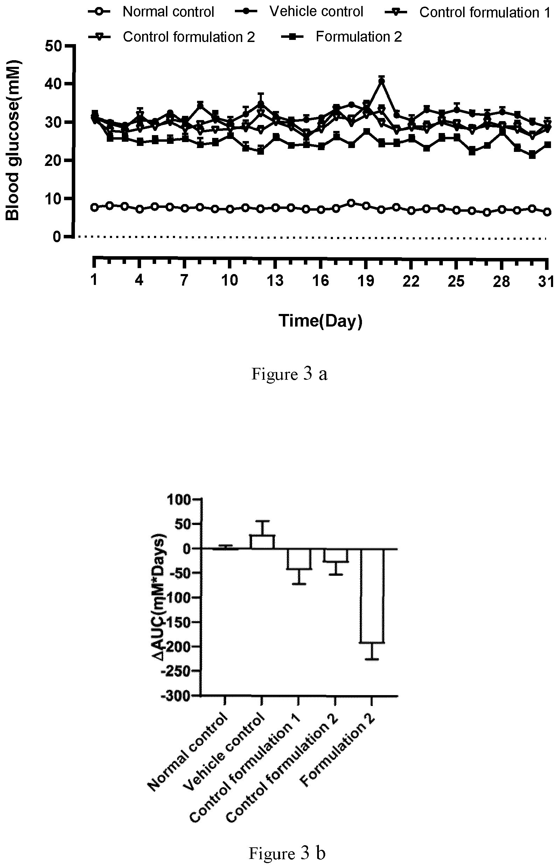

FIG. 3a shows, in correspondence with FIG. 3b, the Δ AUC of hypoglycemic effect of compound 2 mono-formulation, Control Example 1 mono-formulation, degludec formulation and the vehicle on db/db mice.

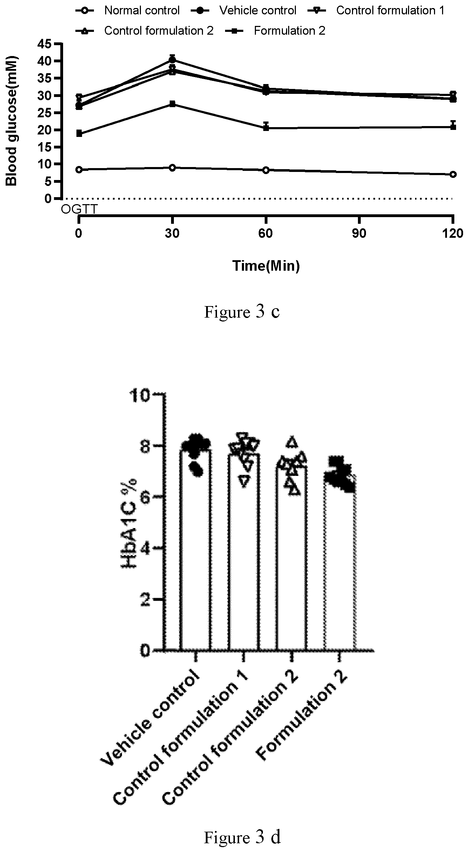

FIG. 3c shows the hypoglycemic effect of compound 2 mono-formulation, Control Example 1 mono-formulation, degludec formulation and vehicle on db/db mice during OGTT 48 hour after first dose.
FIG. 3d shows the HbA1C-reducing effect of compound 2 mono-formulation, Control Example 1 mono-formulation, degludec formulation and the vehicle on db/db mice.

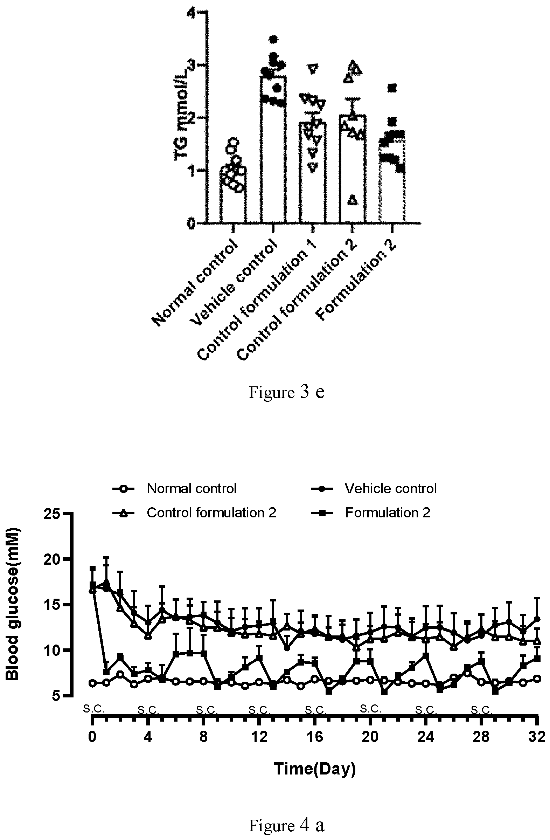

FIG. 3e shows the TG-reducing effect of compound 2 mono-formulation, Control Example 1 mono-formulation, degludec formulation and the vehicle on db/db mice.
FIG. 4a shows the hypoglycemic effect of compound 2 mono-formulation, degludec formulation and the vehicle on GK rats.

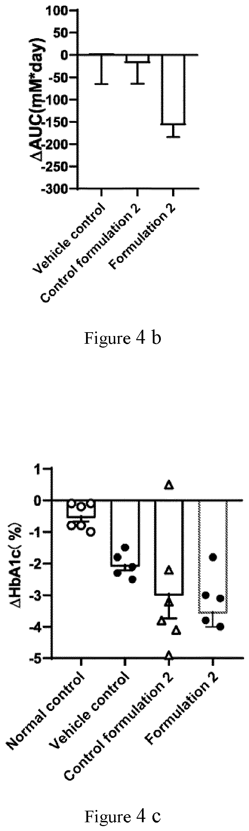

FIG. 4b shows, in correspondence with FIG. 4a, the AUC of the hypoglycemic effect of compound 2 mono-formulation, degludec formulation and vehicle on GK rats.
FIG. 4c shows the HbA1c-reducing effect of compound 2 mono-formulation, degludec formulation and vehicle on GK rats.

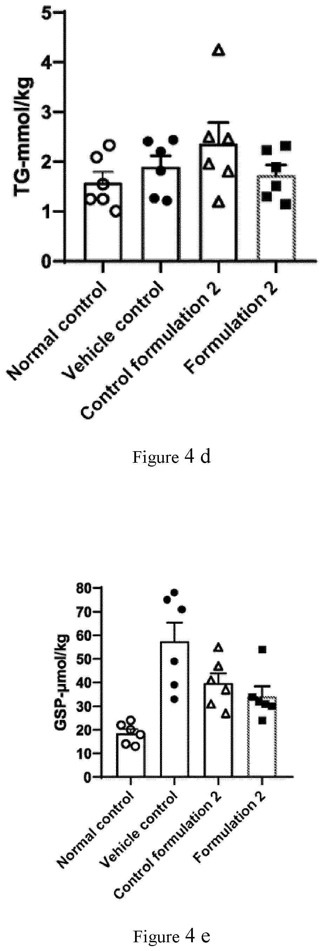

FIG. 4d shows the TG-reducing effect of compound 2 mono-formulation, degludec formulation and vehicle on GK rats.
FIG. 4e shows the GSP-reducing effect of compound 2 mono-formulation, degludec formulation and vehicle on GK rats.

### DETAILED DESCRIPTION OF THE INVENTION

### Definitions

Herein, the term "insulin" encompasses natural insulins, such as human insulin, insulin analogues and insulin derivatives thereof.

The term "insulin analogue" covers a polypeptide having a molecular structure which may be formally derived from the structure of a natural insulin (e.g., human insulin) by deletion and/or substitution (replacement) of one or more amino acid residues presented in the natural insulin and/or by addition of one or more amino acid residues. The amino acid residues for addition and/or substitution may be encodable amino acid residues, or other natural amino acid residues, or purely synthetic amino acid residues. Preferably, the amino acid residues for addition and/or substitution are encodable amino acid residues.

Herein, the term "insulin derivative" refers to a natural insulin or insulin analogue which has been chemically modified, and the modification may be, for example, introducing a side chain at one or more positions of the insulin backbone, oxidizing or reducing groups of amino acid residues on the insulin, converting a free carboxyl group into an ester group, or acylating a free amino group or a hydroxyl group. The acylated insulins of the present invention are insulin derivatives.

The term "insulin parent" (also referred to herein as parent insulin) refers to an insulin moiety of an insulin derivative or an acylated insulin, and, for example, refers to a moiety of an insulin derivative or an acylated insulin without a linking side chain or an added acyl group in the present invention. The insulin parent may be a natural insulin, such as human insulin or porcine insulin. In another aspect, the parent insulin may be an insulin analogue.

Herein, the term "amino acid residue" encompasses amino acids from which a hydrogen atom has been removed from an amino group and/or a hydroxyl group has been removed from a carboxyl group and/or a hydrogen atom has been removed from a mercapto group. Imprecisely, an amino acid residue may be referred to as an amino acid.

Unless otherwise stated, all amino acids referred to herein are L-amino acids.

Herein, the term "alkylene glycol" comprises oligo- and poly-alkylene glycol moieties and monoalkylene glycol moieties. Monoalkylene glycols and polyalkylene glycols include, for example, chains based on monoethylene and polyethylene glycols, monopropylene and polypropylene glycols, and monotetramethylene and polytetramethylene glycols, i.e., chains based on the repeating unit -CH₂CH₂O-, - CH₂CH₂CH₂O- or -CH₂CH₂CH₂CH₂O-. The alkylene glycol moiety can be monodisperse (with well-defined length/molecular weight) and polydisperse (with less well-defined length/average molecular weight). The monoalkylene glycol moiety includes -OCH₂CH₂O-, - OCH₂CH₂CH₂O- or -OCH₂CH₂CH₂CH₂O- comprising different groups at each end.

Herein, the term "fatty diacid" includes linear or branched fatty dicarboxylic acids having at least two carbon atoms and being saturated or unsaturated. Non-limiting examples of fatty diacids are hexanedioic acid, octanedioic acid, decanedioic acid, dodecanedioic acid, tetradecanedioic acid, hexadecanedioic acid, heptadecanedioic acid, octadecanedioic acid, eicosanedioic acid, docosanedioic acid and tetracosanedioic acid.

The term "basal insulin" refers to an insulin having a longer duration of action than conventional or normal human insulin.

As used herein, "drug effect" or "potency" refers to the ability of a drug or an active compound to result in a certain function or effect (e.g., lowering blood glucose). For example, compared with insulin degludec or other existing insulin derivatives, administration of the same dose of an insulin derivative of the present invention will result in a better blood glucose lowering effect or function.

The term "diabetes" includes type 1 diabetes, type 2 diabetes, gestational diabetes (during pregnancy) and other conditions that cause hyperglycemia. The term is used for metabolic disorders in which the pancreas produces insufficient amount of insulin or in which cells of the body fail to respond appropriately to insulin, thereby preventing the cells from taking up glucose. As a result, glucose accumulates in the blood.

Type 1 diabetes, also known as insulin-dependent diabetes mellitus (IDDM) and juvenile onset diabetes, is caused by β -cell destruction and often results in absolute insulin deficiency. Type 2 diabetes, also known as non-insulin dependent diabetes mellitus (NIDDM) and adult onset diabetes, is associated with major insulin resistance and thus major defects in insulin secretion featuring relative insulin deficiency and/or insulin resistance.

As used herein, the term "GLP-1 analogue" or "analogue of GLP-1" refers to a peptide or compound that is a variant of human glucagon-like peptide-1 (GLP-1(7-37)), wherein one or more amino acid residues of GLP-1(7-37) are replaced, and/or one or more amino acid residues are deleted, and/or one or more amino acid residues are added. Specifically, the sequence of GLP-1(7-37) is shown in SEQ ID NO: 5 in the sequence listing. A peptide having the sequence shown in SEQ ID NO: 5 may also be referred to as "natural" GLP-1 or "natural" GLP-1(7-37).

In the sequence listing, the first amino acid residue (His) in SEQ ID NO: 5 is numbered 1. However, in the following, according to established practice in the art, the histidine residue is numbered 7 and the following amino acid residues are numbered sequentially, ending with glycine as No. 37. Thus, in general, based on the numbering for amino acid residues or positions, the GLP-1(7-37) sequence referred to herein is a sequence starting with His at position 7 and ending with Gly at position 37.

[Gly8, Arg34]GLP-1-(7-37) peptide is a GLP-1 analogue having Gly and Arg at positions corresponding to position 8 and position 34, respectively, of GLP-1(7-37) (SEQ ID NO: 5). [Arg34]GLP-1-(7-37) peptide is a GLP-1 analogue having Arg at a position corresponding to position 34 of GLP-1(7-37) (SEQ ID NO: 5). Specifically, the amino acid sequences of [Gly8, Arg34]GLP-1-(7-37) peptide and [Arg34]GLP-1-(7-37) peptide are shown in SEQ ID NO: 6 and SEQ ID NO: 7 in the sequence listing, respectively. In the case of a GLP-1 peptide or an analogue thereof, the term "derivative" as used herein refers to a chemically modified GLP-1 peptide or analogue, wherein one or more substituents have been covalently linked to the peptide. Substituents may also be referred to as side chains.

As used herein, the naming of insulin or GLP-1 compounds follows the following principle: the names are given according to mutations and modifications (e.g., acylation) relative to human insulin, or mutations and modifications (e.g., acylation) of natural GLP-1(7-37). The naming of the acyl moieties is based on the IUPAC nomenclature and, in other cases, the peptide nomenclature. For example, the following acyl moiety: 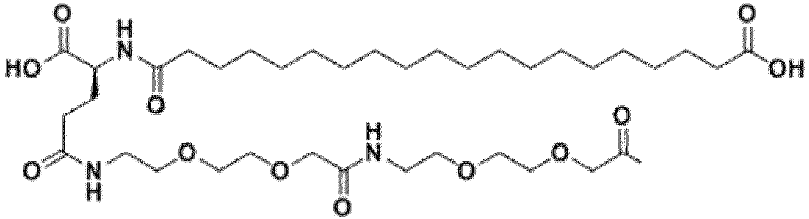 can be named, for example, as "eicosanedioyl-yGlu-OEG-OEG", "eicosanedioyl-γGlu-2×OEG" or "eicosanedioyl-gGlu-2×OEG", or "19-carboxynonadecanoyl-yGlu-OEG-OEG", wherein OEG is the shorthand for the group -NH(CH₂)₂O(CH₂)₂OCH₂CO- (i.e., 2-[2-(2-aminoethoxy)ethoxy]acetyl) and yGlu (or gGlu) is a shorthand for the amino acid γ-glutamic acid in the L configuration. Alternatively, the acyl moieties may be named according to IUPAC nomenclature (OpenEye, IUPAC format). According to this nomenclature, the above acyl moiety of the present invention is referred to as the following name: [2-(2-[2-(2-[2-(2-[4-(19-carboxynonadecanoylamino)-4(*S*)-carboxybutanoylamino] ethoxy)ethoxy] acetylamino)ethoxy]ethoxy)acetyl ], or [2-[2-[2-[2-[2-[2-[(4S)-4-carboxy-4-(19-carboxynonadecanoylamino)butanoyl] -amino -ethoxy] - ethoxy] acetyl] amino] ethoxy] ethoxy]acetyl].

For example, herein structure/sequence is referred to as "A14E, B16H, B25H, B29K(*N^{ε}*-eicosanedioyl-gGlu-2×OEG), desB30 human insulin" or "A14E, B16H, B25H, B29K(*N*(ε)-eicosanedioyl-γGlu-2×OEG), desB30 human insulin", which indicates that amino acid Y at position A14 in human insulin has been mutated to E, amino acid Y at position B16 in human insulin has been mutated to H, amino acid F at position B25 in human insulin has been mutated to H, amino acid K at position B29 in human insulin has been modified by acylation with the residue eicosanedioyl-gGlu-2×OEG on the ε nitrogen (referred to as N^{ε}) of the lysine residue at position B29, and amino acid T at position B30 in human insulin has been deleted.

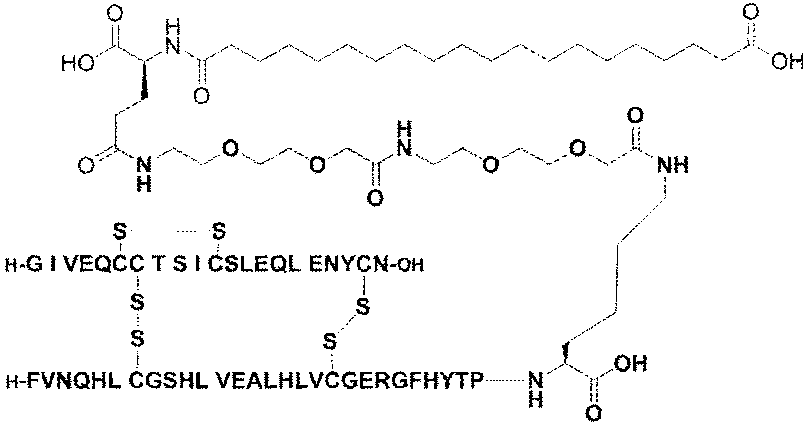

Insulin is a polypeptide hormone secreted by β cells in the pancreas and is composed of two polypeptide chains, namely A chain and B chain, linked by two inter-chain disulfide bonds. In addition, the A chain is characterized by having an intra-chain disulfide bond.

Nucleic acid sequences encoding polypeptides of the insulin analogues can be prepared synthetically by established standard methods, for example, by the method described in Beaucage et al. (1981) Tetrahedron Letters 22:1859-1869 or Matthes et al. (1984) EMBO Journal 3:801-805.

In a specific embodiment, herein pharmaceutical composition comprises at least one pharmaceutically acceptable excipient. The term "excipient" broadly refers to any component other than the active therapeutic ingredient. The excipient may be inert substances, inactive substances and/or non-pharmaceutically active substances. The excipient may be used for various purposes, for example as carriers, vehicles, diluents, adhesives, lubricants, flow enhancers, diluents, and/or for improving administration and/or absorption of the active substances, depending on the pharmaceutical composition. Pharmaceutical compositions of pharmaceutically active ingredients with various excipients are known in the art, see, e.g., Remington: The Science and Practice of Pharmacy (e.g., 19th edition (1995), and any later versions).

In some embodiments, specific values referred to herein and given with respect to numbers or intervals may be understood to be that specific value or about that specific value. In some embodiments, the term "about" refers to ±10% of the value mentioned, so that about 100 mM includes 100 mM ± 10 mM, 10% includes 10% ± 1%, etc.

As used herein, the term "effective amount" refers to a dose that is sufficient to make treatment of a patient effective as compared to no treatment.

For the convenience of the patient, it is assumed that the time intervals (time delays) from the administration of the acylated insulin of the present invention to the next administration of the acylated insulin of the present invention are preferred by the patient to have the same length or approximately the same length in days. It can even be expected that a patient will prefer that administration of the acylated insulin occur once a week, i.e. on the same day of a week, e.g., every Sunday. This would be that the acylated insulin is administered, on average over a period of 1 month, 6 months or 1 year, every 6 days and not at a higher frequency. For some patients, it may be desirable to administer the acylated insulin, on average over a period of 1 month, 6 months or 1 year, every 5 days or approximately every 5 days and not at a higher frequency. For other patients, it may be desirable to administer the acylated insulin, on average over a period of 1 month, 6 months or 1 year, every 4 days or approximately every 4 days and not at a higher frequency. For other patients, it may be desirable to administer the acylated insulin, on average over a period of 1 month, 6 months or 1 year, every 3 days or approximately every 3 days and not at a higher frequency. Other patients may even find it advantageous to administer the acylated insulin twice a week on average over a period of 1 month, 6 months or 1 year, e.g., at intervals of about 3-4 days between administrations. For some patients, it may be desirable to administer the acylated insulin, on average over a period of 1 month, 6 months or 1 year, every 2 days or approximately every 2 days and not at a higher frequency. For other patients, it may be desirable to administer the acylated insulin, on average over a period of 1 month, 6 months or 1 year, every other day or approximately every other day and not at a higher frequency. For some patients, it may be desirable to administer the acylated insulin, on average over a period of 1 month, 6 months or 1 year, every 7 days or approximately every 7 days and not at a higher frequency. Other patients may even not administer the acylated insulin at intervals of exactly the same length of time (in days) weekly, monthly or yearly. On average over a period of 1 month, 6 months or 1 year, some patients may sometimes administer the acylated insulin at intervals of 5-7 days and not at a higher frequency. On average over a period of 1 month, 6 months or 1 year, other patients may sometimes administer the acylated insulin at intervals of 4-6 days and not at a higher frequency. On average over a period of 1 month, 6 months or 1 year, other patients may even sometimes administer the acylated insulin at intervals of 3-7 days and not at a higher frequency.

Diseases and conditions that are the primary targets of the present invention are diabetes (type 1 or type 2) or other conditions characterized by hyperglycemia, but mostly metabolic diseases and conditions in which the metabolic action of insulin has clinical relevance or benefits, such as pre-diabetes, impaired glucose tolerance, metabolic syndrome, obesity, cachexia, *in vivo* β-cell damage/death, bulimia and inflammation. All of these types of conditions are known or believed to benefit from a stable metabolic state in a subject suffering from the disease or condition.

### Abbreviations

Na₂HPO₄: disodium hydrogenphosphate;
NaOH: sodium hydroxide;
OEG: amino acid residue-NH(CH₂)₂O(CH₂)₂OCH₂CO-;
OSu: succinimidyl-1-yloxy-2,5-dioxo-pyrrolidin-1-yloxy;
OtBu: oxy-tert-butyl;
HCl: hydrogen chloride;
yGlu/gGlu: yL- glutamyl (chemistry);
NHS: N-hydroxysuccinimide;
DCC: dicyclohexylcarbodiimide;
AEEA: 2-(2-(2-aminoethoxy)ethoxy)acetic acid;
OH: hydroxyl;
Gly: glycine;
Arg: arginine;
TFA: trifluoroacetic acid;
HbA1c: glycated hemoglobin;
GSP: glycated serum protein;
TG: triglyceride.

### Examples

The following examples are provided by way of illustration but not limitation.

### Control Example 1:

### A14E, B16H, B25H, B29K(N(ε)-eicosanedioyl-γGlu-2xOEG), desB30 human insulin (Control compound 1)

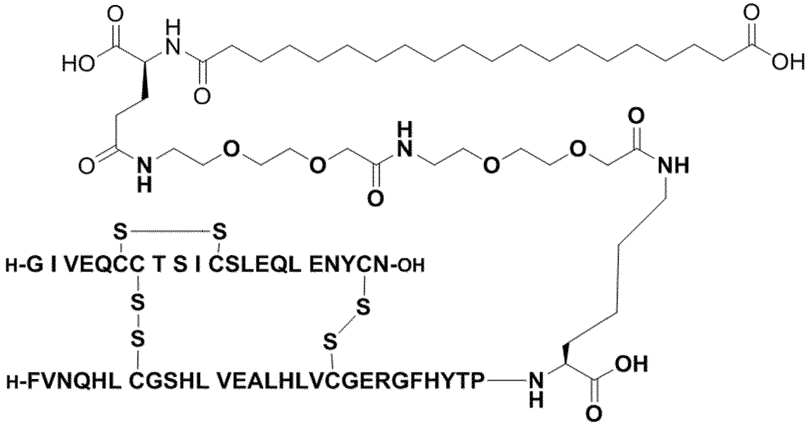

### 1. Preparation of A14E, B16H, B25H, B29K(N(ε)-eicosanedioyl-γGlu-2xOEG), desB30 human insulin

A14E, B16H, B25H, desB30 human insulin was prepared by a general preparation of insulin analogs method (for details, see Glendorf T, Sorensen AR, Nishimura E, Pettersson I, & Kjeldsen T: Importance of the Solvent-Exposed Residues of the Insulin B Chain α-Helix for Receptor Binding; Biochemistry 2008 47 4743-4751 ) A14E, B16H, B25H, desB30 human insulin (5g,0.888mmol) was dissolved in 100 mM aqueous Na₂HPO₄ solution (150 mL) and acetonitrile (100 mL) was added. The pH was adjusted to 10-12.5 with 1 N NaOH. Tert-butyl eicosanedioyl-yGlu(2xOEG-OSu)-OtBu (0.948g,0.976mmol) was dissolved in acetonitrile (50 mL), and the solution was slowly added to insulin solution. The pH value was maintained at 10-12.5. After 120 min, the reaction mixture was added to water (150 mL), and the pH value was adjusted to 5.0 with 1 N aqueous HCl. The precipitate was separated out by centrifugation and lyophilized. The crude product was added to a mixed solution of trifluoroacetic acid (60 mL) and dichloromethane (60 mL), and the mixture was stirred at room temperature for 30 min. The mixture was then concentrated to about 30 mL and poured into ice-cold n-heptane (300 mL), and the precipitated product was isolated by filtration and washed twice with n-heptane. The resulting precipitate was dried in vacuum and purified by ion exchange chromatography (Resource Q, 0.25%-1.25% ammonium acetate gradient in 42.5% ethanol, pH 7.5) and reverse phase chromatography (acetonitrile, water, TFA). The purified fractions were combined, adjusted to pH value 5.2 with 1 N HCl, and separated to obtain the precipitate, which was lyophilized to obtain the control compound 5.
LC-MS(ESI): m/z=1063.6852[M+6H]⁶⁺

### 2. Preparation of intermediate tert-butyl eicosanedioyl-γGlu-(2xOEG-OSu)-OtBu

### 2.1 Tert-butyl eicosanedioyl-OSu

Eicosanedioic acid mono-*tert*-butyl ester (20 g, 50.17 mmol) and NHS (5.77 g, 50.17 mmol) were mixed in dichloromethane under nitrogen atmosphere, and triethylamine (13.95 mL) was added. The resulting turbid mixture was stirred at room temperature, added with DCC (11.39 g, 55.19 mmol) and further stirred overnight. The reaction mixture was filtered, and the resulting filtrate was concentrated to almost dryness. The residue was mixed with cold water and ethyl acetate, and the mixture was stirred for 20 min and subjected to liquid separation. The upper organic phase was washed with saturated brine, and after liquid separation, the upper organic phase was dried over anhydrous sodium sulfate and filtered, and the filtrate was concentrated to almost dryness under reduced pressure and dried in vacuum overnight to obtain tert-butyl eicosanedioyl-OSu (24.12 g, yield 97%).
LC-MS (Scie×100API): m/z = 496.36(M+1)⁺

### 2.2 Tert-butyl eicosanedioyl-yGlu-OtBu

*Tert*-butyl eicosanedioyl-OSu (24.12 g, 48.66 mmol) was dissolved in dichloromethane (250 mL), and the solution was stirred and added with H-Glu-OtBu (10.88 g, 53.53 mmol), triethylamine (12.49 mL) and water sequentially. The mixture was heated to obtain a clarified solution, which was then stirred at room temperature for 4 h. Then, the reaction solution was added with 10% aqueous citric acid solution (200 mL) and subjected to liquid separation. The lower organic phase was washed with saturated brine, and after liquid separation, the lower organic phase was dried over anhydrous sodium sulfate and filtered, and the filtrate was concentrated to almost dryness under reduced pressure and dried in vacuum overnight to obtain *tert*-butyl eicosanedioyl-yGlu-OtBu (27.27 g, yield 96%).
LC-MS (Scie×100API): m/z = 584.44(M+1)⁺

### 2.3 Tert-butyl eicosanedioyl-yGlu-(OSu)-OtBu

*Tert*-butyl eicosanedioyl-yGlu-OtBu (27.27 g, 46.71 mmol) was dissolved in dichloromethane (300 mL) under nitrogen atmosphere, and triethylamine (11.99 mL) was added. The mixture was stirred for 10 min, and NHS (5.38 g, 50.17 mmol) was added, followed by addition of DCC (10.60 g, 51.38 mmol). The reaction mixture was stirred at room temperature overnight. The reaction mixture was filtered, and the resulting filtrate was concentrated to almost dryness. The residue was mixed with cold water and ethyl acetate, and the mixture was stirred for 20 min and subjected to liquid separation. The upper organic phase was washed with saturated brine, and after liquid separation, the upper organic phase was dried over anhydrous sodium sulfate and filtered, and the filtrate was concentrated to almost dryness under reduced pressure. *Tert*-butyl methyl ether was added, and the mixture was stirred for 30 min and filtered in vacuum. The filter cake was dried in vacuum overnight to obtain *tert*-butyl eicosanedioyl-yGlu-(OSu)-OtBu (25.76 g, yield 81%).
LC-MS (Scie×100API): m/z = 681.46(M+1)⁺

### 2.4 Tert-butyl eicosanedioyl-yGlu-(2xOEG-OH)-OtBu

*Tert*-butyl eicosanedioyl-yGlu-(OSu)-OtBu (25.76 g, 37.83 mmol) was dissolved in dichloromethane (250 mL), and the solution was stirred and added with 2× AEEA (11.66 g, 37.83 mmol), triethylamine (9.71 mL) and water (25 mL) sequentially. The mixture was heated to obtain a clear solution, which was then stirred at room temperature for 4 h. Then, the reaction solution was added with 10% aqueous citric acid solution (200 mL) and subjected to liquid separation. The lower organic phase was washed with saturated brine, and after liquid separation, the lower organic phase was dried over anhydrous sodium sulfate and filtered, and the filtrate was concentrated to almost dryness under reduced pressure and dried in vacuum overnight to obtain *tert*-butyl eicosanedioyl-γGlu-(2×OEG-OH)-OtBu (30.75 g, yield 93%).
LC-MS (Scie×100API): m/z = 874.59(M+1)⁺

### 2.5 Tert-butyl eicosanedioyl-γGlu-(2×OEG-OSu)-OtBu

*Tert*-butyl eicosanedioyl-γGlu-(2×OEG-OH)-OtBu (30.75 g, 35.18 mmol) was dissolved in dichloromethane (300 mL) under nitrogen atmosphere, and triethylamine (9.03 mL) was added. The mixture was stirred for 10 min, and NHS (4.05 g, 35.18 mmol) was added, followed by the addition of DCC (7.98 g, 38.70 mmol). The reaction mixture was stirred at room temperature overnight. The reaction mixture was filtered, and the resulting filtrate was concentrated to almost dryness. The residue was mixed with cold water and ethyl acetate, and the mixture was stirred for 20 min and subjected to liquid separation. The upper organic phase was washed with saturated brine, and after liquid separation, the upper organic phase was dried over anhydrous sodium sulfate and filtered, and the filtrate was concentrated to almost dryness under reduced pressure and dried in vacuum overnight to obtain *tert*-butyl eicosanedioyl-γGlu-(2×OEG-OSu)-OtBu (31.09 g, yield 91%).
LC-MS (Scie×100API): m/z = 971.61(M+1)⁺

### Example 1:

### A14E, B16H, B25H, B29K(N(ε)-eicosanedioyl-γGlu-12×OEG), desB30 human insulin (Compound 1)

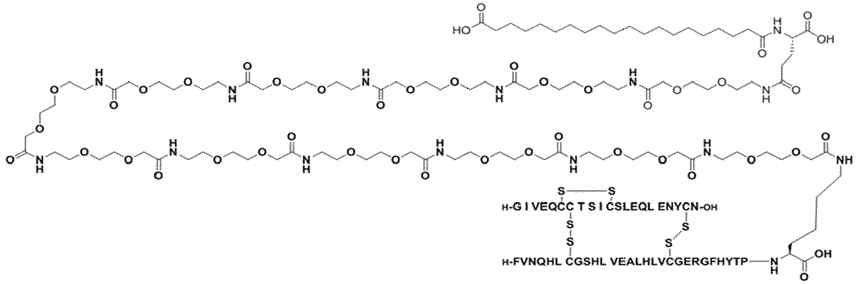

A14E, B16H, B25H, B29K (*N*(ε)-eicosanedioyl-γGlu-12xOEG), desB30 human insulin was prepared by procedures similar to those described in section 1 of Control Example 1.
LC-MS(ESI): m/z=1305.4716[M+6H]⁶⁺

The intermediate *tert*-butyl eicosanedioyl-yGlu-(12xOEG-OSu)-OtBu was prepared by procedures similar to those described in section 2 of Control Example 1.
LC-MS(Scie×100API): m/z=2423.35(M+1)⁺

### Example 2:

### A14E, B16H, B25H, B29K(N(ε)-docosanedioyl-γGlu-12×OEG), desB30 human insulin (Compound 2)

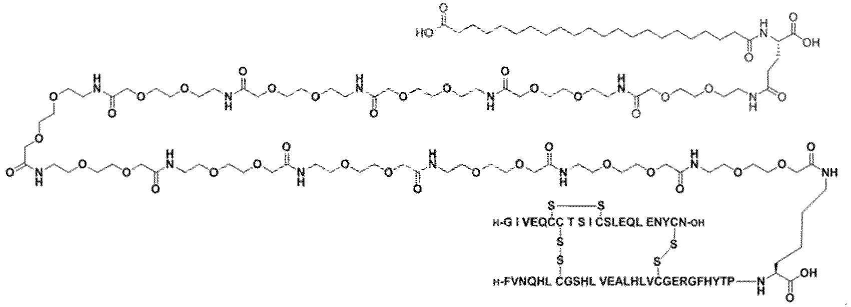

A14E, B16H, B25H, B29K(*N*(ε)-docosanedioyl-γGlu-12×OEG), desB30 human insulin was prepared by procedures similar to those described in section 1 of Control Example 1.
LC-MS(ESI): m/z= 1310.1425[M+6H]⁶⁺

The intermediate *tert*-butyl docosanedioyl-(12xOEG-OSu)-OtBu was prepared by procedures similar to those described in section 2 of Control Example 1.
LC-MS(Scie×100API): m/z=2451.38(M+1)⁺

### Example 3:

### A14E, B16H, B25H, B29K(N(ε)-docosanedioyl-γGlu-18×OEG), desB30 human insulin (Compound 3)

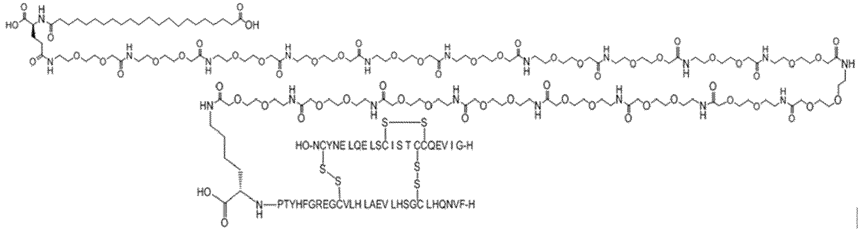

A14E, B16H, B25H, B29K(*N*(*ε*)-docosanedioyl-γGlu-18×OEG), desB30 human insulin was prepared by procedures similar to those described in section 1 of Control Example 1.
LC-MS(ESI): m/z= 1247.47[M+7H]⁷⁺

The intermediate *tert*-butyl docosanedioyl-(18xOEG-OSu)-OtBu was prepared by procedures similar to those described in section 2 of Control Example 1.
LC-MS(Scie×100API): m/z=3320.83(M+1)⁺

### Example 4

### A14E, B16H, B25H, B29K(N(ε)-docosanedioyl-γGlu-24×OEG), desB30 human insulin (Compound 4)

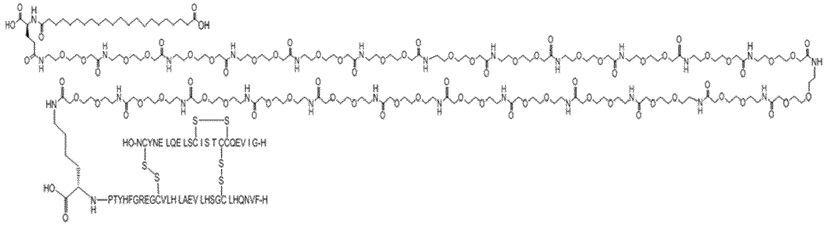

A14E, B16H, B25H, B29K(*N*(*ε*)-docosanedioyl-γGlu-24×OEG), desB30 human insulin was prepared by procedures similar to those described in section 1 of Control Example 1.
LC-MS(ESI): m/z=873.35[M+11H]¹¹⁺

The intermediate *tert*-butyl docosanedioyl-(24xOEG-OSu)-OtBu was prepared by procedures similar to those described in section 2 of Control Example 1.
LC-MS(Scie×100API): m/z=4192.27(M+1)⁺

### Example 5

### N-ε²⁶-[2-(2-[2-(2-[2-(2-[4-(19-carboxynonadecanoylamino)-4(S)-carboxybutanoylamino]ethoxy)ethoxy]acetylamino)ethoxy]ethoxy)acetyl ][Gly8, Arg34]GLP-1-(7-37) peptide (Compound 5)

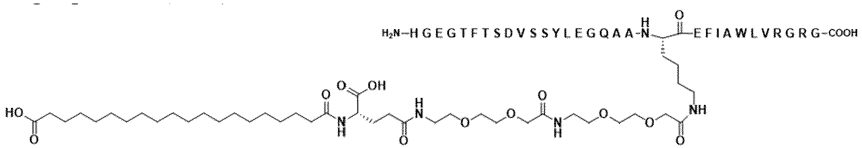

### 1. Preparation of N-ε²⁶-[2-(2-[2-(2-[2-(2-[4-(19-carboxynonadecanoylamino)-4(S)-carboxybutanoylamino]ethoxy)ethoxylacetylamino]ethoxy]ethoxy)acetyl ][Gly8, Arg34]GLP-1-(7-37) peptide

[Gly8, Arg34]GLP-1-(7-37) peptide was prepared by a general protein recombinant expression method (for details, see Molecular Cloning: A Laboratory Manual (Fourth Edition), Michael R. Green, Cold Spring Harbor Press, 2012). [Gly8, Arg34]GLP-1-(7-37) peptide (5 g, 1.48 mmol) was dissolved in 100 mM aqueous Na₂HPO₄ solution (150 mL) and acetonitrile (100 mL) was added. The pH was adjusted to 10-12.5 with 1 N NaOH. *Tert*-butyl eicosanedioyl-γGlu(2×OEG-OtBu)-OtBu (1.59 g, 1.63 mmol) was dissolved in acetonitrile (50 mL), and the solution was slowly added to a [Gly8, Arg34]GLP-1-(7-37) peptide solution. The pH was maintained at 10-12.5. After 120 min, the reaction mixture was added to water (150 mL), and the pH was adjusted to 5.0 with 1 N aqueous HCl. The precipitate was separated out by centrifugation and lyophilized. The crude product was added to a mixed solution of trifluoroacetic acid (60 mL) and dichloromethane (60 mL), and the mixture was stirred at room temperature for 30 min. The mixture was then concentrated to about 30 mL and poured into ice-cold *n*-heptane (300 mL), and the precipitated product was isolated by filtration and washed twice with *n*-heptane. The resulting precipitate was dried in vacuum and purified by ion exchange chromatography (Resource Q, 0.25%-1.25% ammonium acetate gradient in 42.5% ethanol, pH 7.5) and reverse phase chromatography (acetonitrile, water, TFA). The purified fractions were combined, adjusted to pH 5.2 with 1 N HCl, and separated to obtain the precipitate, which was lyophilized to obtain the title compound.
LC-MS (ESI): m/z = 1028.79[M+4H]⁴⁺

### 2. Preparation of intermediate tert-butyl eicosanediovl-yGlu-(2xOEG-OSu)-OtBu

### 2.1 Tert-butyl eicosanedioyl-OSu

Eicosanedioic acid mono-*tert*-butyl ester (20 g, 50.17 mmol) and NHS (5.77 g, 50.17 mmol) were mixed in dichloromethane (400mL) under nitrogen atmosphere, and triethylamine (13.95 mL) was added. The resulting turbid mixture was stirred at room temperature, added with DCC (11.39 g, 55.19 mmol) and further stirred overnight. The reaction mixture was filtered, and the resulting filtrate was concentrated to almost dryness. The residue was mixed with cold water and ethyl acetate, and the mixture was stirred for 20 min and subjected to liquid separation. The upper organic phase was washed with saturated brine, and after liquid separation, the upper organic phase was dried over anhydrous sodium sulfate and filtered, and the filtrate was concentrated to almost dryness under reduced pressure and dried in vacuum overnight to obtain *tert*-butyl eicosanedioyl-OSu (24.12 g, yield 97%).
LC-MS (Scie×100API): m/z = 496.36(M+1)⁺

### 2.2 Tert-butyl eicosanedioyl-yGlu-OtBu

*Tert*-butyl eicosanedioyl-OSu (24.12 g, 48.66 mmol) was dissolved in dichloromethane (250 mL), and the solution was stirred and added with H-Glu-OtBu (10.88 g, 53.53 mmol), triethylamine (12.49 mL) and water (25 mL) sequentially. The mixture was heated to obtain a clarified solution, which was then stirred at room temperature for 4 h. Then, the reaction solution was added with 10% aqueous citric acid solution (200 mL) and subjected to liquid separation. The lower organic phase was washed with saturated brine, and after liquid separation, the lower organic phase was dried over anhydrous sodium sulfate and filtered, and the filtrate was concentrated to almost dryness under reduced pressure and dried in vacuum overnight to obtain *tert*-butyl eicosanedioyl-yGlu-OtBu (27.27 g, yield 96%).
LC-MS (Scie×100API): m/z = 584.44(M+1)⁺

### 2.3 Tert-butyl eicosanedioyl-yGlu-(OSu)-OtBu

*Tert*-butyl eicosanedioyl-yGlu-OtBu (27.27 g, 46.71 mmol) was dissolved in dichloromethane (300 mL) under nitrogen atmosphere, and triethylamine (11.99 mL) was added. The mixture was stirred for 10 min, and NHS (5.38 g, 50.17 mmol) was added, followed by the addition of DCC (10.60 g, 51.38 mmol). The reaction mixture was stirred at room temperature overnight. The reaction mixture was filtered, and the resulting filtrate was concentrated to almost dryness. The residue was mixed with cold water and ethyl acetate, and the mixture was stirred for 20 min and subjected to liquid separation. The upper organic phase was washed with saturated brine, and after liquid separation, the upper organic phase was dried over anhydrous sodium sulfate and filtered, and the filtrate was concentrated to almost dryness under reduced pressure. *Tert*-butyl methyl ether was added, and the mixture was stirred for 30 min and filtered in vacuum. The filter cake was dried in vacuum overnight to obtain *tert*-butyl eicosanedioyl-yGlu-(OSu)-OtBu (25.76 g, yield 81%).
LC-MS (Scie×100API): m/z = 681.46(M+1)⁺

### 2.4 Tert-butyl eicosanedioyl-γGlu-(2×OEG-OH)-OtBu

*Tert*-butyl eicosanedioyl-yGlu-(OSu)-OtBu (25.76 g, 37.83 mmol) was dissolved in dichloromethane (250 mL), and the solution was stirred and added with 2×AEEA (11.66 g, 37.83 mmol), triethylamine (9.71 mL) and water (25 mL) sequentially. The mixture was heated to obtain a clarified solution, which was then stirred at room temperature for 4 h. Then, the reaction solution was added with 10% aqueous citric acid solution (200 mL) and subjected to liquid separation. The lower organic phase was washed with saturated brine, and after liquid separation, the lower organic phase was dried over anhydrous sodium sulfate and filtered, and the filtrate was concentrated to almost dryness under reduced pressure and dried in vacuum overnight to obtain *tert*-butyl eicosanedioyl-γGlu-(2×OEG-OH)-OtBu (30.75 g, yield 93%).
LC-MS (Scie×100API): m/z = 874.59(M+1)⁺

### 2.5 Tert-butyl eicosanedioyl-γGlu-(2×OEG-OSu)-OtBu

*Tert*-butyl eicosanedioyl-γGlu-(2×OEG-OH)-OtBu (30.75 g, 35.18 mmol) was dissolved in dichloromethane (300 mL) under nitrogen atmosphere, and triethylamine (9.03 mL) was added. The mixture was stirred for 10 min, and NHS (4.05 g, 35.18 mmol) was added, followed by the addition of DCC (7.98 g, 38.70 mmol). The reaction mixture was stirred at room temperature overnight. The reaction mixture was filtered, and the resulting filtrate was concentrated to almost dryness. The residue was mixed with cold water and ethyl acetate, and the mixture was stirred for 20 min and subjected to liquid separation. The upper organic phase was washed with saturated brine, and after liquid separation, the upper organic phase was dried over anhydrous sodium sulfate and filtered, and the filtrate was concentrated to almost dryness under reduced pressure and dried in vacuum overnight to obtain *tert*-butyl eicosanedioyl-γGlu-(2×OEG-OSu)-OtBu (31.09 g, yield 91%).
LC-MS (Scie×100API): m/z = 971.61(M+1)⁺

### Example 6

### N-ε²⁶-[2-(2-[2-(4-[19-carboxynonadecanoylamino]-4(S)-carboxybutanoylamino)ethoxy]ethoxy)acetyl] [Gly8, Arg34]GLP-1-(7-37) peptide (Compound 6)

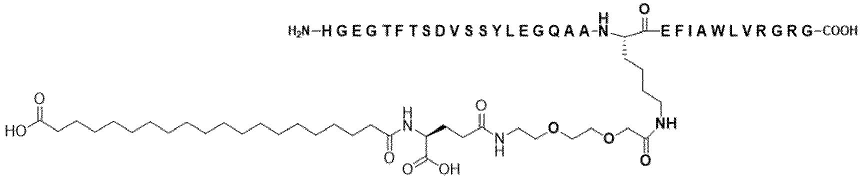

*N*-*E*²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide was prepared by procedures similar to those described in section 1 of Example 5.
LC-MS (ESI): m/z = 992.52[M+4H]⁴⁺

The intermediate *tert*-butyl eicosanedioyl-γGlu-(OEG-OSu)-OtBu was prepared by procedures similar to those described in section 2 of Example 5.
LC-MS (Scie×100API): m/z = 826.54(M+1)⁺

### Example 7

### N-ε²⁶-(19-carboxynonadecanoylamino)-4(S)-carboxybutanoyl-[Gly8, Arg34]GLP-1-(7-37) peptide (Compound 7)

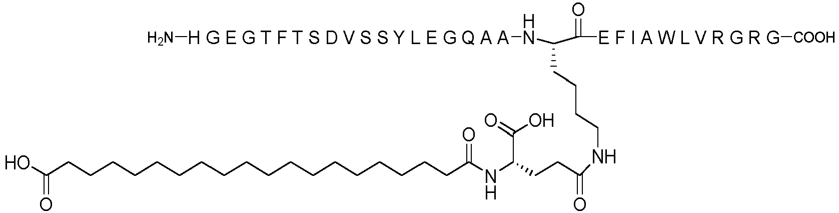

*N*-ε²⁶-(19-carboxynonadecanoylamino)-4(*S*)-carboxybutanoyl-[Gly8, Arg34]GLP-1-(7-37) peptide was prepared by procedures similar to those described in section 1 of Example 5.
LC-MS (ESI): m/z = 956.25[M+4H]⁴⁺

The intermediate *tert*-butyl eicosanediovl-yGlu-(OSu)-OtBu was prepared by procedures similar to those described in section 2 of Example 5.
LC-MS (Scie×100API): m/z = 681.46(M+1)⁺

### Example 8

### N-ε²⁶-(19-carboxynonadecanoylamino)-4(S)-carboxybutanoyl-[Arg34]GLP-1-(7-37) peptide (Compound 8)

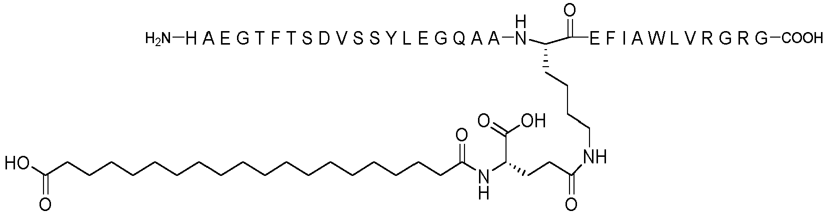

*N*-ε²⁶-19-carboxynonadecanoylamino)-4(*S*)-carboxybutanoyl-[Arg34]GLP-1-(7-37) peptide was prepared by procedures similar to those described in section 1 of Example 5.
LC-MS (ESI): m/z = 959.75[M+4H]⁴⁺

The intermediate *tert*-butyl eicosanedioyl-yGlu-(OSu)-OtBu was prepared by procedures similar to those described in section 2 of Example 5.
LC-MS (Scie×100API): m/z = 681.46(M+1)⁺

### Example 9

### N-ε²⁶-[2-(2-[2-(2-[2-(2-[4-(17-carboxyheptadecanoylamino)-4(S)-carboxybutanoylamino] ethoxy)ethoxy]acetylamino)ethoxy]ethoxy)acetyl ][Gly8, Arg34]GLP-1-(7-37) peptide (Compound 9)

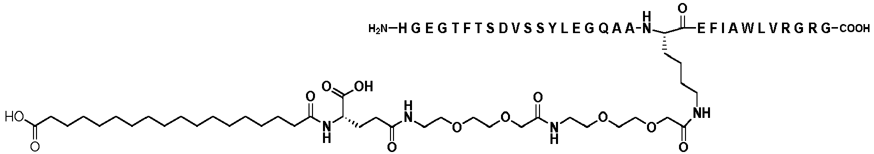

Preparation of *N*-ε²⁶-[2-(2-[2-(2-[2-(2-[4-(17-carboxyheptadecanoylamino)-4(*S*)-carboxybutanoylamino]ethoxy)ethoxylacetylamino)ethoxylethoxy)acetyl ][Gly8, Arg341GLP-1-(7-37) peptide was performed by using similar steps as in Example 5, Part 1.
LC-MS (ESI): m/z = 1021.78 [M+4H]⁴⁺

### Example 10

### N-ε²⁶-(17-carboxyheptadecanoylamino)-4(S)-carboxybutanoyl-[Gly8,Arg34]GLP-1-(7-37) peptide (Compound 10)

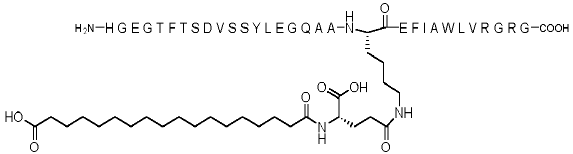

Preparation of *N*-ε²⁶-(17-carboxyheptadecanoylamino)-4(S)-carboxybutanoyl-[Gly8,Arg34]GLP-1-(7-37) peptide was performed by using similar steps as in Example 5, Part 1.
LC-MS (ESI): m/z = 949.24 [M+4H]⁴⁺

The intermediate *tert*-butyl octadecanoyl-vGlu-(OSu)-OtBu was prepared by using similar steps as in Example 5, Part 2.
LC-MS (Scie×100API): m/z = 653.43 (M+1)⁺

### Example 11

### N-ε²⁶-[2-(2-[2-(2-[2-(2-[4-(21-carboxyheneicosanoylamino)-4(S)-carboxybutanoylamino] ethoxy)ethoxy]acetylamino)ethoxy]ethoxy)acetyl ][Gly8, Arg34]GLP-1-(7-37) peptide (Compound 11)

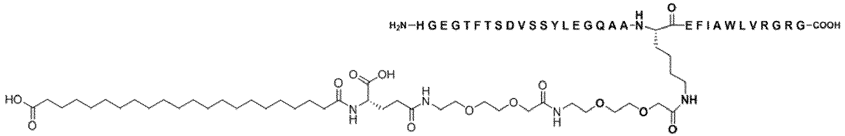

Preparation of *N-ε*²⁶-[2-(2-[2-(2-[2-(2-[4-(21-carboxyheneicosanoylamino)-4(S)-carboxybutanoylamino] ethoxy)ethoxy] acetylamino)ethoxy]ethoxy)acetyl ][Gly8, Arg34]GLP-1-(7-37) peptide was performed by using similar steps as in Example 5, Part 1.
LC-MS (ESI): m/z = 1035.80 [M+4H]⁴⁺

The Intermediate *tert*-butyl docosanedioyl-γGlu-(2×OEG-OSu)-OtBu was prepared by using similar steps as in Example 5, Part 2.
LC-MS (Scie×100API): m/z = 999.64 (M+1)⁺

### Example 12

The purpose of this experiment is to measure the chemical stability of the acylated insulin formulation of the present invention.

### Acylated insulin formulation

Compound 2 was dissolved in a 10 mM anhydrous disodium hydrogen phosphate solution to a concentration two times that of the final insulin concentration shown in the table below. Phenol, m-cresol, glycerol, and sodium chloride were mixed and added to the Compound 2 solution according to the amounts of each component shown in the table below, then the pH was adjusted to 7.4. And the zinc acetate was added to the Compound 2 solution according to the amount of zinc acetate shown in the table below, then the pH was adjusted to 7.4. The final acylated insulin formulation is described in Table 1 , and the content of Zn is represented by Zn/6 moles of acylated insulin (abbreviated as "Zn/6ins").

**Table 1**

| | Formulation 1 | Formulation 2 | Formulation 3 |
|---|---|---|---|
| Compound 2 (mmol/L) | 0.9 | 1.2 | 1.5 |
| *m*-Cresol (mmol/L) | 10 | 10 | 10 |
| Disodium hydrogen phosphate (mmol/L) | 5 | 5 | 5 |
| Glycerol (mg/ml) | 15 | 15 | 15 |
| Phenol (mmol/L) | 45 | 45 | 45 |
| Zinc ion (Zn/6ins) | 2.3 | 2.3 | 2.3 |
| NaCl (mmol/L) | 20 | 20 | 20 |
| pH value | 7.4 | 7.4 | 7.4 |

The chemical stability of the formulation in this embodiment can be represented by the changes in High Molecular Weight Protein (HMWP) relative to day 0 after storage for 14 and 21 days at 25°C and 37°C. Meanwhile, the chemical stability can also be represented by the changes in the amount of related substances after storage for 14 and 21 days at 25°C and 37°C.

### Measurement of High Molecular Weight Protein (HMWP):

The content of HMWP was determined by using High-Performance Liquid Chromatography (HPLC) on a Shodex PROTEIN kw-802.5 column. The column temperature was set at 30°C, and the sample pool temperature was maintained at 5°C. The analysis was conducted using a mobile phase with a flow rate of 0.5 ml/min, consisting of a 0.1% arginine solution (3L), ice acetic acid (750ml), and acetonitrile (1250ml). The detection wavelength is 276nm, and the injection volume is 10µl. Table 2 shows the increase in the amount of HMWP at 25 °C and 37 °C on day 14 and day 21 relative to day 0.

**Table 2**

| | 25°C | 25°C | 37°C | 37°C |
|---|---|---|---|---|
| | The increase in amount of HMWP on day 14 relative to day 0 (%) | The increase in amount of HMWP on day 21 relative to day 0 (%) | The increase in amount of HMWP on day 14 relative to day 0 (%) | The increase in amount of HMWP on day 21 relative to day 0 (%) |
| Formulation 1 | 0.18 | 0.26 | 0.72 | 0.99 |
| Formulation 2 | 0.13 | 0.18 | 0.55 | 0.73 |
| Formulation 3 | 0.09 | 0.16 | 0.51 | 0.67 |

From the table above, it can be observed that the amount of HMWP in the acylated insulin formulations of the present invention, under different concentration conditions, increases very slowly over time. This indicates that the acylated insulin formulations mentioned above exhibit excellent chemical stability.

### Measurement of the amount of Related Substance

The content of insulin-related substances was determined by HPLC using a Kromasil 300A-5µm-C4 (4.6* 150mm) column. The column temperature was set to 35°C, and the sample pool temperature was set to 5°C. The analysis was carried out using a mobile phase with a flow rate of 1.0 ml/min. The elution phase consists of mobile phase consisting of:
Phase A, containing 0.18M anhydrous sodium sulfate and 10% acetonitrile (v/v), with the pH adjusted to 2.3 by using 85% phosphoric acid; and
Phase B, consisting of 75% acetonitrile (v/v).

Gradient: a linear change 64%/36% A/B from 0 min to 40 min, a linear change to 10%/90% A/B from 40 min to 65 min, a linear change to 64%/36% A/B from 65 min to 66 min, and an isocratic gradient of 64%/36% A/B from 60 min to 70 min.

The detection wavelength was set to 214nm.

Table 3 shows the increase in the amount of the related substances at 25°C and 37°C on day 14 and day 20relative to day 0.

**Table 3**

| | 25 °C | 25 °C | 37°C | 37°C |
|---|---|---|---|---|
| | The increase in the amount of the related substances on day 14 relative to day 0 (%) | The increase in the amount of the related substances on day 21 relative to day 0 (%) | The increase in the amount of the related substances on day 14 relative to day 0 (%) | The increase in the amount of the related substances on day 21 relative to day 0 (%) |
| Formulation 1 | 0.34 | 0.45 | 1.25 | 1.93 |
| Formulation 2 | 0.43 | 0.50 | 1.23 | 1.71 |
| Formulation 3 | 0.19 | 0.30 | 0.94 | 1.45 |

From the table above, it can be observed that the amount of insulin-related substances in the acylated insulin formulations of the present invention, under different concentration conditions, also increases very slowly over time. This indicates that the acylated insulin formulations mentioned above are highly stable.

### Example 13

The purpose of this experiment is to measure the chemical stability of the acylated insulin formulation of the present invention.

Based on the amounts of the components in Table 4 below, the acylated insulin formulations in Table 4 were prepared by following similar steps as Example 12. The changes of HMWP and related substances relative to day 0 were determined on day 14 and day 35 by using procedures similar to Example 12. Table 5 and 6 show the changes of HMWP and related substances for acylated insulin formulations with different pH values.

**Table 4**

| | Formulation 4 | Formulation 5 | Formulation 6 | Formulation 2 | Formulation 7 | Formulation 8 | Formulation 9 |
|---|---|---|---|---|---|---|---|
| Compound 2 (mmol/L) | 1.2 | 1.2 | 1.2 | 1.2 | 1.2 | 1.2 | 1.2 |
| *m*-cresol(mmol/L) | 10 | 10 | 10 | 10 | 10 | 10 | 10 |
| Sodium phosphate dibasic (mmol/L) | 5 | 5 | 5 | 5 | 5 | 5 | 5 |
| Glycerol (mg/ml) | 15 | 15 | 15 | 15 | 15 | 15 | 15 |
| Phenol (mmol/L) | 45 | 45 | 45 | 45 | 45 | 45 | 45 |
| Zinc ions (Zn/6ins) | 2.3 | 2.3 | 2.3 | 2.3 | 2.3 | 2.3 | 2.3 |
| NaCl (mmol/L) | 20 | 20 | 20 | 20 | 20 | 20 | 20 |
| pH value | 6.5 | 7.0 | 7.2 | 7.4 | 7.6 | 7.8 | 8.0 |

**Table 5**

| | 25 °C | 25°C | 37°C | 37°C |
|---|---|---|---|---|
| | The increase in the amount of HMWP on day 14 relative to day 0 (%) | The increase in the amount of HMWP on day 35 relative to day 0 (%) | The increase in the amount of HMWP on day 14 relative to day 0 (%) | The increase in the amount of HMWP on day 35 relative to day 0 (%) |
| Formulation 4 | 0.10 | 0.25 | 0.48 | 1.17 |
| Formulation 5 | 0.12 | 0.26 | 0.45 | 1.16 |
| Formulation 6 | 0.17 | 0.33 | 0.50 | 1.35 |
| Formulation 2 | 0.17 | 0.36 | 0.47 | 1.28 |
| Formulation 7 | 0.13 | 0.32 | 0.51 | 1.40 |
| Formulation 8 | 0.16 | 0.41 | 0.63 | 1.50 |
| Formulation 9 | 0.19 | 0.49 | 0.69 | 1.78 |

**Table 6**

| | 25 °C | 25 °C | 37°C | 37°C |
|---|---|---|---|---|
| | The increase in the amount of related substances on day 14 relative to day 0 (%) | The increase in the amount of related substances on day 35 relative to day 0 (%) | The increase in the amount of related substances on day 14 relative to day 0 (%) | The increase in the amount of related substances on day 35 relative to day 0 (%) |
| Formulation 4 | 0.49 | 0.70 | 1.41 | 3.27 |
| Formulation 5 | 0.38 | 0.68 | 1.24 | 2.91 |
| Formulation 6 | 0.43 | 0.75 | 1.22 | 2.99 |
| Formulation 2 | 0.44 | 0.74 | 1.25 | 3.16 |
| Formulation 7 | 0.40 | 0.75 | 1.34 | 3.42 |
| Formulation 8 | 0.36 | 0.86 | 1.55 | 3.93 |
| Formulation 9 | 0.43 | 0.83 | 2.02 | 4.85 |

From the tables above, it can be observed that the amount of HMWP and related substances in the acylated insulin formulations of the present invention increases very slowly over time, indicating that the acylated insulin formulations mentioned above are highly stable.

### Example 14

The purpose of this experiment is to measure the chemical stability of the combination of acylated insulin and GLP-1 compound of the present invention.

### Composition of acylated insulin and GLP-1 compound

Based on the amounts of the components in Table 7 below, the composition containing acylated insulin (compound 2) and GLP-1 (compound 11) were prepared by following similar steps as Example 12. The changes of HMWP of acylated insulin in the composition were determined on day 14 and day 35 by using procedures similar to Example 12. Table 8 shows the changes of HMWP of acylated insulin in the compositions with different formulations.

**Table 7**

| | Composition 1 | Composition 2 | Composition 3 | Composition 4 | Composition 5 | Composition 6 | Composition 7 | Composition 8 |
|---|---|---|---|---|---|---|---|---|
| Compound 2 (mmol/L) | 1.2 | 1.2 | 1.2 | 1.2 | 1.2 | 1.2 | 1.2 | 1.2 |
| Compound 11 (mmol/L) | 0.096 | 0.12 | 0.16 | 0.192 | 0.24 | 0.32 | 0.48 | 0.64 |
| *m*-cresol (mmol/L) | 10 | 10 | 10 | 10 | 10 | 10 | 10 | 10 |
| Sodium phosphate dibasic (mmol/L) | 10 | 10 | 10 | 10 | 10 | 10 | 10 | 10 |
| Glycerol (mg/ml) | 15 | 15 | 15 | 15 | 15 | 15 | 15 | 15 |
| Phenol (mmol/L) | 45 | 45 | 45 | 45 | 45 | 45 | 45 | 45 |
| Zinc ions (Zn/6ins) | 2.3 | 2.3 | 2.3 | 2.3 | 2.3 | 2.3 | 2.3 | 2.3 |
| NaCl (mmol/L) | 20 | 20 | 20 | 20 | 20 | 20 | 20 | 20 |
| pH value | 7.4 | 7.4 | 7.4 | 7.4 | 7.4 | 7.4 | 7.4 | 7.4 |

**Table 8**

| | 25 °C | 25 °C | 37°C | 37°C |
|---|---|---|---|---|
| | The increase in the amount of HMWP on day 14 relative to day 0 (%) | The increase in the amount of HMWP on day 35 relative to day 0 (%) | The increase in the amount of HMWP on day 14 relative to day 0 (%) | The increase in the amount of HMWP on day 35 relative to day 0 (%) |
| Composition 1 | 0.23 | 0.36 | 0.45 | 1.31 |
| Composition 2 | 0.16 | 0.32 | 0.40 | 1.20 |
| Composition 3 | 0.17 | 0.32 | 0.40 | 1.12 |
| Composition 4 | 0.13 | 0.30 | 0.50 | 1.07 |
| Composition 5 | 0.11 | 0.16 | 0.33 | 0.91 |
| Composition 6 | 0.13 | 0.26 | 0.29 | 0.84 |
| Composition 7 | 0.07 | 0.16 | 0.13 | 0.39 |
| Composition 8 | 0.06 | 0.13 | 0.19 | 0.53 |

From the above table, it can be seen that the amount of HMWP (high molecular weight protein) of acylated insulin in the composition of acylated insulin and GLP-1 (glucagon-like peptide 1) of the present invention increases very slowly over time, indicating that the presence of GLP-1 compound does not affect the stability of acylated insulin.

### Example 15

### Study of pharmacodynamics in type 2 diabetic db/db mice

The purpose of this study is to demonstrate the long-term hypoglycemic effects and HbA1c-lowering effects of the composition containing acylated insulin and GLP-1 compound of the present invention in type2 diabetes db/db mice in the case of diabetes.in type.

In type 2 diabetes db/db mice, combinations 1-3 and 5-7 containing compounds 2 and 11, mono-formulation 10 containing compound 2 as reference formulations, mono-formulation 1 containing compound 11 and vehicle group were tested..

The components of the formulations or compositions are shown in Table 9.

**Table 9**

| | Composition 1 | Composition 2 | Composition 3 | Composition 5 | Composition 6 | Composition 7 |
|---|---|---|---|---|---|---|
| Compound 2 | 1.2mmol/L (200U) | 1.2mmol/L (200U) | 1.2mmol/L (200U) | 1.2mmol/L (200U) | 1.2mmol/L (200U) | 1.2mmol/L (200U) |
| Compound 11 | 0.096mmol/L (0.4mg/ml) | 0.12mmol/L (0.5mg/ml) | 0.16mmol/L. (0.7mg/ml) | 0.24mmol/L (1.0mg/ml) | 0.32mmol/L (1.35mg/ml) | 0.48mmol/L (2.0mg/ml) |
| *m*-cresol (mmol/L) | 10 | 10 | 10 | 10 | 10 | 10 |
| Sodium phosphate dibasic (mmol/L) | 10 | 10 | 10 | 10 | 10 | 10 |
| Glycerol (mg/ml) | 15 | 15 | 15 | 15 | 15 | 15 |
| Phenol (mmol/L) | 45 | 45 | 45 | 45 | 45 | 45 |
| Zinc ions (Zn/6ins) | 2.3 | 2.3 | 2.3 | 2.3 | 2.3 | 2.3 |
| NaCl (mmol/L) | 20 | 20 | 20 | 20 | 20 | 20 |
| Propylene glycol (mmol/L) | - | - | - | - | - | - |
| pH value (mmol/L) | 7.4 | 7.4 | 7.4 | 7.4 | 7.4 | 7.4 |

**Table 9-continued**

| | Formulation 10 | Formulation 11 | Vehicle group |
|---|---|---|---|
| Compound 2 | 1.2 mmol/L(200U) | - | - |
| Compound 11 | - | 0.48mmol/L (2.0mg/ml) | - |
| *m*-cresol(mmol/L) | 10 | - | 10 |
| Sodium phosphate dibasic (mmol/L) | 5 | 10 | 5 |
| Glycerol (mg/ml) | 15 | - | 15 |
| Phenol (mmol/L) | 45 | 60 | 45 |
| Zinc ions (Zn/6ins) | 2.3 | - | - |
| NaCl(mmol/L) | 20 | - | 20 |
| Propylene glycol (mmol/L) | - | 184 | - |
| pH value (mmol/L) | 7.4 | 7.3 | 7.4 |

Male db/db (Cavins) mice aged 8-9 weeks were raised in a barrier environment in appropriate-sized cages with free access to standard food and purified water. The environmental conditions were controlled at a relative humidity of 40%-60% and a temperature of 22°C-24°C. After an adaptation period of 1-2 weeks, the mice were used for the experiments. On the day of the experiment, random blood glucose levels were evaluated, and the mice were weighed. Based on random blood glucose and body weight, the mice were randomly assigned to the vehicle group or treatment groups. There were a total of 13 groups, with 5 mice in each group, and they received the following treatments: subcutaneous injection of the vehicle, or subcutaneous injection of low dose (5U/kg) of the drug combinations 1, 2, 5, 6, 7 containing compound 2, or subcutaneous injection of medium dose (10U/kg) of the drug combinations 1, 3, 5, 6, 7 containing compound 2, or subcutaneous injection of formulation 10 (containing compound 2 at 10U/kg), or subcutaneous injection of formulation 11 (containing compound 11 at a calculated dose of 96.8pg/kg). The drugs were administered subcutaneously (5 ml/kg) by injection on the back of the neck. The mice were treated on day 0, 3, 6, 9, 12, 15, 18, and 21, and random blood glucose levels were measured at 3, 6, 9, 24, 48, and 72 hours after the first injection, followed by daily monitoring once. At the end of the study, whole blood was collected using EDTA as an anticoagulant for the measurement of glycated hemoglobin (HbA1c) percentage.

The tail of the mouse was cleaned with an alcohol swab, and a disposable blood collection needle was used to collect blood drop from the tail. Blood glucose levels were measured by using a glucose meter and test strips (Roche). Dose response curves of blood glucose-time were plotted for each drug combination. To illustrate the effect of the drug combinationson blood glucose of present invention, the area difference under the curve (ΔAUC) of the blood glucose-time curve from 0 to the end of the monitoring period was calculated for each individual dose-response curve. A smaller ΔAUC value indicates better hypoglycemic effect and better efficacy.

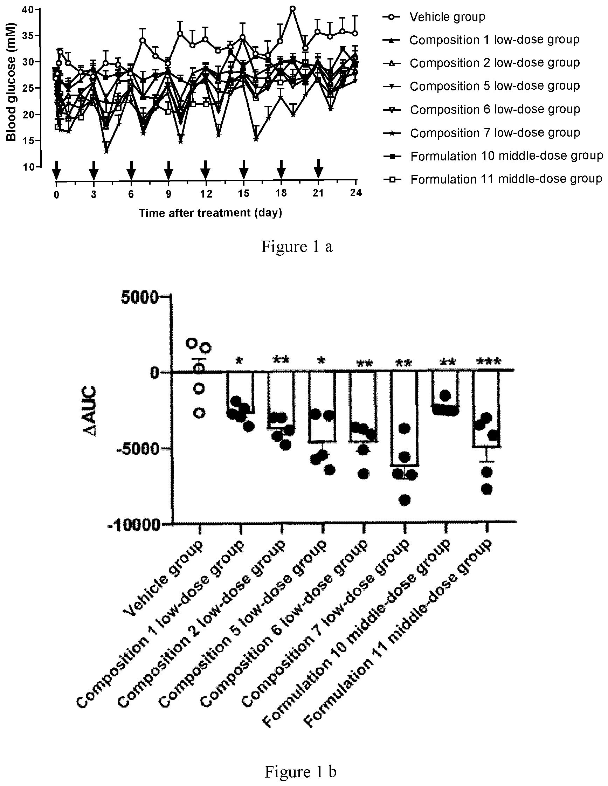

Figures 1a-1f show the unexpected synergistic hypoglycemic effect and reduction in HbA1c in type 2 diabetic db/db mice after administration of the drug combinations containing acylated insulin and GLP-1 compound of the present invention compared to acylated insulin alone and GLP-1 alone.

Specifically, Figures 1a and 1b show that the hypoglycemic effect of the drug composition 7 containing acylated insulin and GLP-1 compound at a low dose is significantly superior to formulation 10 and formulation 11 containing acylated insulin alone and GLP-1 alone, respectively. The amount of acylated insulin in formulation 10 is equivalent to twice the amount of acylated insulin in combination 7 at the low dose, and the amount of GLP-1 compound in formulation 11 is equivalent to twice the amount of GLP-1 compound in combination 7 at the low dose, demonstrating the unexpected synergistic hypoglycemic effect of the acylated insulin and GLP-1 compound in the drug combination of the present invention. Composition 1 and composition 2 at the low dose have comparable or better hypoglycemic effects than formulation 10, and composition 5 and composition 6 at the low dose have comparable or better hypoglycemic effects than formulation 11, indicating that, in the presence of synergistic effects between acylated insulin and GLP-1 compound, equivalent or better hypoglycemic effects can be achieved with lower total amounts of acylated insulin and GLP-1 compound.

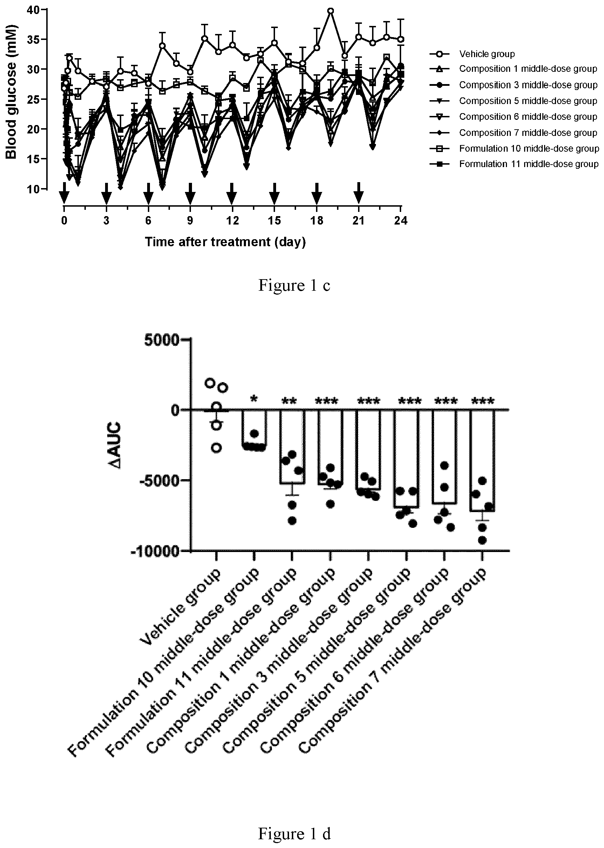

Figures 1c and 1d show that, in the presence of synergistic hypoglycemic effects between acylated insulin and GLP-1 compound, the addition of a small amount of GLP-1 compound to the drug combination with the same dose of acylated insulin results in significantly improved hypoglycemic effects compared to the drug combination containing acylated insulin alone. Composition 1, composition 3, composition 5, composition 6, and composition 7 at the medium dose have comparable or better hypoglycemic effects than formulation 11.

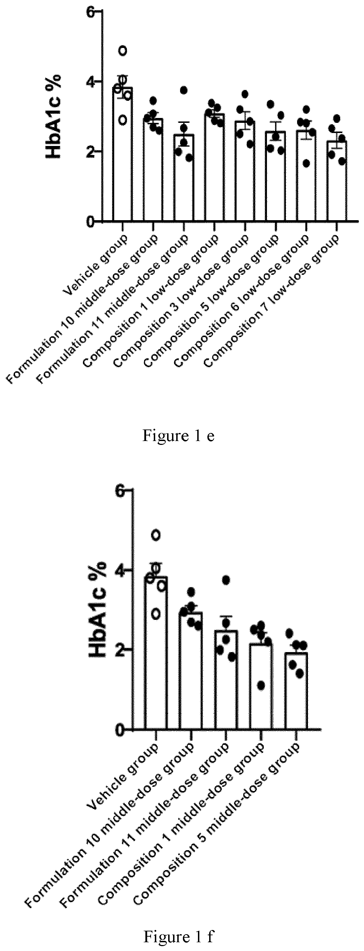

Figure 1e shows that composition 7 at the low dose has a better effect in reducing HbA1c compared to formulation 10 and formulation 11, indicating the unexpected synergistic effect in reducing HbA1c of the acylated insulin and GLP-1 compound.

Figure 1f shows that, in the presence of synergistic reduction in HbA1c effects between acylated insulin and GLP-1 compound, the addition of a small amount of GLP-1 compound to the drug combination with the same dose of acylated insulin results in significantly improved reduction in HbA1c compared to the drug combination containing acylated insulin alone or GLP-1 alone.

### Example 16

Long-term study of pharmacodynamics in Type 2 Diabetic db/db Mice Refer to similar experimental procedure as described in Example 15, a long-term study of pharmacodynamics was conducted in type 2 diabetic db/db mice.

The compositions 5, 9, and 10, containing compound 2 and compound 11, were tested on db/db mice with type 2 diabetes. In addition, reference formulations including formulation 10 containing compound 2, formulation 11 containing compound 11, the dulaglutide composition, and the vehicle group were also tested.

Among these formulations or compounds, dulaglutide composition served as the control composition, which was purchased from Novo Nordisk under the name of Dulaglutide insulin liraglutide injection (Trade name: Xultophy). The specific compositions of the remaining formulations or compounds are shown in Table 10.

**Table 10**

| | Composition 5 | Composition 9 | Composition 10 | FormulationlO | Formulation 11 | Vehicle group |
|---|---|---|---|---|---|---|
| Compound 2 | 1.2mmol/L (200U) | 1.2mmol/L (200U) | 1.2mmol/L (200U) | 1.2mmol/L (200U) | - | - |
| Compound 11 | 0.24mmol/L (1.0mg/ml) | 0.42mmol/L (1.75mg/ml) | 0.60mmol/L (2.5mg/ml) | - | 0.48mmol/L (2.0mg/ml) | - |
| *m*-cresol (mmol/L) | 10 | 10 | 10 | 10 | - | 10 |
| Sodium phosphate dibasic (mmol/L) | 10 | 10 | 10 | 5 | 10 | 5 |
| Glycerol (mg/ml) | 15 | 15 | 15 | 15 | - | 15 |
| Phenol (mmol/L) | 45 | 45 | 45 | 45 | 60 | 45 |
| Zinc ions (Zn/6ins) | 2.3 | 2.3 | 2.3 | 2.3 | - | - |
| NaCl (mmol/L) | 20 | 20 | 20 | 20 | - | 20 |
| Propylene glycol (mmol/L) | - | - | - | - | 184 | - |
| pH value | 7.4 | 7.4 | 7.4 | 7.4 | 7.3 | 7.4 |

8-9 week-old male db/db (Cavens) mice were raised in a barrier environment in appropriate-sized cages with free access to standard food and purified water. The environmental conditions were controlled at a relative humidity of 40%-60% and a temperature of 22°C-24°C. After an adaptation period of 1-2 weeks, the mice were used for the experiment.

Before the day of the experiment, random blood glucose levels were evaluated, and the mice were weighed. Based on random blood glucose levels and body weight, the mice were randomly assigned to the vehicle group or the treatment group. There were a total of 14 groups, with 7 mice in each group, and they received the following treatments: subcutaneous injection of the vehicle, low-dose of drug composition 5, drug composition 9, drug composition 10, medium-dose drug composition 5, medium-dose drug composition 9, medium-dose of drug composition 10, high-dose of drug composition 5, high-dose drug composition 9, high-dose of drug composition 10, medium-dose formulation 10, high-dose of formulation 10, high-dose of formulation 11, or high-dose of degludec liraglutide composition. In the low-dose group, the dosages were calculated based on 5U/kg of acylated insulin. In the medium-dose group, the dosages were calculated based on 10U/kg of acylated insulin. In the high-dose group, the dosages of acylated insulin in composition 5, composition 9, composition 10, and formulation 10 were calculated as 20U/kg, and the dosage of dulaglutide insulin in the dulaglutide combination was 7U/kg, while the dosage of GLP-1 compound in formulation 11 was 250µg/kg. For the second to eleventh administrations, the dosages of acylated insulin in composition 5, composition 9, composition 10, and formulation 10 were calculated as 15U/kg, the dosage of dulaglutide insulin in the dulaglutide combination was 5.25U/kg, and the dosage of GLP-1 compound in formulation 11 was 189µg/kg.

The drugs were administered subcutaneously (5ml/kg body weight) with injections given at the nape of the neck. The vehicle and degludec liraglutide composition were administered once daily for a total of 33 doses. The drug composition 5, 9, and 10, as well as formulation 10 and formulation 11, were administered on day 0, 3, 6, 9, 12, 15, 18, 21, 24, 27, and 30. Blood glucose levels were assessed at 3, 6, 9, 12, 24, and 48 hours after the first administration and then once daily from day 3 to day 33 before the drug administered. The area under the curve (AUC) of blood glucose levels over time was calculated. Prior to grouping and on the 16th day after administration, glycated hemoglobin (Hb1Ac) percentages in mice were measured to calculate the change in glycated hemoglobin (△ Hb1Ac). At the end of the experiment, non-anticoagulated whole blood was collected from the mice, and after centrifugation, serum samples were collected and triglyceride (TG) levels were measured by using an ELISA kit.

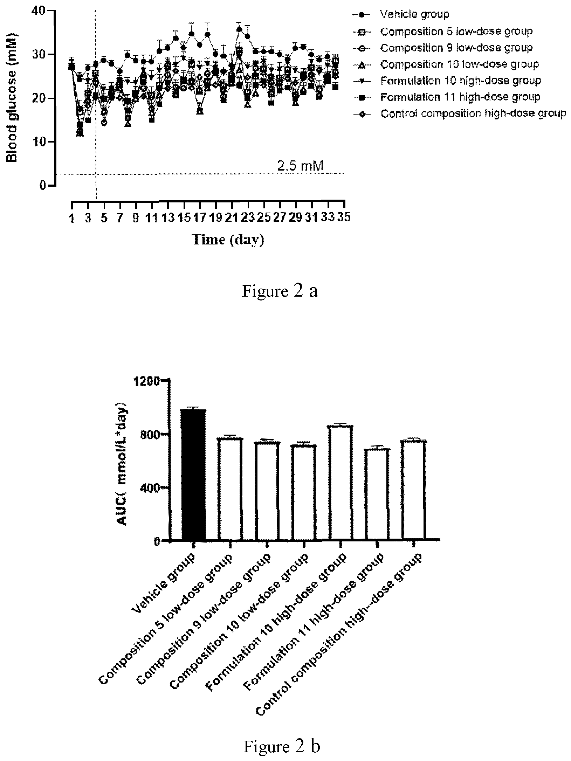

Figures 2a-2d demonstrate the unexpected synergistic effects of the drug compositions containing acylated insulin and GLP-1 compound in lowering blood glucose levels, reducing HblAc, and decreasing triglyceride levels in type 2 diabetic db/db mice compared to acylated insulin alone or GLP-1 alone. Furthermore, the drug compositions of the present invention administered every three days showed superior efficacy compared to the once-daily high-dose composition consisting of insulin degludec and liraglutide.

Specifically, Figures 2a and 2b show that at low doses, the drug compositions 5, 9, and 10 containing acylated insulin and GLP-1 compound exhibited comparable blood glucose-lowering effects to the high-dose formulation 10. The amount of acylated insulin in formulation 10 was more than three times that in the low-dose drug composition 5, indicating that lower doses of acylated insulin combined with a small amount of GLP-1 compound can achieve comparable or better blood glucose-lowering effects than higher doses of acylated insulin alone. The blood glucose-lowering effect of the low-dose drug composition 9 administered every three days was equivalent to that of the once-daily high-dose control composition (composition consisting of insulin degludec and liraglutide), demonstrating the superior blood glucose-lowering effects of the drug combinations containing acylated insulin and GLP-1 compound compared to the marketed combination product composition consisting of insulin degludec and liraglutide. The blood glucose-lowering effect of the low-dose drug combination 10 was significantly superior to that of the high-dose formulation 10 and comparable to that of the high-dose formulation 11. The amount of acylated insulin in formulation 11 was more than three times that in the high-dose drug composition 10, and the amount of GLP-1 compound in formulation 11 was more than three times that in the high-dose drug composition 10. This indicates the synergistic blood glucose-lowering effects of the acylated insulin and GLP-1 compound in the drug compositions of the present invention.

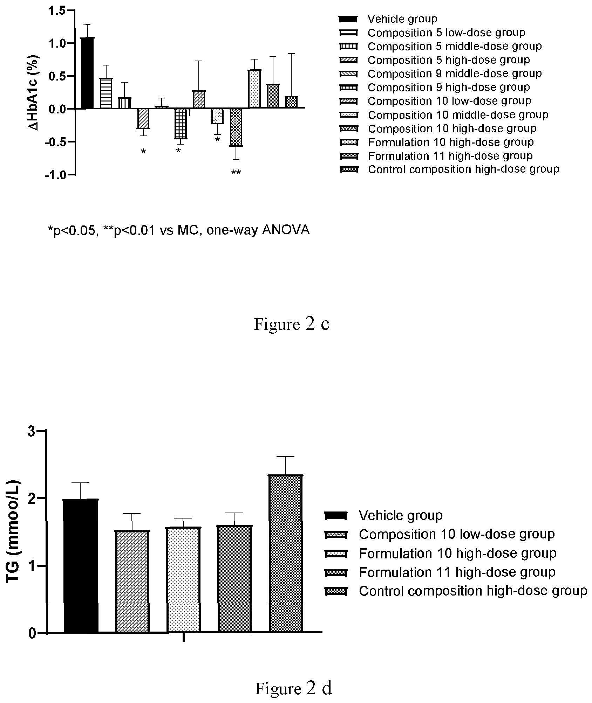

Figure 2c shows that the HbA1c-lowering effect of the medium-dose drug composition 5 administered every three days is comparable to that of the once-daily high-dose degludec liraglutide composition. This demonstrates that the drug compositionof the present invention can achieve HbA1c-lowering effects equivalent to the high-dose composition consisting of insulin degludec and liraglutide with a lower dosage, indicating the superior efficacy of the drug composition of the present invention. The HbA1c-lowering effect of the low-dose drug composition 10 is significantly superior to that of the high-dose formulation 10 and formulation 11, indicating the synergistic HbA1c-lowering effect of the acylated insulin and GLP-1 compound in the drug composition of the present invention.

Figure 2d shows that the TG-lowering effect of the low-dose drug composition 10 is significantly superior to that of the high-dose composition consisting of insulin degludec and liraglutide, indicating that the triglyceride-lowering effect of the drug composition of the present invention is superior to that of the combination consisting of insulin degludec and liraglutide. The TG-lowering effect of the low-dose drug composition 10 is comparable to that of the high-dose formulation 10 and high-dose formulation 11, suggesting the synergistic triglyceride-lowering effect of the acylated insulin and GLP-1 compound in the drug composition of the present invention.

### Example 17

Long-term study of pharmacodynamics in type 2 diabetic db/db mice
The purpose of this study is to confirm the long-term blood glucose-lowering, HbA1c-lowering, and TG-lowering effects of the acylated insulin formulation of the present invention in type 2 diabetic db/db mice. The study tested formulations containing compound 2 and reference formulations containing control compound 1, degludec formulation, a normal control group, and a vehicle control group in type 2 diabetic db/db mice.

The degludec formulation served as the controlformulation 2 and was purchased from Novo Nordisk under the trade name "Tresiba", The specific compositions of the remaining formulations are shown in Table 11.

**Table 11**

| | Formulation 2 | Control formulation 1 | Vehicle |
|---|---|---|---|
| Compound 2 | 1.2mmol/L (200U) | - | - |
| Control compound 1 | - | 4.2 mmol/L(700U) | - |
| *m*-cresol(mmol/L) | 10 | 10 | 10 |
| Sodium phosphate dibasic (mmol/L) | 5 | - | 5 |
| Glycerol (mg/ml) | 15 | 15 | 15 |
| Phenol (mmol/L) | 45 | 58.5 | 45 |
| Zinc ions (Zn/6ins) | 2.3 | 2.2 | - |
| NaCl(mmol/L) | 20 | 20 | 20 |
| pH value | 7.4 | 7.4 | 7.4 |

8-9-week-old db/db (Cavines) mice and normal mice were raised in barrier environments in appropriate-sized cages, with free access to standard food and purified water. The environmental conditions were maintained at a relative humidity of 40%-60% and a temperature of 22°C-24°C. After an adaptation period of 1-2 weeks, the mice were used for the experiments.

Before the day of the experiment, random blood glucose levels were assessed, and the mice were weighed. Based on the random blood glucose levels and body weight, the mice were randomly assigned to control groups or treatment groups. There were a total of 4 groups, with 10 mice in each group, and they received the following treatments: subcutaneous injection of the vehicle, subcutaneous injection of formulation 2 containing compound 2, subcutaneous injection of control formulation 1 containing the control compound 1, or subcutaneous injection of the control formulation 2 containing insulin degludec. The dose administered for the 1st to 4th injections was 30 U/kg, and for the subsequent 5th to 10th injections, the dose was 37.5 U/kg.

Subcutaneous administration (5 ml/kg body weight) was performed via injections in the nape of the neck. The injections were given on day 0, 3, 7, 10, 13, 16, 19, 22, 25, and 28. Blood glucose levels were evaluated before each administration and 72 hours after the final administration, and the changes in the area under the blood glucose-time curve (ΔAUC) were calculated. To simulate a meal, an oral glucose tolerance test (OGTT) was conducted starting 48 hours after the first administration. During the OGTT, the mice were orally administered glucose solutions (100 mg/mL, 10 mL/kg), and blood glucose levels were measured at 30 minutes, 60 minutes, and 120 minutes after administration. At the end of the study, EDTA-anticoagulated whole blood was collected to measure the percentage of glycated hemoglobin (HbA1c), and non-anticoagulated whole blood was collected for serum collection. The serum was separated by centrifugation, and triglyceride (TG) levels were measured by using an ELISA kit. Figures 3a-3f show that at the same dosage, the acylated insulin formulation of the present invention has unexpectedly improved blood glucose-lowering, HbA1c-lowering, and TG-lowering effects in type 2 diabetic db/db mice compared to the degludec formulation and the control formulation containing the control compound 1.

### Example 18

### Long-term study of pharmacodynamics in early-stage non-obese type 2 diabetic (T2DM) model rats (GK rats)

The purpose of this study is to confirm the long-term blood glucose-lowering, HbA1c-lowering, TG-lowering, and GSP-lowering effects of the acylated insulin formulation of the present invention in early-stage non-obese type 2 diabetic (T2DM) model rats (GK rats).

The study tested a formulation containing compound 2, as well as a reference degludec formulation, a normal control group, and a vehicle control group in GK rats. The compositions of the formulations are shown in Table 12.

Qualified GK rats after screened (with random blood glucose and glycosylated hemoglobin levels 20% and 30% higher than normal Wistar rats) and normal rats raisedin barrier environments in appropriate-sized cages, with free access to standard food and purified water. The environmental conditions were maintained at a relative humidity of 40%-60% and a temperature of 22°C-24°C. After an adaptation period of 1-2 weeks, the mice were used for the experiments.

Before the day of the experiment, random blood glucose levels were assessed, and GK rats were weighed. Based on random blood glucose, body weight, and glycosylated hemoglobin levels, the rats were randomly assigned to control or treatment groups. There were a total of 3 groups, with 6 rats in each group, and they received the following treatments: subcutaneous injection of vehicle, subcutaneous injection of formulation 2 containing compound 2, or subcutaneous injection of control formulation 2 containing dulaglutide. The dosages for all treatments were 10U/kg.

Subcutaneous administration (Iml/kg body weight) was performed by injecting the solutions into the nape of the neck (S.C.). The vehicle and dulaglutide formulations were administered once daily for a total of 32 doses. Formulation 2 containing compound 2 was administered on days 0, 4, 8, 12, 16, 20, 24, and 28. Blood glucose levels were evaluated before each administration and 72 hours after the last administration, and the change of area under the blood glucose-time curve (ΔAUC) was calculated. The rats were fasted for 4 hours prior to the experiment to measure glycosylated hemoglobin (HbA1c). At the end of the study, whole blood was collected by using EDTA anticoagulant to measure the percentage of glycosylated hemoglobin (Hb1Ac). Non-anticoagulated whole blood was also collected, centrifuged to obtain serum, and ELISA assay kits were used to measure glycosylated serum protein (GSP) and triglyceride (TG) levels.

Figures 4a-4e demonstrate that, at the same dosage, the formulation containing acylated insulin of the present invention exhibits unexpectedly improved effects in reducing blood glucose, lowering Hb1Ac, and decreasing GSP and TG levels in early-stage non-obese type II diabetes mellitus (T2DM) model rats compared to the dulaglutide formulation.

### Example 19

The purpose of this experiment is to measure the chemical stability of the combination consisting of acylated insulin and GLP-1 compound of the present invention.

According to the amounts of the components listed in Table 12 below, the combinations specified in Table 12 were prepared following a similar procedure as described in Example 12. The changes in HMWP on day 19 and day 33 relative to day 0, as well as the changes in related substances on day 19 relative to day 0, were determined using a procedure similar to that described in Example 12. Table 13 and Table 14 show the changes in HMWP and related substances of compound 2 in different compositions.

**Table 12**

| Compositions | Composition 11 | Composition 12 | Composition 13 | Composition 14 | Composition 15 | Composition 16 |
|---|---|---|---|---|---|---|
| Compound 2(mmol/L) | 1.5 | 1.5 | 1.5 | 1.5 | 1.5 | 1.5 |
| Compound11(mg/ml) | 1.5 | 2.0 | 2.5 | 3.0 | 2.0 | 2.0 |
| Phenol (mmol/L) | 40 | 40 | 40 | 40 | 58.5 | 58.5 |
| *m*-Cresol (mmol/L) | 10 | 10 | 10 | 10 | 10 | - |
| Zinc ions (Zn/6ins) | 3 | 3 | 3 | 3 | 3 | 3 |
| Glycerol(mg/ml) | 15 | 15 | 15 | 15 | 15 | - |
| NaCl(mmol/L) | 20 | 20 | 20 | 20 | 20 | 20 |
| propylene glycol (mmol/L) | - | - | - | - | - | 184 |
| Disodium phosphate (mmol/L) | - | - | - | - | - | 9 |
| pH value | 7.4 | 7.4 | 7.4 | 7.4 | 7.4 | 7.4 |

**Table 13**

| | 25°C | 25°C | 37°C | 37°C |
|---|---|---|---|---|
| | The increase in the amount of HMWP on day 19 relative to day 0 (%) | The increase in the amount of HMWP on day 33 relative to day 0 (%) | The increase in the amount of HMWP on day 19 relative to day 0 (%) | The increase in the amount of HMWP on day 33 relative to day 0 (%) |
| Composition 11 | 0.17 | 0.30 | 0.68 | 1.32 |
| Composition 12 | 0.11 | 0.21 | 0.51 | 1.19 |
| Composition 13 | 0.11 | 0.20 | 0.39 | 0.96 |
| Composition 14 | 0.09 | 0.19 | 0.38 | 0.83 |
| Composition 15 | 0.14 | 0.28 | 0.55 | 1.06 |
| Composition 16 | 0.10 | 0.23 | 0.41 | 0.72 |

**Table 14**

| | 25 °C | 37°C |
|---|---|---|
| | The increase in the amount of related substance on day 19 relative to day 0 (%) | The increase in the amount of related substance on day 19 relative to day 0 (%) |
| Composition 11 | 2.14 | 3.9 |
| Composition 12 | 2.65 | 4.14 |
| Composition 13 | 2.16 | 3.63 |
| Composition 14 | 2.81 | 3.2 |
| Composition 15 | 2.08 | 3.53 |
| Composition 16 | 1.91 | 2.98 |

From the above table, it can be seen that the HMWP of acylated insulin and the related substance in the acylated insulin and GLP-1 composition of the present invention increase very slowly over time, indicating that the presence of the GLP-1 compound does not affect the stability of the acylated insulin.

### Example 20

### A14E, B16H, B25H, B29K(N(ε)-docosanedioyl-γGlu-6xOEG), desB30 human insulin (Compound 12)

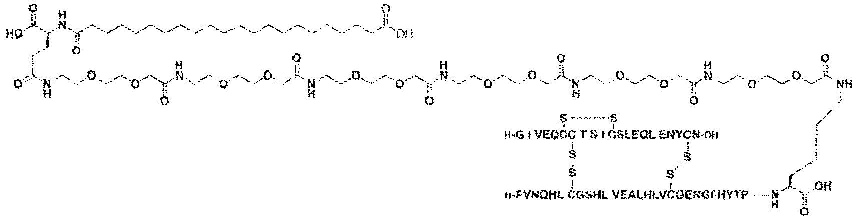

Preparation of compound A14E, B16H, B25H, B29K (*N(ε)-*docosanedioyl-yGlu-6xOEG), desB30 human insulin was conducted by following the procedure similar to Part 1 of Control Example 1.
LC-MS (ESI): m/z = 1165.0674 [M+11H]⁶⁺

Preparation of the intermediate t-butyl docosanedioyl-yGlu-(6xOEG-OSu)-OtBu was carried out by using the procedure similar to Part 2 of Control Example 1.
LC-MS (Scie×100API): m/z = 1579.94 (M+1)⁺

### Example 21

### A14E, B16H, B25H, B29K (N(ε)-eicosanedioyl-γGlu-6xOEG), desB30 human insulin (Compound 13)

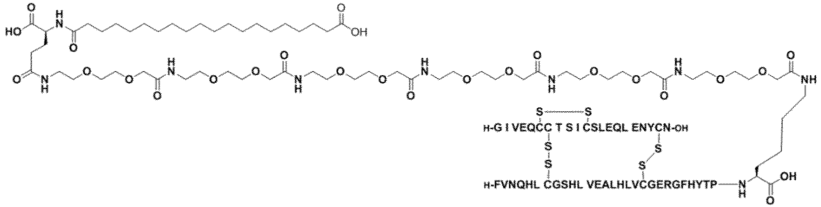

Preparation of compound A14E, B16H, B25H, B29K (*N(ε)*-eicosanedioyl-yGlu-6xOEG), desB30 human insulin was conducted by following a procedure similar to Part 1 of Control Example 1.
LC-MS (ESI): m/z = 1160.3997 [M+6H]⁶⁺

Preparation of the intermediate t-butyl eicosanedioyl-yGlu-(6xOEG-OSu)-OtBu was carried out by using the procedure similar to Part 2 of Control Example 1.

### Example 22 Pharmacokinetics

The purpose of this example is to demonstrate the in vivo pharmacokinetic properties of the formulations of the present invention.

### Pharmacokinetics in Sprague-Dawley (SD) rats

48 SD rats, divided into four groups of 12 rats in each group(equal numbers of males and females), were assigned to receive subcutaneous injection of low, medium, high doses of formulation 2 (15, 30, or 60 nmol/kg) and intravenous injection (30 nmol/kg). Blood samples were collected at various time points (0 min, 3h, 6h, 9h, 12h, 24h, 36h, 48h, 72h, 96h, and 120h) for the subcutaneous groups and (0 min, 5 min, 15 min, 0.5h, 3h, 9h, 24h, 36h, 48h, 72h, 96h, and 120h) for the intravenous group. The blood samples were collected to measure the drug concentration. Pharmacokinetic parameters including t_{1/2}, Tₘₐₓ, Cₘₐₓ, AUCₗₐₛₜ, AUC_{inf}, Vd, Cl, MRTₗₐₛₜ, and F were calculated by using the non-compartmental model in WinNonLin (8.0.0.3176) software.

**Table 15: Pharmacokinetic parameters after subcutaneous or intravenous administration of formulation 2 in SD rats**

| Doses nmol/kg | Gender | parameter | t_{1/2} (h) | Tₘₐₓ (h) | Cₘₐₓ (ng/mL) | AUCₗₐₛₜ (h·ng/Ml) | AUCᵢₙₜ (h·ng/Ml) | Vd (mL/kg) | Cl (mL/h/kg) | MRTₗₐₛₜ (h) | F (%) |
|---|---|---|---|---|---|---|---|---|---|---|---|
| 15 | male | Mean | 13.99 | 9.6 | 381.52 | 11524.13 | 11632.94 | 210.17 | 10.44 | 24.54 | - |
| | | SD | 0.56 | 2.51 | 91 | 2304.32 | 2306.4 | 37.04 | 1.95 | 1.55 | |
| | female | Mean | 12.61 | 9 | 416.77 | 11595.76 | 11710.25 | 197.3 | 10.83 | 21.31 | - |
| | | SD | 0.38 | 2.68 | 108.56 | 3244.92 | 3211.28 | 63.7 | 3.41 | 1.63 | |
| 30 | male | Mean | 13.65 | 12 | 1044.81 | 30724.45 | 30853.01 | 151.57 | 7.7 | 23.43 | 67.44 |
| | | SD | 0.74 | 0 | 67.67 | 2894.7 | 2853.24 | 16.8 | 0.77 | 1.69 | |
| | female | Mean | 12.94 | 12 | 967.34 | 28350.7 | 28448.79 | 155.37 | 8.34 | 22.44 | 71.71 |
| | | SD | 1.42 | 0 | 90.47 | 2466.55 | 2495.72 | 18.31 | 0.74 | 1.68 | |
| 60 | male | Mean | 13.39 | 12 | 1840.7 | 58246.89 | 58403.57 | 157.51 | 8.16 | 24.78 | - |
| | | SD | 0.48 | 0 | 317.68 | 7075.65 | 7110 | 16.8 | 0.91 | 0.85 | |
| | female | Mean | 12.48 | 11 | 1425.39 | 41782.48 | 41904.63 | 204.91 | 11.38 | 23.19 | - |
| | | SD | 0.88 | 1.55 | 173.41 | 5189.05 | 5195.64 | 27.48 | 1.26 | 1.89 | |
| 30 (i.v.) | male | Mean | 12.49 | - | 6037.02 | 45561.12 | 45654.23 | 94.59 | 5.23 | 10.98 | - |
| | | SD | 1.17 | - | 699.23 | 5681.95 | 5658.78 | 17.71 | 0.69 | 0.38 | |
| | female | Mean | 12.58 | - | 5977.44 | 39537.46 | 39634.66 | 110 | 6.08 | 9.14 | - |
| | | SD | 0.7 | - | 715.15 | 6419.76 | 6417.2 | 16.93 | 0.97 | 1.03 | |

| | | | | | | | | | | | |
|---|---|---|---|---|---|---|---|---|---|---|---|
| t_{1/2} = terminal elimination half-life, Tₘₐₓ = time to reach maximum concentration, Cₘₐₓ = maximum concentration, AUCₗₐₛₜ = area under the plasma drug concentration-time curve from administration time to the last time point, AUC_{inf} = area under the plasma drug concentration-time curve from administration time to infinity, V_{d} = apparent volume of distribution, Cl = clearance, MRTₗₐₛₜ= mean residence time, F=absolute bioavailability From the above experimental results, it can be observed that formulation 2 of the present invention exhibits a longer half-life, longer mean residence time (MRT), and higher bioavailability in SD rats. | | | | | | | | | | | |

### Sequence list

SEQ ID NO.1:
   A **chain** of A14E, **B16H, B25H,** desB30 Human **insulin:** 
Gly Ile Val Glu Gln Cys Cys Thr Ser Ile Cys Ser Leu Glu Gln Leu Glu
Asn Tyr Cys Asn
SEQ ID NO.2:
   **B chain** of A14E, **B16H, B25H,** desB30 Human **insulin:** 
Phe Val Asn Gln His Leu Cys Gly Ser His Leu Val Glu Ala Leu His Leu
Val Cys Gly Glu Arg Gly Phe His Tyr Thr Pro Lys
SEQ ID NO.3:
   **A chain of A14E, B16E, B25H, desB30 Human insulin:** 
Gly Ile Val Glu Gln Cys Cys Thr Ser Ile Cys Ser Leu Glu Gln Leu Glu
Asn Tyr Cys Asn
SEQ ID NO.4:
   **B chain of A14E, B16E, B25H, desB30 Human insulin:** 
Phe Val Asn Gln His Leu Cys Gly Ser His Leu Val Glu Ala Leu Glu Leu
Val Cys Gly Glu Arg Gly Phe His Tyr Thr Pro Lys
SEQ ID NO.5:
   GLP-1-(7-37) peptide 
His Ala Glu Gly Thr Phe Thr Ser Asp Val Ser Ser Tyr Leu Glu Gly Gln
Ala Ala Lys Glu Phe Ile Ala Trp Leu Val Lys Gly Arg Gly
SEQ ID NO.6:
   [Gly8, Arg34]GLP-1-(7-37) peptide 
His Gly Glu Gly Thr Phe Thr Ser Asp Val Ser Ser Tyr Leu Glu Gly Gln
Ala Ala Lys Glu Phe Ile Ala Trp Leu Val Arg Gly Arg Gly
SEQ ID NO.7:
   [Arg34]GLP-1-(7-37) peptide

His Ala Glu Gly Thr Phe Thr Ser Asp Val Ser Ser Tyr Leu Glu Gly Gln
Ala Ala Lys Glu Phe Ile Ala Trp Leu Val Arg Gly Arg Gly

## Claims

1. A pharmaceutical composition, comprising:
an acylated insulin;
2.3 moles of zinc ions/6 moles of the acylated insulin;
45 mM-65 mM phenol;
0-10 mM *m*-cresol;
10 mM-20 mM NaCl;
1.5% (weight/weight) glycerol; and
5 mM-10 mM Na₂HPO₄,
wherein,
the insulin parent of the acylated insulin is A14E, B16H, B25H, desB30 human insulin or A14E, B16E, B25H, desB30 human insulin, and an acyl moiety of the acylated insulin is linked to an amino group of a lysine residue or an N-terminal amino acid residue of the insulin parent, and the acyl moiety is shown as formula (D):
W1-(W2)ₘ-(W3)ₙ- (D),
wherein,
m is 0, 1, 2, 3, 4, 5, 6, 7, 8, 9 or 10, n is 5, 6, 7, 8, 9, 10, 11, 12, 13, 14, 15, 16, 17, 18, 19 or 20;
W3 is a neutral and alkylene glycol-containing amino acid residue;
W2 is an acidic amino acid residue;
W1 is a fatty diacid comprising 20-24 carbon atoms, wherein formally a hydroxyl group has been removed from one of the carboxyl groups of the fatty diacid;
W1, W2, and W3 are linked by amide bonds; and
the order of W2 and W3 presented in formula (D) can be interchanged independently.

2. The pharmaceutical composition according to claim 1, wherein the n is 5, 6, 7, 8, 9, 11, 12, 13, 14, 15, 16, 17, 18 or 19, preferably, n is 5, 6, 7, 8, 11, 12, 13, 14, 15, 16, 17, or 18, preferably, n is 5, 6, 7, 8, 11, 12, 13, 14, 15, or 16, Preferably, n is 5, 6, 7, 8, 11, 12, 13, 14, or 15; and/or
m is an integer of 1 to 6, preferably, m is 1, 2, 3, or 4, preferably, m is 1 or 2, preferably, m is 1; and/or
W1 is a fatty diacid comprising 20-23 carbon atoms, preferably W1 is a fatty diacid comprising 20, 21, or 22 carbon atoms, wherein formally ahydroxyl group has been removed from the one of the carboxyl groups of the fatty diacid.

3. The pharmaceutical composition according to claim 1 or 2, wherein the W3 is: -HN-(CH₂)₂-O-(CH₂)₂-O-CH₂-CO-, -HN-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₃-O-(CH₂)₄-O-(CH₂)₃-NH-CO-, -HN-(CH₂)₃-O-(CH₂)₄-O-(CH₂)₃-NH-CO-CH₂-O-CH₂-CO-, -HN-(CH₂)₃-O-(CH₂)₄-O-(CH₂)₃-NH-CO-(CH₂)₂-CO-, -HN-(CH₂)₂-O-(CH₂)₂-O-CH₂-CO-CH₂-O-CH₂-CO-, - HN-(CH₂)₃-O-(CH₂)₂-O-(CH₂)₂-O-(CH₂)₃-NH-CO-(CH₂)₂-CO-, -HN-(CH₂)₃-O-(CH₂)₂-O-(CH₂)₂-O-(CH₂)₃-NH-CO-CH₂-O-CH₂-CO-, -HN-(CH₂)₂-O-(CH₂)₂-O-(CH₂)₂-NH-CO-(CH₂)₂-CO-, -HN-(CH₂)₂-O-(CH₂)₂-O-(CH₂)₂-NH-CO-CH₂-O-CH₂-CO-, -HN-(CH₂)₃-O-(CH₂)₂-O-(CH₂)₂-O-(CH₂)₃-NH-CO-CH₂-O-CH₂-CO-, -HN-(CH₂)₃-O-(CH₂)₃-O-CH₂-CO-, or -HN-(CH₂)₄-O-(CH₂)₄-O-CH₂-CO-; Preferably W3 is -HN-(CH₂)₂-O-(CH₂)₂-O-CH₂-CO-; and/or
W2 is an amino acid residue selected from the group consisting of γGlu, αGlu, βAsp, αAsp, γ-D-Glu, α-D-Glu, β-D-Asp or α-D-Asp, preferably W2 is selected from γGlu or βAsp; and/or
W1 is HOOC-(CH₂)₁₈-CO-, HOOC-(CH₂)₁₉-CO-, HOOC-(CH₂)₂₀-CO-, HOOC-(CH₂)₂₁-CO or HOOC-(CH₂)₂₂-CO-, preferably W1 is HOOC-(CH₂)₁₈-CO-, HOOC-(CH₂)₂₀-CO- or HOOC-(CH₂)₂₂-CO-.

4. The pharmaceutical composition according to any one of claims 1-3, wherein the formula (D) is linked to the amino group of the lysine residue or the N-terminal amino acid residue of the insulin parent through the C-terminal of W3.

5. The pharmaceutical composition according to any one of claims 1-4, wherein the acyl moiety is linked to an ε amino group of the lysine residue of the insulin parent.

6. The pharmaceutical composition according to any one of claims 1-5, wherein the acylated insulin is selected from the following insulins: A14E, B16H, B25H, B29K (N(s)-eicosanedioyl-γGlu-5xOEG), desB30 human insulin; A14E, B16H, B25H, B29K (N(ε)-eicosanedioyl-γGlu-6xOEG), desB30 human insulin; A14E, B16H, B25H, B29K (N(ε)-docosanedioyl-yGlu-5xOEG), desB30 human insulin; A14E, B16H, B25H, B29K (N(ε)-docosanedioyl-yGlu-6xOEG), desB30 human insulin; A14E, B16H, B25H, B29K (N(ε)-eicosanedioyl-γGlu-7xOEG), desB30 human insulin; A14E, B16H, B25H, B29K (N(ε)-eicosanedioyl-γGlu-8xOEG), desB30 human insulin; A14E, B16H, B25H, B29K (N(ε)-docosanedioyl-γGlu-7xOEG), desB30 human insulin; A14E, B16H, B25H, B29K (N(ε)-docosanedioyl-yGlu-8xOEG), desB30 human insulin; A14E, B16H, B25H, B29K (N(ε)-eicosanedioyl-γGlu-9xOEG), desB30 human insulin; A14E, B16H, B25H, B29K (N(ε)-docosanedioyl-γGlu-9xOEG), desB30 human insulin; or A14E, B16H, B25H, B29K (N(ε)-docosanedioyl-γGlu-10xOEG), desB30 human insulin; A14E, B16H, B25H, B29K (N(ε)-eicosanedioyl-yGlu-12xOEG), desB30 human insulin; A14E, B16H, B25H, B29K (N(ε)-docosanedioyl-γGlu-12xOEG), desB30 human insulin; A14E, B16H, B25H, B29K (N(ε)-eicosanedioyl-γGlu-11xOEG), desB30 human insulin; A14E, B16H, B25H, B29K (N(ε)-docosanedioyl-γGlu-11xOEG), desB30 human insulin; A14E, B16H, B25H, B29K (N(ε)-heneicosanedioyl-yGlu-12xOEG), desB30 human insulin; A14E, B16H, B25H, B29K (N(ε)-tricosanedioyl-γGlu-12xOEG), A14E, B16H, B25H, B29K(N(ε)-tetracosanedioyl-γGlu-12xOEG), desB30 human insulin; desB30 human insulin; A14E, B16H, B25H, B29K (N(ε)-eicosanedioyl-γGlu-13xOEG), desB30 human insulin; A14E, B16H, B25H, B29K (N(ε)-eicosanedioyl-yGlu-14xOEG), desB30 human insulin; A14E, B16H, B25H, B29K (N(ε)-eicosanedioyl-γGlu-15xOEG), desB30 human insulin; A14E, B16H, B25H, B29K (N(ε)-docosanedioyl-γGlu-13xOEG), desB30 human insulin; A14E, B16H, B25H, B29K (N(ε)-docosanedioyl-γGlu-14xOEG), desB30 human insulin; A14E, B16H, B25H, B29K (N(ε)-eicosanedioyl-yGlu-15xOEG), desB30 human insulin; A14E, B16H, B25H, B29K (N(ε)-eicosanedioyl-γGlu-16xOEG), desB30 human insulin; A14E, B16H, B25H, B29K (N(ε)-eicosanedioyl-γGlu-17xOEG), desB30 human insulin; A14E, B16H, B25H, B29K (N(ε)-docosanedioyl-γGlu-16xOEG), desB30 human insulin; A14E, B16H, B25H, B29K (N(ε)-docosanedioyl-yGlu-17xOEG), desB30 human insulin; A14E, B16H, B25H, B29K (N(ε)-eicosanedioyl-γGlu-18xOEG), desB30 human insulin; A14E, B16H, B25H, B29K (N(ε)-eicosanedioyl-γGlu-19xOEG), desB30 human insulin; A14E, B16H, B25H, B29K (N(ε)-docosanedioyl-γGlu-18xOEG), desB30 human insulin; A14E, B16H, B25H, B29K (N(ε)-docosanedioyl-yGlu-19xOEG), desB30 human insulin; A14E, B16H, B25H, B29K (N(ε)-eicosanedioyl-γGlu-20xOEG), desB30 human insulin; A14E, B16H, B25H, B29K (N(ε)-docosanedioyl-γGlu-20xOEG), desB30 human insulin; A14E, B16H, B25H, B29K (N(ε)-docosanedioyl-γGlu-24xOEG), desB30 human insulin; A14E, B16E, B25H, B29K (N(ε)-eicosanedioyl-yGlu-5xOEG), desB30 human insulin; A14E, B16E, B25H, B29K (N(ε)-eicosanedioyl-γGlu-6xOEG), desB30 human insulin; A14E, B16E, B25H, B29K (N(ε)-docosanedioyl-γGlu-5xOEG), desB30 human insulin; A14E, B16E, B25H, B29K (N(ε)-docosanedioyl-γGlu-6xOEG), desB30 human insulin; A14E, B16E, B25H, B29K ( N(ε)-eicosanedioyl-yGlu-7xOEG), desB30 human insulin; A14E, B16E, B25H, B29K (N(ε)-eicosanedioyl-yGlu-8xOEG), desB30 human insulin; A14E, B16E, B25H, B29K (N(ε)-docosanedioyl-γGlu-7xOEG), desB30 human insulin; A14E, B16E, B25H, B29K (N(ε)-docosanedioyl-γGlu-8xOEG), desB30 human insulin; A14E, B16E, B25H, B29K (N(ε)-eicosanedioyl-γGlu-9xOEG), desB30 human insulin; A14E, B16E, B25H, B29K (N(ε)-docosanedioyl-yGlu-9xOEG), desB30 human insulin; or A14E, B16E, B25H, B29K (N(ε)-docosanedioyl-γGlu-10xOEG), desB30 human insulin; A14E, B16E, B25H, B29K (N(ε)-eicosanedioyl-γGlu-12xOEG), desB30 human insulin; A14E, B16E, B25H, B29K (N(ε)-docosanedioyl-γGlu-12xOEG), desB30 human insulin; A14E, B16E, B25H, B29K (N(ε)-eicosanedioyl-γGlu-11xOEG), desB30 human insulin; A14E, B16E, B25H, B29K (N(ε)-docosanedioyl-γGlu-11xOEG), desB30 human insulin; A14E, B16E, B25H, B29K (N(ε)-heneicosanedioyl-γGlu-12xOEG), desB30 human insulin; A14E, B16E, B25H, B29K (N(ε)-tricosanedioyl-γGlu-12xOEG), desB30 human insulin; A14E, B16E, B25H, B29K (N(ε)-tetracosanedioyl-γGlu-12xOEG), desB30 human insulin; A14E, B16E, B25H, B29K (N(ε)-eicosanedioyl-yGlu-13xOEG), desB30 human insulin; A14E, B16E, B25H, B29K (N(ε)-eicosanedioyl-γGlu-14xOEG), desB30 human insulin; A14E, B16E, B25H, B29K (N(ε)-eicosanedioyl-γGlu-15xOEG), desB30 human insulin; A14E, B16E, B25H, B29K (N(ε)-docosanedioyl-γGlu-13xOEG), desB30 human insulin; A14E, B16E, B25H, B29K (N(ε)-docosanedioyl-yGlu-14xOEG), desB30 human insulin; A14E, B16E, B25H, B29K (N(ε)-eicosanedioyl-γGlu-15xOEG), desB30 human insulin; A14E, B16E, B25H, B29K (N(ε)-eicosanedioyl-γGlu-16xOEG), desB30 human insulin; A14E, B16E, B25H, B29K (N(ε)-eicosanedioyl-γGlu-17xOEG), desB30 human insulin; A14E, B16E, B25H, B29K (N(ε)-docosanedioyl-yGlu-16xOEG), desB30 human insulin; A14E, B16E, B25H, B29K (N(ε)-docosanedioyl-γGlu-17xOEG), desB30 human insulin; A14E, B16E, B25H, B29K (N(ε)-eicosanedioyl-γGlu-18xOEG), desB30 human insulin; A14E, B16E, B25H, B29K (N(ε)-eicosanedioyl-γGlu-19xOEG), desB30 human insulin; A14E, B16E, B25H, B29K(N(ε)-docosanedioyl-yGlu-18xOEG), desB30 human insulin; A14E, B16E, B25H, B29K(N(ε)-docosanedioyl-γGlu-19xOEG), desB30 human insulin; A14E, B16E, B25H, B29K(N(ε)-eicosanedioyl-γGlu-20xOEG), desB30 human insulin; or A14E, B16E, B25H, B29K (N(ε)-docosanedioyl-γGlu-20xOEG), desB30 human insulin;
Preferably, the acylated insulin is selected from the following insulins:
A14E, B16H, B25H, B29K(N(ε)-eicosanedioyl-γGlu-5xOEG), desB30 human insulin; A14E, B16H, B25H, B29K(N(ε)-docosanedioyl-γGlu-5xOEG), desB30 human insulin; A14E, B16H, B25H, B29K (N(ε)-eicosanedioyl-yGlu-6xOEG), desB30 human insulin; A14E, B16H, B25H, B29K (N(ε)-docosanedioyl-γGlu-6xOEG), desB30 human insulin; A14E, B16H, B25H, B29K (N(ε)-eicosanedioyl-γGlu-8xOEG ), desB30 human insulin; A14E, B16H, B25H, B29K (N(ε)-docosanedioyl-γGlu-8xOEG), desB30 human insulin; A14E, B16H, B25H, B29K (N(ε)-eicosanedioyl-γGlu-11xOEG), desB30 human insulin; A14E, B16H, B25H, B29K (N(ε)-eicosanedioyl-yGlu-12xOEG), desB30 human insulin; A14E, B16H, B25H, B29K(N(ε)-docosanedioyl-γGlu-11xOEG), desB30 human insulin; A14E, B16H, B25H, B29K(N(ε)-docosanedioyl-γGlu-12xOEG), desB30 human insulin; or A14E, B16H, B25H, B29K (N(ε)-docosanedioyl-γGlu-18xOEG), desB30 human insulin;
Preferably, the acylated insulin is selected from the following insulins: A14E, B16H, B25H, B29K(N(ε)-eicosanedioyl-γGlu-12xOEG), desB30 human insulin; or A14E, B16H, B25H, B29K (N(ε)-docosanedioyl-γGlu-12xOEG), desB30 human insulin.

7. The pharmaceutical composition according to any one of claims 1-6, wherein the content of the acylated insulin is more than about 0.3 mM, preferably about 0.3-9mM, preferably about 0.6-8.4mM, preferably about 0.6-7.2 mM, preferably about 0.6-6.0 mM, preferably about 0.6-4.2 mM, preferably about 0.6-3.6 mM, preferably about 0.6-3.0 mM, preferably about 0.6-2.4 mM, preferably about 0.6-2.1 mM , preferably about 0.9-1.8 mM, preferably about 0.9-1.5 mM, preferably about 1.2-1.5 mM; and/or
the content of phenol is about 45 mM, about 46 mM, about 47 mM, about 48 mM, about 49 mM, 50 mM, about 51 mM, about 52 mM, about 53 mM, about 54 mM, about 55 mM, about 56 mM, about 57 mM, about 58 mM, about 59 mM, or about 60 mM; and/or
the content of *m*-cresol is about 0 mM, about 1 mM, about 2 mM, about 3 mM, about 4 mM, about 5 mM, about 6 mM, about 7 mM, about 8 mM, about 9 mM, or about 10 mM; and/or
the content of NaCl is about 10 mM, about 11 mM, about 12 mM, about 13 mM, about 14 mM, about 15 mM, about 16 mM, about 17 mM, about 18 mM, about 19 mM, or about 20 mM; and/or
the pharmaceutical composition has a pH value of 6.5-8.5; preferably a pH value of 6.5-8.0; preferably a pH value of 7.0-7.8; preferably a pH value of 7.2-7.6; more preferably a pH value of 7.4.

8. A pharmaceutical composition comprising:
about 0.9-1.5 mM acylated insulin;
about 2.3 moles of zinc ions/6 moles of acylated insulin;
about 45 mM phenol;
about 10 mM *m*-cresol;
about 20 mM NaCl;
about 15 mg/ml glycerin;
about 5-10 mM Na₂HPO₄; and
having a pH value of about 6.5-8.0,
wherein the acylated insulin is A14E, B16H, B25H, B29K (N(ε)-eicosanedioyl-yGlu-5xOEG), desB30 human insulin; A14E, B16H, B25H, B29K (N(ε)-docosanedioyl-γGlu-5xOEG), desB30 human insulin; A14E, B16H, B25H, B29K (N(ε)-eicosanedioyl-γGlu-6xOEG), desB30 human insulin; A14E, B16H, B25H, B29K (N(ε)-docosanedioyl-γGlu-6xOEG), desB30 human insulin; A14E, B16H, B25H, B29K (N(ε)-eicosanedioyl-yGlu-8xOEG), desB30 human insulin; A14E, B16H, B25H, B29K (N(ε)-docosanedioyl-yGlu-8xOEG), desB30 human insulin; A14E, B16H, B25H, B29K (N(ε)-eicosanedioyl-γGlu-11xOEG ), desB30 human insulin; A14E, B16H, B25H, B29K (N(ε)-eicosanedioyl-γGlu-12xOEG), desB30 human insulin; A14E, B16H, B25H, B29K (N(ε)-docosanedioyl-γGlu-11xOEG), desB30 human insulin; A14E, B16H, B25H, B29K (N(ε)-docosanedioyl-yGlu-12xOEG), desB30 human insulin; or A14E, B16H, B25H, B29K (N(ε)-docosanedioyl-γGlu-18xOEG), desB30 human insulin.

9. A pharmaceutical composition comprising:
about 1.2 mM acylated insulin;
about 2.3 moles of zinc ions/6 moles of acylated insulin;
about 45 mM phenol;
about 10 mM *m*-cresol;
about 20 mM NaCl;
about 15 mg/ml glycerin;
about 5 mM Na₂HPO₄; and
having a pH value of about 7.4,
wherein the acylated insulin is A14E, B16H, B25H, B29K (N(ε)-eicosanedioyl-yGlu-5xOEG), desB30 human insulin; A14E, B16H, B25H, B29K (N(ε)-docosanedioyl-γGlu-5xOEG), desB30 human insulin; A14E, B16H, B25H, B29K (N(ε)-eicosanedioyl-γGlu-6xOEG), desB30 human insulin; A14E, B16H, B25H, B29K(N(ε)-docosanedioyl-γGlu-6xOEG), desB30 human insulin; A14E, B16H, B25H, B29K (N(ε)-eicosanedioyl-yGlu-8xOEG), desB30 human insulin; A14E, B16H, B25H, B29K (N(ε)-docosanedioyl-yGlu-8xOEG), desB30 human insulin; A14E, B16H, B25H, B29K (N(ε)-eicosanedioyl-γGlu-11xOEG), desB30 human insulin; A14E, B16H, B25H, B29K (N(ε)-eicosanedioyl-γGlu-12xOEG), desB30 human insulin; A14E, B16H, B25H, B29K (N(ε)-docosanedioyl-γGlu-11xOEG), desB30 human insulin; A14E, B16H, B25H, B29K (N(ε)-docosanedioyl-yGlu-12xOEG), desB30 human insulin; or A14E, B16H, B25H, B29K(N(ε)-docosanedioyl-γGlu-18xOEG), desB30 human insulin.

10. A pharmaceutical composition comprising: insulinotropic GLP-1 compounds and acylated insulins,
wherein, the molar ratio of the insulinotropic GLP-1 compound to the acylated insulin is at least about 1:100, preferably at least about 3:100, preferably at least about 5:100, preferably at least about 8:100, preferably at least about (3:100)-(100:100), preferably about (5:100)-(80:100), preferably about (8:100)-(50:100), preferably about (10:100)-(50:100), preferably about (13:100)-(50:100), preferably about (13:100)-(40:100), preferably about (13:100)-(35:100), preferably about (13:100)-(27:100), preferably about (13:100)-(20:100),
the acylated insulin is as defined in any one of claims 1-9,
the insulinotropic GLP-1 compound is *N*-ε²⁶-(17-carboxyheptadecanoylamino)-4(*S*)-carboxybutanoyl-[Arg34]GLP-1-(7-37) peptide, *N*-ε²⁶-(17-carboxyheptadecanoylamino)-4(*S*)-carboxybutanoyl-[Gly8, Arg34]GLP-1-(7-37) peptide, *N*-ε²⁶-[2-(2-[2-(2-[2-(2-[4-(17-carboxyheptadecanoylamino)-4(*S*)-carboxybutanoylamino] ethoxy)ethoxy]acetylamino)ethoxy]ethoxy)acetyl ][Aib8, Arg34]GLP-1-(7-37) peptide, or *N*-ε²⁶-[2-(2-[2-(2-[2-(2-[4-(17-carboxyheptadecanoylamino)-4(S)-carboxybutanoylamino]ethoxy)ethoxy]acetylamino)ethoxy]ethoxy)acetyl ][Gly8, Arg34]GLP-1-(7-37) peptide, *N*-ε³⁰-[2-(2-[2-(2-[2-(2-[4-(17-carboxyheptadecanylamino)-4(*S*)-carboxybutanoylamino]ethoxy)ethoxy] acetylamino)ethoxy]ethoxy) acetyl] (Val⁸ Glu²² Lys³⁰ Arg^{26,34}-GLP-1(7-37)) peptide, or *N*-ε²³-[2-(2-[2-(2-[2-(2-[4-(17-carboxyheptadecanylamino)-4(S)-carboxybutanoylamino] ethoxy)ethoxy]acetylamino)ethoxy]ethoxy)acetyl ] (Val⁸ Glu²² Lys²³ Arg^{26,34}-GLP-1(7-37)) peptide, or
the insulinotropic GLP-1 compound is represented by formula (B),
[Acy-(L1)ᵣ-(L2)_{q}]-G1 (B),
wherein G1 is a GLP-1 analogue with Arg and Ala or Gly, respectively at positions corresponding to position 34 and position 8, respectively, of GLP-1(7-37) (SEQ ID NO: 5), and [Acy-(L1)ᵣ-(L2)_{q}] is a substituent linked to an ε amino group of the Lys residue at position 26 of the GLP-1 analogue, wherein
r is an integer from 1 to 10, and q is an integer of 0 or from 1 to 10;
Acy is a fatty diacid comprising 20-24 carbon atoms, wherein formally a hydroxyl group has been removed from one of the carboxyl groups of the fatty diacid;
L1 is an amino acid residue selected from the group consisting of γGlu, αGlu, βAsp, αAsp, γ-D-Glu, α-D-Glu, β-D-Asp or α-D-Asp;
L2 is a neutral and alkylene glycol-containing amino acid residue;
Acy, L1, and L2 are linked by amide bonds; and
the order of L1 and L2 presented in formula (B) can be interchanged independently.

11. Pharmaceutical composition according to claim 10, wherein,
G1 is [Gly8, Arg34]GLP-1-(7-37) peptide (SEQ ID NO:6) or [Arg34]GLP-1-(7-37) peptide (SEQ ID NO:7), preferably [Gly8, Arg34] GLP-1-(7-37) peptide; and/or
r is 1, 2, 3, 4, 5 or 6, preferably r is 1, 2, 3 or 4, preferably r is 1 or 2, preferably r is 1; and/or
q is 0, 1, 2, 3, 4, 5, 6, 7 or 8, preferably, q is 0, 1, 2, 3 or 4, more preferably, q is 0, 1 or 2; and/or
Acy is a fatty diacid comprising 20-23 carbon atoms, preferably Acy is a fatty diacid comprising 20, 21, or 22 carbon atoms, wherein formally ahydroxyl group has been removed from one of the carboxyl groups of the fatty diacid.

12. The pharmaceutical composition according to any one of claims 10-11, wherein,
L2 is: -HN-(CH₂)₂-O-(CH₂)₂-O-CH₂-CO-, -HN-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₂-O-(CH₂)₂-O-(CH₂)₂-O-(CH₂)₂-O-(CH₂)₂-O-(CH₂)₂-O-(CH₂)₂-O-(CH₂)₂-O-(CH₂)₂-CO-, -HN-(CH₂)₃-O-(CH₂)₄-O-(CH₂)₃-NH-CO-, -HN-(CH₂)₃-O-(CH₂)₄-O-(CH₂)₃-NH-CO-CH₂-O-CH₂-CO-, -HN-(CH₂)₃-O-(CH₂)₄-O-(CH₂)₃-NH-CO-(CH₂)₂-CO-, -HN-(CH₂)₂-O-(CH₂)₂-O-CH₂-CO-CH₂-O-CH₂-CO-, - HN-(CH₂)₃-O-(CH₂)₂-O-(CH₂)₂-O-(CH₂)₃-NH-CO-(CH₂)₂-CO-, -HN-(CH₂)₃-O-(CH₂)₂-O-(CH₂)₂-O-(CH₂)₃-NH-CO-CH₂-O-CH₂-CO-, -HN-(CH₂)₂-O-(CH₂)₂-O-(CH₂)₂-NH-CO-(CH₂)₂-CO-, -HN-(CH₂)₂-O-(CH₂)₂-O-(CH₂)₂-NH-CO-CH₂-O-CH₂-CO-, -HN-(CH₂)₃-O-(CH₂)₂-O-(CH₂)₂-O-(CH₂)₃-NH-CO-CH₂-O-CH₂-CO-, -HN-(CH₂)₃-O-(CH₂)₃-O-CH₂-CO-, or -HN-(CH₂)₄-O-(CH₂)₄-O-CH₂-CO-; preferably L2 is -HN-(CH₂)₂-O-(CH₂)₂-O-CH₂-CO-; and/or
L1 is selected from γGlu or βAsp, preferably L1 is yGlu; and/or
Acy is HOOC-(CH₂)₁₈-CO-, HOOC-(CH₂)₁₉-CO-, HOOC-(CH₂)₂₀-CO-, HOOC-(CH₂)₂₁-CO- or HOOC-(CH₂)₂₂-CO-, preferably, Acy is HOOC-(CH₂)₁₈-CO-, HOOC-(CH₂)₂₀-CO- or HOOC-(CH₂)₂₂-CO-.

13. The pharmaceutical composition according to any one of claims 10-12, wherein, in the formula (B), Acy, L1, and L2 are sequentially connected by amide bonds, and the C-terminal of L2 is linked to the ε amino group of the Lys residue at position 26 of the GLP-1 analogue.

14. The pharmaceutical composition according to any one of claims 10-13, wherein the insulinotropic GLP-1 compound is selected from the following compounds:
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl] [Gly8, Arg34]GLP-1-(7-37) peptide,
*N*-ε²⁶-[2-(2-[2-(2-[2-(2-[4-(21-carboxyheneicosanoylamino)-4(*S*)-carboxybutanoylamino] ethoxy)ethoxy]acetylamino)ethoxy]ethoxy)acetyl ][Gly8, Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl] [Gly8, Arg34]GLP-1-(7-37) peptide,
*N*-ε²⁶-[2-(2-[2-(2-[2-(2-[4-(23-carboxytricosanoylamino)-4(S)-carboxybutanoylamino] ethoxy)ethoxy]acetylamino)ethoxy]ethoxy)acetyl ][Gly8, Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl] [Gly8, Arg34]GLP-1-(7-37) peptide,
*N*-ε²⁶-(23-carboxytricosanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(21-carboxyheneicosanoylamino)-4(*S*)-carboxybutanoyl-[Gly8, Arg34]GLP-1-(7-37) peptide,
*N*-ε²⁶-[2-(2-[2-(2-[2-(2-[4-(19-carboxynonadecanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl] [Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl] [Arg34]GLP-1-(7-37) peptide,
*N*-ε²⁶-[2-(2-[2-(2-[2-(2-[4-(23-carboxytricosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[23-carboxytricosanoylamino]-4(*S*)-carboxybutanoylamino)ethoxy]ethoxy)acetyl] [Arg34]GLP-1-(7-37) peptide,
*N*-ε²⁶-(23-carboxytricosanoylamino)-4(*S*)-carboxybutanoyl-[Arg34]GLP-1-(7-37) peptide,
*N*-ε²⁶-(19-carboxynonadecanoylamino)-4(*S*)-carboxybutanoyl-[Arg34]GLP-1-(7-37) peptide,
*N*-ε²⁶-(21-carboxyheneicosanoylamino)-4(*S*)-carboxybutanoyl-[Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl] [Gly8, Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl] [Gly8, Arg34]GLP-1-(7-37) peptide,
*N*-ε²⁶-(20-carboxyeicosanoylamino)-4(*S*)-carboxybutanoyl-[Gly8, Arg34]GLP-1-(7-37) peptide,
*N*-ε²⁶-(22-carboxydocosanoylamino)-4(*S*)-carboxybutanoyl-[Gly8, Arg34]GLP-1-(7-37) peptide,
*N*-ε²⁶-[2-(2-[2-(2-[2-(2-[4-(20-carboxyeicosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[20-carboxyeicosanoylamino]-4(*S*)-carboxybutanoylamino)ethoxy]ethoxy)acetyl][Arg34]GLP-1-(7-37) peptide,
*N*-ε²⁶-[2-(2-[2-(2-[2-(2-[4-(22-carboxydocosanoylamino)-4(*S*)-carboxybutanoylamino]ethoxy)ethoxy]acetylamino)ethoxy]ethoxy)acetyl ][Arg34]GLP-1-(7-37) peptide,
*N*-ε²⁶-[2-(2-[2-(4-[22-carboxydocosanoylamino]-4(*S*)-carboxybutanoylamino)ethoxy]ethoxy)acetyl] [Arg34]GLP-1-(7-37) peptide,
*N*-ε²⁶-(20-carboxyeicosanoylamino)-4(*S*)-carboxybutanoyl-[Arg34]GLP-1-(7-37) peptide, or
*N*-ε²⁶-(22-carboxydocosanoylamino)-4(*S*)-carboxybutanoyl-[Arg34]GLP-1-(7-37) peptide; and/or
the acylated insulin is selected from the following insulins: A14E, B16H, B25H, B29K (N(ε)-eicosanedioyl-γGlu-5xOEG), desB30 human insulin; A14E, B16H, B25H, B29K (N(ε))-eicosanedioyl-γGlu-6xOEG), desB30 human insulin; A14E, B16H, B25H, B29K (N(ε)-docosanedioyl-γGlu-5xOEG), desB30 human insulin; A14E, B16H, B25H, B29K (N(ε)-docosanedioyl-yGlu-6xOEG), desB30 human insulin; A14E, B16H, B25H, B29K (N(ε)-eicosanedioyl-γGlu-7xOEG), desB30 human insulin; A14E, B16H, B25H, B29K (N(ε)-eicosanedioyl-γGlu-8xOEG), desB30 human insulin; A14E, B16H, B25H, B29K (N(ε)-docosanedioyl-γGlu-7xOEG), desB30 human insulin; A14E, B16H, B25H, B29K (N(ε)-docosanedioyl-yGlu-8xOEG), desB30 human insulin; A14E, B16H, B25H, B29K (N(ε)-eicosanedioyl-γGlu-9xOEG), desB30 human insulin; A14E, B16H, B25H, B29K (N(ε)- docosanedioyl-yGlu-9xOEG), desB30 human insulin; or A14E, B16H , B25H, B29K (N(ε)- docosanedioyl-yGlu-10xOEG), desB30 human insulin; A14E, B16H, B25H, B29K (N(ε)-eicosanedioyl-yGlu-12xOEG), desB30 human insulin; A14E, B16H, B25H, B29K(N(ε)-docosanedioyl-γGlu-12xOEG), desB30 human insulin; A14E, B16H, B25H, B29K (N(ε)-eicosanedioyl-γGlu-11xOEG), desB30 human insulin; A14E, B16H, B25H, B29K (N(ε)-docosanedioyl-γGlu-11xOEG), desB30 human insulin; A14E, B16H, B25H, B29K (N(ε)-heneicosanedioyl-yGlu-12xOEG), desB30 human insulin; A14E, B16H, B25H, B29K (N(ε)-tricosanedioyl-γGlu-12xOEG), desB30 human insulin; A14E, B16H, B25H, B29K(N(ε)-tetracosanedioyl-γGlu-12xOEG), desB30 human insulin; A14E, B16H, B25H, B29K (N(ε)-eicosanedioyl-γGlu-13xOEG), desB30 human insulin; A14E, B16H, B25H, B29K (N(ε)-eicosanedioyl-yGlu-14xOEG), desB30 human insulin; A14E, B16H, B25H, B29K (N(ε)-eicosanedioyl-γGlu-15xOEG), desB30 human insulin; A14E, B16H, B25H, B29K (N(ε)-docosanedioyl-γGlu-13xOEG), desB30 human insulin; A14E, B16H, B25H, B29K (N(ε)-docosanedioyl-γGlu-14xOEG), desB30 human insulin; A14E, B16H, B25H, B29K (N(ε)-eicosanedioyl-yGlu-15xOEG), desB30 human insulin; A14E, B16H, B25H, B29K (N(ε)-eicosanedioyl-γGlu-16xOEG), desB30 human insulin; A14E, B16H, B25H, B29K (N(ε)-eicosanedioyl-γGlu-17xOEG), desB30 human insulin; A14E, B16H, B25H, B29K (N(ε)-docosanedioyl-γGlu-16xOEG), desB30 human insulin; A14E, B16H, B25H, B29K (N(ε)-docosanedioyl-yGlu-17xOEG), desB30 human insulin; A14E, B16H, B25H, B29K (N(ε)-eicosanedioyl-γGlu-18xOEG), desB30 human insulin; A14E, B16H, B25H, B29K (N(ε) )-eicosanedioyl-yGlu-19xOEG), desB30 human insulin; A14E, B16H, B25H, B29K (N(ε)-docosanedioyl-γGlu-18xOEG), desB30 human insulin; A14E, B16H, B25H, B29K (N(ε)-docosanedioyl-yGlu-19xOEG), desB30 human insulin; A14E, B16H, B25H, B29K (N(ε)-eicosanedioyl-γGlu-20xOEG), desB30 Human insulin; A14E, B16H, B25H, B29K (N(ε)-docosanedioyl-γGlu-20xOEG), desB30 human insulin; A14E, B16H, B25H, B29K (N(ε)-docosanedioyl-γGlu-24xOEG), desB30 human insulin; A14E, B16E, B25H, B29K (N(ε)-eicosanedioyl-yGlu-5xOEG), desB30 human insulin; A14E, B16E, B25H, B29K (N(ε)-eicosanedioyl-γGlu-6xOEG), desB30 human insulin; A14E, B16E, B25H, B29K (N(ε)-docosanedioyl-γGlu-5xOEG), desB30 human insulin; A14E, B16E, B25H, B29K (N(ε)-docosanedioyl-γGlu-6xOEG), desB30 human insulin; A14E, B16E, B25H, B29K (N(ε)-eicosanedioyl-yGlu-7xOEG), desB30 Human insulin; A14E, B16E, B25H, B29K (N(ε)-eicosanedioyl-yGlu-8xOEG), desB30 human insulin; A14E, B16E, B25H, B29K (N(ε)-docosanedioyl-γGlu-7xOEG), desB30 human insulin; A14E, B16E, B25H, B29K (N(ε)-docosanedioyl-γGlu-8xOEG), desB30 human insulin; A14E, B16E, B25H, B29K (N(ε))-eicosanedioyl-γGlu-9xOEG), desB30 human insulin; A14E, B16E, B25H, B29K (N(ε)-docosanedioyl-yGlu-9xOEG), desB30 human insulin; or A14E, B16E , B25H, B29K (N(ε)-docosanedioyl-γGlu-10xOEG), desB30 human insulin; A14E, B16E, B25H, B29K (N(ε)-eicosanedioyl-γGlu-12xOEG), desB30 human insulin; A14E, B16E, B25H, B29K (N(ε)-docosanedioyl-γGlu-12xOEG), desB30 human insulin; A14E, B16E, B25H, B29K (N(ε)-eicosanedioyl-γGlu-11xOEG), desB30 human insulin; A14E, B16E, B25H, B29K (N(ε)-docosanedioyl-γGlu-11xOEG), desB30 human insulin; A14E, B16E, B25H, B29K (N(ε)-heneicosanedioyl-γGlu-12xOEG), desB30 human insulin; A14E, B16E, B25H, B29K (N(ε)-tricosanedioyl-γGlu-12xOEG), desB30 human insulin; A14E, B16E, B25H, B29K (N(ε)-tetracosanedioyl-γGlu-12xOEG), desB30 human insulin; A14E, B16E, B25H, B29K (N(ε)-eicosanedioyl-yGlu-13xOEG), desB30 human insulin; A14E, B16E, B25H, B29K (N(ε)-eicosanedioyl-γGlu-14xOEG), desB30 human insulin; A14E, B16E, B25H, B29K (N(ε)-eicosanedioyl-γGlu-15xOEG), desB30 human insulin; A14E, B16E, B25H, B29K (N(ε)-docosanedioyl-γGlu-13xOEG), desB30 human insulin; A14E, B16E, B25H, B29K (N(ε)-docosanedioyl-yGlu-14xOEG), desB30 human insulin; A14E, B16E, B25H, B29K (N(ε)-eicosanedioyl-γGlu-15xOEG), desB30 human insulin; A14E, B16E , B25H, B29K(N(ε)-eicosanedioyl-γGlu-16xOEG), desB30 human insulin; A14E, B16E, B25H, B29K (N(ε)-eicosanedioyl-γGlu-17xOEG), desB30 Human insulin; A14E, B16E, B25H, B29K (N(ε)-docosanedioyl-yGlu-16xOEG), desB30 human insulin; A14E, B16E, B25H, B29K (N(ε)-docosanedioyl-γGlu-17xOEG), desB30 human insulin; A14E, B16E, B25H, B29K(N(ε)-eicosanedioyl-γGlu-18xOEG), desB30 human insulin; A14E, B16E, B25H, B29K (N(ε))-eicosanedioyl-γGlu-19xOEG), desB30 human insulin; A14E, B16E, B25H, B29K (N(ε)-docosanedioyl-yGlu-18xOEG), desB30 human insulin; A14E, B16E, B25H, B29K (N(ε)-docosanedioyl-γGlu-19xOEG), desB30 human insulin; A14E, B16E, B25H, B29K (N(ε)-eicosanedioyl-γGlu-20xOEG), desB30 Human insulin; or A14E, B16E, B25H, B29K (N(ε)-docosanedioyl-yGlu-20xOEG), desB30 human insulin.

15. The pharmaceutical composition according to any one of claims 10-14, wherein,
the insulinotropic GLP-1 compound is selected from the following compounds:
*N*-ε²⁶-[2-(2-[2-(2-[2-(2-[4-(19-carboxynonadecanoylamino)-4(*S*)-carboxybutanoylamino] ethoxy)ethoxy]acetylamino)ethoxy]ethoxy)acetyl ][Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(19-carboxynonadecanoylamino)-4(S)-carboxybutanoyl-[Arg34]GLP-1-(7-37) peptide,
*N*-ε²⁶-[2-(2-[2-(2-[2-(2-[4-(21-carboxyheneicosanoylamino)-4(*S*)-carboxybutanoylamino] ethoxy)ethoxy]acetylamino)ethoxy]ethoxy)acetyl ][Gly8, Arg34]GLP-1-(7-37) peptide,
*N*-ε²⁶-[2-(2-[2-(4-[21-carboxyheneicosanoylamino]-4(*S*)-carboxybutanoylamino)ethoxy]ethoxy)acetyl][Gly8, Arg34]GLP-1-(7-37) peptide; and/or
the acylated insulin is selected from the following insulins: A14E, B16H, B25H, B29K (N(ε)-eicosanedioyl-γGlu-5xOEG), desB30 human insulin; A14E, B16H, B25H, B29K (N(ε))-docosanedioyl-γGlu-5xOEG), desB30 human insulin; A14E, B16H, B25H, B29K (N(ε)-eicosanedioyl-γGlu-6xOEG), desB30 human insulin; A14E, B16H, B25H, B29K (N(ε)-docosanedioyl-yGlu-6xOEG), desB30 human insulin; A14E, B16H, B25H, B29K (N(ε)-eicosanedioyl-γGlu-8xOEG), desB30 Human insulin; A14E, B16H, B25H, B29K (N(ε)-docosanedioyl-γGlu-8xOEG), desB30 human insulin; A14E, B16H, B25H, B29K (N(ε)-eicosanedioyl-γGlu-11xOEG), desB30 human insulin; A14E, B16H, B25H, B29K (N(ε)-eicosanedioyl-yGlu-12xOEG), desB30 human insulin; A14E, B16H, B25H, B29K (N(ε)-docosanedioyl-γGlu-11xOEG), desB30 human insulin; A14E, B16H, B25H, B29K (N(ε)-docosanedioyl-γGlu-12xOEG), desB30 human insulin; or A14E, B16H, B25H, B29K(N(ε)-docosanedioyl-γGlu-18xOEG), desB30 human insulin.

16. The pharmaceutical composition according to any one of claims 10-15, wherein the content of the acylated insulin is more than about 0.3 mM, preferably about 0.3-9 mM, preferably about 0.6-8.4 mM, preferably about 0.6-7.2 mM, preferably about 0.6-6.0 mM, preferably about 0.6-4.2 mM, preferably about 0.6-3.6 mM, preferably about 0.6-3.0 mM, preferably about 0.6-2.4 mM, preferably about 0.6-2.1 mM, preferably about 0.9-1.8 mM, preferably about 0.9-1.5 mM, preferably about 1.2-1.5 mM.

17. The pharmaceutical composition according to any one of claims 10-16, the composition further comprising: zinc ions, glycerol, phenol, m-cresol, NaCl, and/or Na₂HPO₄.

18. The pharmaceutical composition of claim 17, wherein,
the content of zinc ions is at least about 1.5 moles of zinc ions/6 moles of acylated insulin, preferably about 1.5-12 moles of zinc ions/6 moles of acylated insulin, preferably about 1.5-10 moles of zinc ions/6 moles of acylated insulin, preferably about 1.5-8 moles of zinc ions/6 moles of acylated insulin, preferably about 1.5-6 moles of zinc ions/6 moles of acylated insulin, preferably about 1.5-4.5 moles of zinc ions/6 moles of acylated insulin, preferably about 1.5-3.5 moles of zinc ions /6 moles of acylated insulin, preferably about 1.5-2.3 moles of zinc ions/6 moles of acylated insulin; and/ or
the content of glycerin is no more than about 2.5% (weight/weight), preferably no more than about 2% (weight/weight), preferably about 0.3% to about 2% (weight/weight), preferably about 0.5% to about 1.8% (weight/weight), preferably about 0.7% to about 1.8% (weight/weight), preferably about 1% to about 1.8% (weight/weight), preferably about 1.5% to about 1.7% (weight/weight); and/or
the content of the phenol is about 30-70 mM, preferably about 40-65 mM, preferably about 45-60 mM; preferably about 45 mM, about 46 mM, about 47 mM, about 48 mM, about 49 mM, 50 mM, about 51mM, about 52 mM, about 53 mM, about 54 mM, about 55 mM, about 56 mM, about 57 mM, about 58 mM, about 59 mM, about 60 mM, about 61 mM, about 62 mM, about 63 mM, about 64 mM, or about 65 mM; and/or
the content of the *m-cresol* is about 0-35 mM, preferably about 0-20 mM, preferably about 0-10mM, preferably about 0 mM, about 1 mM, about 2 mM, about 3 mM, about 4 mM, about 5 mM, about 6m M, about 7 mM, about 8 mM, about 9 mM, about 10 mM, about 11 mM, about 12 mM, about 13 mM, about 14 mM, or about 15 mM; and/or
the content of the NaCl is about 0-150 mM, preferably about 5-120 mM, preferably about 10-120 mM, preferably about 10-100 mM, preferably about 10-75 mM, preferably about 10-50 mM, preferably about 10 -30 mM, preferably about 10-20 mM; preferably about 10 mM, about 11 mM, about 12 mM, about 13 mM, about 14 mM, about 15 mM, about 16 mM, about 17 mM, about 18 mM, about 19 mM, or about 20 mM; and/or
the content of Na₂HPO₄ is about 0-75 mM, preferably about 5-60 mM, preferably about 5-50 mM, preferably about 5-25 mM, preferably about 5-15 mM; preferably about 5-10 mM; and/or
the pharmaceutical composition has a pH value of about 6.5-8.5; preferably a pH value of about 6.8-8.2; preferably a pH value of about 7.0-8.2; preferably a pH value of 7.2-7.6; more preferably a pH value of 7.4 or 7.6.

19. A pharmaceutical composition comprising:
about 0.9-1.5 mM acylated insulin;
insulinotropic GLP-1 compound, the molar ratio of the insulinotropic GLP-1 compound to the acylated insulin is at least about 8:100, preferably about (8:100)-(50:100), preferably about (10:100)-(50:100), preferably about (13:100)-(50:100), preferably about (13:100)-(40:100), preferably about (13:100)- (35:100), preferably about (13:100)-(27:100), preferably about (13:100)-(20:100);
about 1.5-2.3 moles of zinc ions per 6 moles of acylated insulin;
about 45 mM-60 mM phenol;
about 0-10 mM *m*-cresol;
about 10-20 mM NaCl;
about 15-17 mg/ml glycerin;
about 5-10 mM Na₂HPO₄; and
having a pH value of about 6.5-8.0,
wherein the acylated insulin is A14E, B16H, B25H, B29K (N(ε)-eicosanedioyl-yGlu-5xOEG), desB30 human insulin; A14E, B16H, B25H, B29K (N(ε)-docosanedioyl-γGlu-5xOEG), desB30 human insulin; A14E, B16H, B25H, B29K (N(ε)-eicosanedioyl-γGlu-6xOEG), desB30 human insulin; A14E, B16H, B25H, B29K (N(ε)-docosanedioyl-γGlu-6xOEG), desB30 human insulin; A14E, B16H, B25H, B29K (N(ε)-eicosanedioyl-yGlu-8xOEG), desB30 human insulin; A14E, B16H, B25H, B29K (N(ε)-docosanedioyl-yGlu-8xOEG), desB30 human insulin; A14E, B16H, B25H, B29K (N(ε)-eicosanedioyl-γGlu-11xOEG ), desB30 human insulin; A14E, B16H, B25H, B29K (N(ε)-eicosanedioyl-γGlu-12xOEG), desB30 human insulin; A14E, B16H, B25H, B29K (N(ε)-docosanedioyl-γGlu-11xOEG), desB30 human insulin; A14E, B16H, B25H, B29K (N(ε)-docosanedioyl-yGlu-12xOEG), desB30 human insulin; or A14E, B16H, B25H, B29K (N(ε)-docosanedioyl-γGlu-18xOEG), desB30 human insulin, and
the insulinotropic GLP-1 compound is *N*-ε²⁶-[2-(2-[2-(2-[2-(2-[4-(19-carboxynonadecanoylamino)-4(*S*) carboxybutanoylamino] ethoxy)ethoxy]acetylamino)ethoxy]ethoxy)acetyl ][Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly8, Arg34] GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl - [Arg34]GLP-1-(7-37) peptide,
*N*-ε²⁶-[2-(2-[2-(2-[2-(2-[4-(21-carboxyheneicosanoylamino)-4(*S*)-carboxybutanoylamino] ethoxy)ethoxy]acetylamino)ethoxy]ethoxy)acetyl ] [Gly8, Arg34] GLP-1-(7-37) peptide, or
*N*-ε²⁶-[2-(2-[2-(4-[21-carboxyheneicosanoylamino]-4(*S*)-carboxybutanoylamino)ethoxy]ethoxy)acetyl][Gly8, Arg34] GLP-1-(7-37) peptide.

20. A pharmaceutical composition comprising:
about 1.2 mM acylated insulin;
at least about 0.096 mM, preferably about 0.096-0.6 mM, preferably about 0.12-0.6 mM, preferably about 0.16-0.6 mM, preferably about 0.16-0.48 mM, preferably about 0.16-0.42 mM, preferably about 0.16-0.32 mM, preferably about 0.16-0.24 mM insulinotropic GLP-1 compound;
about 1.5-2.3 moles of zinc ions per 6 moles of acylated insulin;
about 45 mM-60 mM phenol;
about 0-10 mM *m*-cresol;
about 10-20 mM NaCl;
about 15-17 mg/ml glycerin;
about 5-10 mM Na₂HPO₄; and
having a pH value of about 6.5-8.0,
wherein the acylated insulin is A14E, B16H, B25H, B29K (N(ε)-eicosanedioyl-yGlu-5xOEG), desB30 human insulin; A14E, B16H, B25H, B29K (N(ε)-docosanedioyl-γGlu-5xOEG), desB30 human insulin; A14E, B16H, B25H, B29K (N(ε)-eicosanedioyl-γGlu-6xOEG), desB30 human insulin; A14E, B16H, B25H, B29K (N(ε)-docosanedioyl-γGlu-6xOEG), desB30 human insulin; A14E, B16H, B25H, B29K (N(ε)-eicosanedioyl-γGlu-8xOEG), desB30 human insulin; A14E, B16H, B25H, B29K (N(ε)-docosanedioyl-yGlu-8xOEG), desB30 human insulin; A14E, B16H, B25H, B29K (N(ε)-eicosanedioyl-γGlu-11xOEG ), desB30 human insulin; A14E, B16H, B25H, B29K (N(ε)-eicosanedioyl-γGlu-12xOEG), desB30 human insulin; A14E, B16H, B25H, B29K (N(ε)-docosanedioyl-γGlu-11xOEG), desB30 human insulin; A14E, B16H, B25H, B29K(N(ε)-docosanedioyl-yGlu-12xOEG), desB30 human insulin; or A14E, B16H, B25H, B29K (N(ε)-docosanedioyl-γGlu-18xOEG), desB30 human insulin, and
the insulinotropic GLP-1 compound is *N*-ε²⁶-[2-(2-[2-(2-[2-(2-[4-(19-carboxynonadecanoylamino)-4(*S*) carboxybutanoylamino]ethoxy)ethoxy]acetylamino)ethoxy]ethoxy)acetyl ][Gly8, Arg34]GLP-1-(7-37) peptide,
*N*-ε²⁶-[2-(2-[2-(4-[19-carboxynonadecanoylamino]-4(*S*)-carboxybutanoylamino)ethoxy]ethoxy)acetyl][Gly8, Arg34]GLP-1-(7-37) peptide,
*N*-ε²⁶-(19-carboxynonadecanoylamino)-4(*S*)-carboxybutanoyl-[Gly8, Arg34]GLP-1-(7-37) peptide,
*N*-ε²⁶-(19-carboxynonadecanoylamino)-4(*S*)-carboxybutanoyl-[Arg34]GLP-1-(7-37) peptide,
*N*-ε²⁶-[2-(2-[2-(2-[2-(2-[4-(21-carboxyheneicosanoylamino)-4(*S*)-carboxybutanoylamino]ethoxy)ethoxy]acetylamino)ethoxy]ethoxy) acetyl] [Gly8, Arg34] GLP-1-(7-37) peptide, or
N-ε²⁶-[2-(2-[2-(4-[21-carboxyheneicosanoylamino]-4(*S*)-carboxybutanoylamino)ethoxy]ethoxy)acetyl][Gly8, Arg34] GLP-1-(7-37) peptide.

21. The pharmaceutical composition according to any one of claims 1-20 for use as a medicament.

22. The pharmaceutical composition according to any one of claims 1-20 for use in treating or preventing diabetes, hyperglycemia, and/or impaired glucose tolerance.

23. The use of the pharmaceutical composition according to any one of claims 1-20 in the preparation of medicaments for treating or preventing diabetes, hyperglycemia, and/or impaired glucose tolerance.

24. A method for treating or preventing diabetes, hyperglycemia, and/or impaired glucose tolerance, comprising administering a therapeutically effective amount of the pharmaceutical composition according to any one of claims 1-20.
